(19) Europäisches Patentamt / European Patent Office / Office européen des brevets

(11) **EP 2 630 259 B1**

(12) **EUROPEAN PATENT SPECIFICATION**

(45) Date of publication and mention of the grant of the patent:
**15.04.2015 Bulletin 2015/16**

(51) Int Cl.:
***C12Q 1/68*** (2006.01)

(21) Application number: **11785107.1**

(22) Date of filing: **20.10.2011**

(86) International application number:
**PCT/IB2011/054688**

(87) International publication number:
**WO 2012/052954 (26.04.2012 Gazette 2012/17)**

(54) **SIGNATURES OF CLINICAL OUTCOME IN GASTRO INTESTINAL STROMAL TUMORS AND METHOD OF TREATMENT OF GASTROINSTESTINAL STROMAL TUMORS**

SIGNATUREN VON KLINISCHER BEDEUTUNG IN GASTROINTESTINALEN STROMALEN TUMOREN UND BEHANDLUNGSMETHODE DERSELBEN

SIGNATURES DE L'EVOLUTION CLINIQUE DE TUMEURS STROMALES GASTRO-INTESTINALES ET METHODES DE TRAITEMENT DE TUMEURS STROMALES GASTRO-INTESTINALES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.10.2010 EP 10013806**

(43) Date of publication of application:
**28.08.2013 Bulletin 2013/35**

(73) Proprietors:
• **Université Bordeaux Segalen**
  **33076 Bordeaux Cedex (FR)**
• **Inserm**
  **75654 Paris Cedex 13 (FR)**
• **Institut Bergonié**
  **33076 Bordeaux Cedex (FR)**
• **Institut Curie**
  **75005 Paris (FR)**

(72) Inventors:
• **CHIBON, Frédéric**
  **F-33640 Isle-Saint-Georges (FR)**
• **COINDRE, Jean-Michel**
  **F-33000 Bordeaux (FR)**
• **AURIAS, Alain**
  **F-91440 Bures-sur-Yvette (FR)**

(74) Representative: **Novagraaf Technologies**
  **Bâtiment O2**
  **2, rue Sarah Bernhardt**
  **CS90017**
  **92665 Asnières-sur-Seine Cedex (FR)**

(56) References cited:
WO-A1-2006/129064    WO-A1-2008/025526
WO-A1-2010/068951    WO-A2-2006/130613
WO-A2-2008/156614

• YAMASHITA KIMIHIRO ET AL: "Chromosomal numerical abnormality profiles of gastrointestinal stromal tumors", JAPANESE JOURNAL OF CLINICAL ONCOLOGY, vol. 36, no. 2, February 2006 (2006-02), pages 85-92, XP002668812,
• MENDIOLA M ET AL: "Aurora kinases as prognostic biomarkers in ovarian carcinoma", HUMAN PATHOLOGY, SAUNDERS, PHILADELPHIA, PA, US, vol. 40, no. 5, 1 May 2009 (2009-05-01), pages 631-638, XP026115079, ISSN: 0046-8177, DOI: 10.1016/J.HUMPATH. 2008.10.011 [retrieved on 2009-01-20]

SEQUENCE LISTING

<110> Université Bordeaux Segalen
INSERM
Institut Bergonié
Institut Curie

<120> Signatures of clinical outcome in gastrointestinal stromal tumors and method of treatment of gastrointestinal stromal tumors

<130> BIP208465PC00

<150> EP10013806
<151> 2010-10-20

<160> 15

<170> PatentIn version 3.5

<210> 1
<211> 403
<212> PRT
<213> Homo sapiens

<400> 1

```
Met Asp Arg Ser Lys Glu Asn Cys Ile Ser Gly Pro Val Lys Ala Thr
1               5                   10                  15

Ala Pro Val Gly Gly Pro Lys Arg Val Leu Val Thr Gln Gln Phe Pro
            20                  25                  30

Cys Gln Asn Pro Leu Pro Val Asn Ser Gly Gln Ala Gln Arg Val Leu
        35                  40                  45

Cys Pro Ser Asn Ser Ser Gln Arg Ile Pro Leu Gln Ala Gln Lys Leu
    50                  55                  60

Val Ser Ser His Lys Pro Val Gln Asn Gln Lys Gln Lys Gln Leu Gln
65                  70                  75                  80

Ala Thr Ser Val Pro His Pro Val Ser Arg Pro Leu Asn Asn Thr Gln
            85                  90                  95

Lys Ser Lys Gln Pro Leu Pro Ser Ala Pro Glu Asn Asn Pro Glu Glu
        100                 105                 110

Glu Leu Ala Ser Lys Gln Lys Asn Glu Glu Ser Lys Lys Arg Gln Trp
        115                 120                 125

Ala Leu Glu Asp Phe Glu Ile Gly Arg Pro Leu Gly Lys Gly Lys Phe
    130                 135                 140

Gly Asn Val Tyr Leu Ala Arg Glu Lys Gln Ser Lys Phe Ile Leu Ala
```

145               150               155               160

Leu Lys Val Leu Phe Lys Ala Gln Leu Glu Lys Ala Gly Val Glu His
              165            170              175

Gln Leu Arg Arg Glu Val Glu Ile Gln Ser His Leu Arg His Pro Asn
        180            185            190

Ile Leu Arg Leu Tyr Gly Tyr Phe His Asp Ala Thr Arg Val Tyr Leu
        195            200            205

Ile Leu Glu Tyr Ala Pro Leu Gly Thr Val Tyr Arg Glu Leu Gln Lys
    210            215            220

Leu Ser Lys Phe Asp Glu Gln Arg Thr Ala Thr Tyr Ile Thr Glu Leu
225            230            235            240

Ala Asn Ala Leu Ser Tyr Cys His Ser Lys Arg Val Ile His Arg Asp
        245            250            255

Ile Lys Pro Glu Asn Leu Leu Leu Gly Ser Ala Gly Glu Leu Lys Ile
        260            265            270

Ala Asp Phe Gly Trp Ser Val His Ala Pro Ser Ser Arg Arg Thr Thr
        275            280            285

Leu Cys Gly Thr Leu Asp Tyr Leu Pro Pro Glu Met Ile Glu Gly Arg
        290            295            300

Met His Asp Glu Lys Val Asp Leu Trp Ser Leu Gly Val Leu Cys Tyr
305            310            315            320

Glu Phe Leu Val Gly Lys Pro Pro Phe Glu Ala Asn Thr Tyr Gln Glu
        325            330            335

Thr Tyr Lys Arg Ile Ser Arg Val Glu Phe Thr Phe Pro Asp Phe Val
        340            345            350

Thr Glu Gly Ala Arg Asp Leu Ile Ser Arg Leu Leu Lys His Asn Pro
        355            360            365

Ser Gln Arg Pro Met Leu Arg Glu Val Leu Glu His Pro Trp Ile Thr
        370            375            380

Ala Asn Ser Ser Lys Pro Ser Asn Cys Gln Asn Lys Glu Ser Ala Ser
385            390            395            400

Lys Gln Ser

<210> 2
<211> 2339
<212> DNA
<213> cDNA corresponding to mRNA

<400> 2

```
acaaggcagc ctcgctcgag cgcaggccaa tcggctttct agctagaggg tttaactcct        60

atttaaaaag aagaaccttt gaattctaac ggctgagctc ttggaagact tgggtccttg       120

ggtcgcaggt gggagccgac gggtgggtag accgtggggg atatctcagt ggcggacgag       180

gacggcgggg acaaggggcg gctggtcgga gtggcggagc gtcaagtccc ctgtcggttc       240

ctccgtccct gagtgtcctt ggcgctgcct tgtgcccgcc cagcgccttt gcatccgctc       300

ctgggcaccg aggcgccctg taggatactg cttgttactt attacagcta gaggcatcat       360

ggaccgatct aaagaaaact gcatttcagg acctgttaag gctacagctc cagttggagg       420

tccaaaacgt gttctcgtga ctcagcaatt tccttgtcag aatccattac ctgtaaatag       480

tggccaggct cagcgggtct tgtgtccttc aaattcttcc cagcgcattc ctttgcaagc       540

acaaaagctt gtctccagtc acaagccggt tcagaatcag aagcagaagc aattgcaggc       600

aaccagtgta cctcatcctg tctccaggcc actgaataac acccaaaaga gcaagcagcc       660

cctgccatcg gcacctgaaa ataatcctga ggaggaactg gcatcaaaac agaaaaatga       720

agaatcaaaa aagaggcagt gggctttgga agactttgaa attggtcgcc ctctgggtaa       780

aggaaagttt ggtaatgttt atttggcaag agaaaagcaa agcaagttta ttctggctct       840

taaagtgtta tttaaagctc agctggagaa agccggagtg gagcatcagc tcagaagaga       900

agtagaaata cagtcccacc ttcggcatcc taatattctt agactgtatg gttatttcca       960

tgatgctacc agagtctacc taattctgga atatgcacca cttggaacag tttatagaga      1020

acttcagaaa ctttcaaagt ttgatgagca gagaactgct acttatataa cagaattggc      1080

aaatgccctg tcttactgtc attcgaagag agttattcat agagacatta agccagagaa      1140

cttacttctt ggatcagctg gagagcttaa aattgcagat tttgggtggt cagtacatgc      1200

tccatcttcc aggaggacca ctctctgtgg caccctggac tacctgcccc ctgaaatgat      1260

tgaaggtcgg atgcatgatg agaaggtgga tctctggagc cttggagttc tttgctatga      1320

atttttagtt gggaagcctc cttttgaggc aaacacatac caagagacct acaaaagaat      1380

atcacgggtt gaattcacat tccctgactt tgtaacagag ggagccaggg acctcatttc      1440

aagactgttg aagcataatc ccagccagag gccaatgctc agagaagtac ttgaacaccc      1500

ctggatcaca gcaaattcat caaaaccatc aaattgccaa aacaaagaat cagctagcaa      1560

acagtcttag gaatcgtgca gggggagaaa tccttgagcc agggctgcca tataacctga      1620
```

```
caggaacatg ctactgaagt ttattttacc attgactgct gccctcaatc tagaacgcta      1680

cacaagaaat atttgtttta ctcagcaggt gtgccttaac ctccctattc agaaagctcc      1740

acatcaataa acatgacact ctgaagtgaa agtagccacg agaattgtgc tacttatact      1800

ggttcataat ctggaggcaa ggttcgactg cagccgcccc gtcagcctgt gctaggcatg      1860

gtgtcttcac aggaggcaaa tccagagcct ggctgtgggg aaagtgacca ctctgccctg      1920

accccgatca gttaaggagc tgtgcaataa ccttcctagt acctgagtga gtgtgtaact      1980

tattgggttg gcgaagcctg gtaaagctgt tggaatgagt atgtgattct ttttaagtat      2040

gaaaataaag atatatgtac agacttgtat tttttctctg gtggcattcc tttaggaatg      2100

ctgtgtgtct gtccggcacc ccggtaggcc tgattgggtt tctagtcctc cttaaccact      2160

tatctcccat atgagagtgt gaaaaatagg aacacgtgct ctacctccat ttagggattt      2220

gcttgggata cagaagaggc catgtgtctc agagctgtta agggcttatt tttttaaaac      2280

attggagtca tagcatgtgt gtaaacttta aatatgcaaa taaataagta tctatgtct      2339
```

<210> 3
<211> 20
<212> DNA
<213> Artificial Sequence

<220>
<223> artificial sequence

<400> 3
tggatctgct ggttgtccaa        20

<210> 4
<211> 22
<212> DNA
<213> Artificial Sequence

<220>
<223> artificial sequence

<400> 4
ccagtcttga tcagctgcac at        22

<210> 5
<211> 34
<212> DNA
<213> Artificial Sequence

<220>
<223> artificial sequence

<400> 5
aggtgtttac tgccccacta ttatctggtt caga        34

<210> 6
<211> 22

<212> DNA
<213> Artificial Sequence

<220>
<223> artificial sequence

<400> 6
tcatgtcaga gagagagctt gg          22

<210> 7
<211> 20
<212> DNA
<213> Artificial Sequence

<220>
<223> artificial sequence

<400> 7
cgtgcactcc tgttctgacc          20

<210> 8
<211> 20
<212> DNA
<213> Artificial Sequence

<220>
<223> artificial sequence

<400> 8
aatggttcac ctcgaacacc          20

<210> 9
<211> 22
<212> DNA
<213> Artificial Sequence

<220>
<223> artificial sequence

<400> 9
ctcggtaata caagcgaact cc          22

<210> 10
<211> 21
<212> DNA
<213> artificial sequence

<220>
<223> artificial sequence

<400> 10
cctccacaca ctccagttag g          21

<210> 11
<211> 20
<212> DNA
<213> Artificial Sequence

<220>
<223> artificial sequence

<400> 11
tgatcagttg gtccttctcg          20

<210> 12
<211> 20
<212> DNA
<213> Artificial Sequence

<220>
<223> artificial sequence

<400> 12
gcatggctct cagattcacc          20

<210> 13
<211> 21
<212> DNA
<213> Artificial Sequence

<220>
<223> artificial sequence

<400> 13
tcgaggaatg tgaggtattg g          21

<210> 14
<211> 20
<212> DNA
<213> Artificial Sequence

<220>
<223> artificial sequence

<400> 14
tcttcctcat gctgttcagg          20

<210> 15
<211> 21
<212> DNA
<213> Artificial Sequence

<220>
<223> artificial sequence

<400> 15
tgtacacagt gtccaccaag g          21

## Description

## Technical field

The present invention refers to a method for in vitro predicting survival and/or metastatic outcome of gastrointestinal stromal tumors (GISTs). **It describes** a kit for (i) the in vitro prediction of the survival outcome of a patient suffering from GIST, (ii) and/or the development of metastases in a patient treated for or suffering from GIST, (ii) and/or the prediction of the efficacy of a treatment for GIST. The present invention also **describes** a method for screening for compounds for the use in the treatment of GISTs, and to a compound for its use in the treatment of GISTs.

Therefore, the present invention has utility in the medical and pharmaceutical fields, especially in the field of diagnosis.

In the description below, the references into brackets ([]) refer to the listing of references situated at the end of the text.

## Background of the Invention

Gastrointestinal stromal tumors (GISTs) are the most frequent mesenchymal tumors of the gastrointestinal tract and account for approximately 25% of soft tissue sarcomas. GISTs are thought to arise from the intestinal cells of Gajal (1), or from a common progenitor cell (2).

The KIT tyrosine kinase or the platelet-derived factor receptor a (PDGFRA) activating mutations are early oncogenic events in GISTs. Most GISTs (80%) are characterized by activating mutations of the *KIT* tyrosine kinase receptor, while a subset (8%) harbours platelet-derived factor receptor $\alpha$ (*PDGFRA* mutations (3,4). In addition to these mutations, other genetic changes do occur, the most frequent alterations reported being 14q, 22q and 1p deletions (5). Overall, GIST cytogenetics is quite simple and imbalances manly involve full chromosomes or chromosome arms. Notably, GIST molecular and cytogenetic profiles correlate with disease progression. Nevertheless, it has been observed that changes are more frequent and more complex in advanced tumors (6). Furthermore, the genetic basis of the metastatic outcome of GISTs is still poorly understood.

Clinical management of GISTs consists mainly of surgical resection with adjuvant or neo-adjuvant targeted therapy with Imatinib Mesylate (Gleevec, formerly STI571, Novartis Pharma AG) which has been demonstrated to target the KIT- or PDGFRA-aberrant signaling induced by activating mutations (7). The majority of cases can be cured by surgical resection alone, but 20-40% of patients relapse with distant liver metastasis being the most common manifestation of the recurrent disease.

Many pathological criteria based on tumor site, size, cell type, degree of necrosis and mitotic rate have been proposed for predicting the outcome of patients with GISTs. A consensus was found by the National Institute of Health (NIH) in 2001 to estimate the relative risk of GISTs based on tumor size and mitotic count (8) and in 2006, the Armed Forces Institute of Pathology (AFIP) proposed an updated system taking into account also a tumor location (9). Even if these two systems are particularly efficient in determining the metastatic risk of GISTs, they are based on an indirect histopathological reflection of tumor aggressiveness. Moreover, the cutoff values for these criteria have been determined empirically leading to subjectivity that is inevitable in skilled pathologists' assessments. Hence, there is a need to more deeply understand the biology underlining the aggressiveness of GISTs in order to identify objective biomarkers that enhance the specificity and the reproducibility of outcome prediction.

The development of a valid and reliable, investigator-independent method of GIST prognostication is essential for the proper clinical management of GIST patients, especially in the context of adjuvant treatment, where many patients are exposed to imatinib while only a small proportion will likely benefit from such treatment (19).

To achieve this purpose, genomic and expression profiling has already been used but only partial and heterogeneous results have been reported. At the genomic level, it has been shown that the genome complexity level increases with tumor stage (6, 10), but no threshold has ever been defined and no specific alteration has been proposed except for p16$^{INK4A}$ alterations whose role in metastasis development is still controversial (11-16). At the expression level, Yamaguchi and colleagues have proposed a gene-expression signature : they identified CD26 as a prognostic marker but only in GISTs of gastric origin. Nevertheless, the authors concluded that CD26 might not be the cause of malignant progression of gastric GISTs. Moreover, this signature is limited as it has been established on only a few cases (32 GISTs), it predicts outcome only in gastric GISTs (but not in GIST of the small intestine) and it has not been compared to histopathological grading methods considered as "gold standard" (17).

In the few genome or expression profiling analyses using smaller numbers of GISTs that have been conducted before this study, only one (35) presents an integrative analysis gathering genome and transcription profiling as we present here. This study was based on 25 patients and aimed to identify target genes located in altered regions described within the last 15 years. Essentially, many studies have described GISTs genome and demonstrated that GISTs cytogenetics is quite simple, reflected by only few aberrations, deletions being more frequent than gains (6, 10, 11, 12, 36, 37).

All these studies concluded that chromosome 14, 22 and 1p deletions are the most frequent aberrations. It has also been shown that changes are more frequent in high-risk and overly malignant GISTs than in low/intermediate-risk GISTs (6, 10), but a strong association between one alteration and prognosis has not yet been identified, except *CDKN2A* alterations as discussed above. At the expression level, most of the studies have been set up to enhance delineation of diagnosis (38, 39) or to identify expression differences according to *KIT* or *PDGFRA* mutation status (40-42).

The AURKA, encoded for a gene that maps to chromosome 20q13, is a mitotic centrosomal protein kinase (20). It is a well known oncogene, which main role in tumor development is the control of chromosome segregation during mitosis (21). Gene amplification and AURKA overexpression have been widely described in many cancer types (22). In particular, as it has been clearly demonstrated that AURKA overexpression induces centrosome duplication-distribution abnormalities and aneuploidy leading to transformation in breast cancer cells (23). Actually, centrosomes maintain genomic stability through the establishment of the bipolar spindle body during cell division, ensuring equal segregation of replicated chromosomes to two daughter cells. The AURKA expression has also been associated with poor prognosis mainly in breast carcinoma (24), colon carcinoma (25, 26), neuroblastoma (27) and head and neck squamous cell carcinoma (28). Taken together, these data indicate that up-regulation of AURKA expression could be a major driving event in establishing genome complexity leading to wild gene expression reprogramming, creating optimal conditions for development of metastasis. AURKA inhibitors are currently under clinical studies (29-33).

Yamashita et al. ("Chromosomal numerical abnormality profiles of gastrointestinal stromal tumors", Japanese Journal of clinical oncology, vol. 36, no. 2, 2006-02, pages 85-92) describe the examination by means of FISH for chromosomal abnormality in GISTs, in order to test chromosomal numerical abnormality (CNA) as a possible biological predictor of biological behaviour of GISTs. Yamashita et al. also describe that CNA in the primary sites was more extensive in the GISTs with recurrence and metastasis than in those without, especially as to the loss of chromosome 20 and genomic imbalance of AURKA -containing BAC probe on 20q in the cases with metastases.

WO2010/068951 document describes the use of an inhibitor of AURKA and an inhibitor of Src in *in vitro* synergy assays to test whether C1368 (a selective AURKA inhibitor) and dasatinib (Src kinase inhibitor) synergize to induce cell death in HCT116 human cancer cell line, which is a colorectal cancer cell line (see p. 3, 1. 27-28). GIST is not a cancer in which the association between AURKA and Src inhibitor is tested.

WO2006/129064 document teaches that AURKA inhibitors that may be useful for the treatment of tumours. However, this document describes no result of such inhibitors on cancer cell lines or patient; D3 is only putative.

In view of all these elements, it clearly still exists a need of new tools allowing to predict outcome of GISTs, notably palliating the failures, drawbacks and obstacles of the state of the art.


**Description of the invention**


After important researches, the Applicant found surprisingly a one gene-expression signature prognostic for clinical outcome of primary GISTs.

Surprisingly, it has been demonstrated by the Applicant that the CINSARC signature (CINSARC for Complexity INdex in SARComa, a 67 genes-expression prognostic signature related to genome complexity in sarcomas, PCT/FR2010/000323, [55]) and/or a new one-gene-expression signature predict metastatic outcome in GIST and that the combination of each of these signatures with genome imbalances outperforms current histopathological grading method in determining patient prognosis. More specifically, these molecular signatures identify "at-risk patients" within cases stratified as intermediate-risk according to the Armed Forces Institut of Pathology classification.

The Applicant manages to show that a positive correlation exists between GI (Genomic Index) and *AURKA* expression.

The Applicant surprisingly manages to construct a decisional algorithm based on GI and *AURKA* expression.

Advantageously, application of the signature permits more selective imatinib therapy leading to decreased iatrogenic morbidity and improved outcomes for individual patients.

Accordingly, in a first aspect, the invention **describes** a method for *in vitro* predicting survival and/or metastatic outcome of gastrointestinal stromal tumors (GISTs), the method comprising the measure of the level, in a patient-derived biological sample of GIST, of a pool of polypeptides or polynucleotides consisting in Aurora kinase A (AURKA).

Method for in vitro predicting survival and/or metastatic outcome of gastrointestinal stromal tumors (GISTs), characterized in that it comprises the measure of the level, in a patient-derived biological sample of GIST, of a pool of polypeptides or polynucleotides consisting in Aurora kinase A (AURKA), wherein GIST is classified in a group with high risk to develop metastases within 5 years, i.e. with a risk to develop metastases within 5 years of more than 80%, when AURKA is up-regulated compared to a group with no risk to develop metastases within 5 years, wherein AURKA is down-regulated and less than 9.13, wherein 9.13 is calculated from the AURKA mean expression of stratified tumor samples.

"Predicting survival and/or metastatic outcome" refers herein to predicting whether a patient has a chance to survive, or a risk to develop metastases following the outcome of a GIST. The survival or development of metastases may be calculated from invention, GIST may be classified in a group with high risk to develop metastases within 5 years of an outcome of GIST, or in a group with no risk to develop metastases within 5 years, or in an intermediate group. More particularly, the group of patient with high risk to develop metastases within 5 years is characterized by a risk to develop metastases within 5 years of more than 80%, when AURKA is up-regulated, compared to a group with no risk to develop metastases within 5 years when AURKA is down-regulated.

"Patient-derived biological sample of GIST" refers herein to any biological sample containing GIST cells and obtained from a patient treated for or suffering from GIST. For example, GIST may be primary untreated tumors.

"Polypeptide" refers herein to the AURKA protein (Genbank accession number NM_198433; SEQ ID NO: 1), a AURKA protein fragment, a AURKA protein region or a derivative of AURKA protein. For example, the polypeptide may be a polypeptide having at least 70% of sequence identity with the peptidic sequence of AURKA protein, or a polypeptide having at least 80% of sequence identity with the peptidic sequence of AURKA protein, or a polypeptide having at least 90% of sequence identity with the peptidic sequence of AURKA protein.

"Polynucleotide" refers herein to any polynucleotide coding for the polypeptide as defined above, or to any polynucleotide hybridizing under stringent conditions to a polypeptide coding for the polypeptide as defined above. The polynucleotide of the invention may be any of DNA and RNA, for example the sequence SEQ ID NO: 2. The DNA may be in any form of genomic DNA, a genomic DNA library, cDNA or a synthetic DNA. Moreover, the polynucleotide of the present invention may be any of those amplified directly by an RT-PCR method using total RNA or an mRNA fraction prepared from a GIST. The polynucleotide of the present invention includes a polynucleotide that hybridizes under stringent conditions to a polynucleotide.

The measure of the level of polypeptides may be realized by any appropriate technique known by the man skilled in the art. It may be, for example, an immunohistochemistry technique, in which the expression of the protein is measured after hybridization of an antibody recognizing specifically the AURKA protein.

The measure of the level of polynucleotides may be realized be any appropriate technique known by the man skilled in the art. It may be, for example, a method of genomic qPCR (quantitative polymerization chain reaction), CGH-array (Comparative Genomic Hibridization) or RT-qPCR (real time qPCR) in order to check copy number of genomic DNA or quantify expression of genomic DNA.

Advantageously, the AURKA expression allows to predicting survival and/or metastatic outcome of GISTs, and no other gene or protein expression has to be measured. The method of the invention may comprise the calculation of the Genomic Index (GI), i.e. the number and type of alterations of the GIST genome, according to the formula as follows:

$$GI = A^2 \times C,$$

wherein A is the number of alterations in GIST genome and C is the number of involved chromosomes in GIST.

"Number of alterations in GIST genome" refers herein to different numerical and segmental gains and losses. The alterations may for example involve whole chromosome arms or chromosome without rearrangement, or intra-chromosome gains or losses. It may be measured by techniques known in the art, such as CGH-array.

"Number of involved chromosomes in GIST" refers herein to the number of chromosomes of GIST cells having an alteration. The number of chromosome may be measured by CGH-array.

Advantageously, GIST is classified in a group of metastasis- and disease-free survival group when AURKA is down-regulated and the GI is equal or less than 10. In this case, AURKA expression may be less than 9.13, or less than 9, or less than 8, or less than 7, or less than 6, or less than 5. In this case, there is a survival of 5 years, i.e. there are no metastasis or disease during 5 years after GIST outcome or after the end of a treatment. In a particular embodiment, GIST may be classified in a group with low risk to develop metastases within 5 years, i.e. with a risk to develop metastases within 5 years comprises between 0% and 10%, when AURKA expression is equal or less than the mean of AURKA expression, and GI is equal or less than 10, said mean being the mean of AURKA expression/level in several GISTs, for example a series of 50 to 70 GISTs, for example 60 GISTs. In this case, there is no metastasis or disease during 5 years.

Alternatively, GIST may be classified in a group with high risk to develop metastases within 5 years, i.e. with a risk to develop metastases within 5 years more than 75%, when AURKA expression is more than the mean of AURKA expression and GI is more than 10, said mean being the mean of AURKA expression in several GISTs, for example in a series of 50 to 70 GISTs, for example 60 GISTs. In this case, there are 75% of cases of metastasis or disease during 5 years after GIST outcome or after the end of a treatment.

A kit for the *in vitro* prediction of the survival outcome of a patient suffering from GIST, and/or the development of metastases in a patient treated for or suffering from GIST, and/or the prediction of the efficacy of a treatment for GIST, characterized in that it comprises means for detecting and/or quantify, in a sample, AURKA expression/level, and means for the calculation of the GI is described.

"Means for detecting and/or AURKA expression/level" may be any means for detecting levels of proteins or of polynucleotides known by the man skilled in the art. The means may be for example the means to realize an immunohistochemistry analysis, a western blot or a q-PCR.

"Means for the calculation of the GI" refers herein to any means allowing the calculation of the number of alterations in GIST genome and of the number of involved chromosomes in GIST.

A method for screening for compounds for the use in the treatment of GISTs **is described**, comprising the steps of :

a. Contacting a test compound with a patient-derived biological sample containing GISTs cells,
b. Measuring the expression or the level of AURKA,
c. Comparing said expression or level of AURKA with the expression of AURKA before the contact between said test compound and said sample,
d. Selecting said test compound that allows a down-regulation of the expression/level of AURKA.

"Down-regulation" refers herein to any diminution of the expression or level of AURKA protein or polynucleotide.

The method may also comprise the steps of:

e. Calculating GI,
f. Comparing said GI with the GI before the contact between said test compound and said sample,
g. Selecting said test compound allowing a down-regulation of the GI to 10 or less.

An AURKA inhibitor for its use in the treatment of GISTs **is described**.

"Inhibitor" refers herein to any compound allowing a decrease of the expression/level of AURKA protein, or in a decrease of a biological effect of AURKA, when the inhibitor is contacted with GIST. The AURKA inhibitor may be PHA-739358 (29, 54), MLN8237 (30), MK-5108 (33).

Another object of the invention is a method of treatment of GIST, in a subject in need thereof, comprising the step of administering to the patient a pharmaceutically effective dose of a inhibitor as defined above.

**Brief description of the figures**

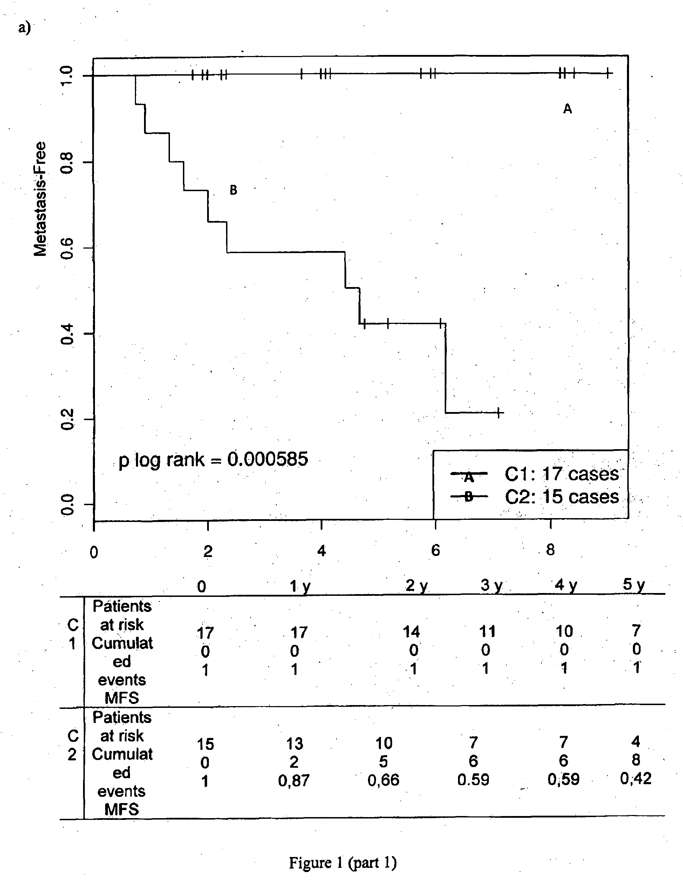

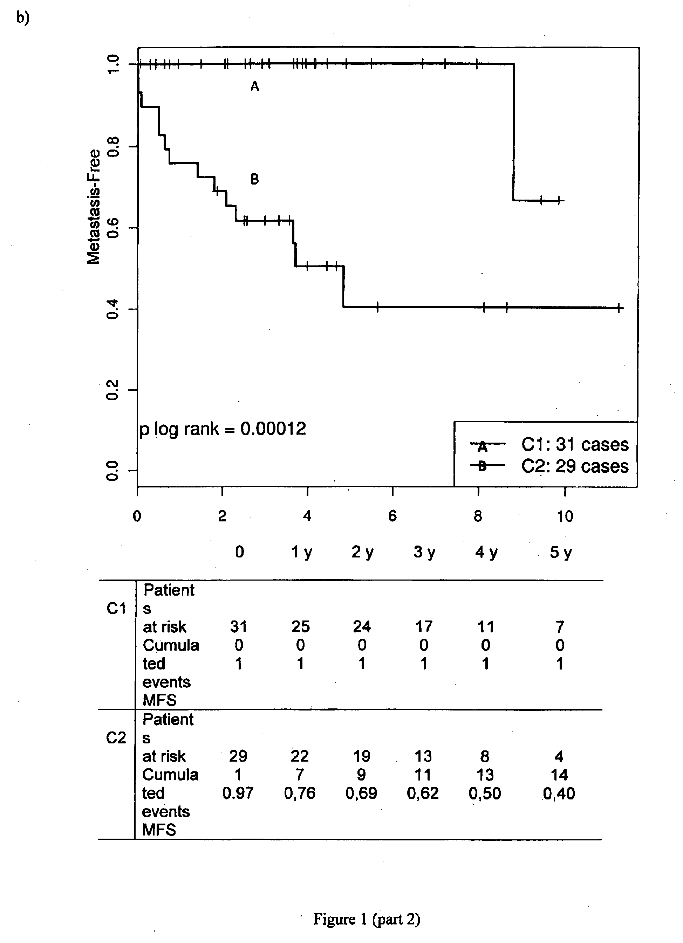

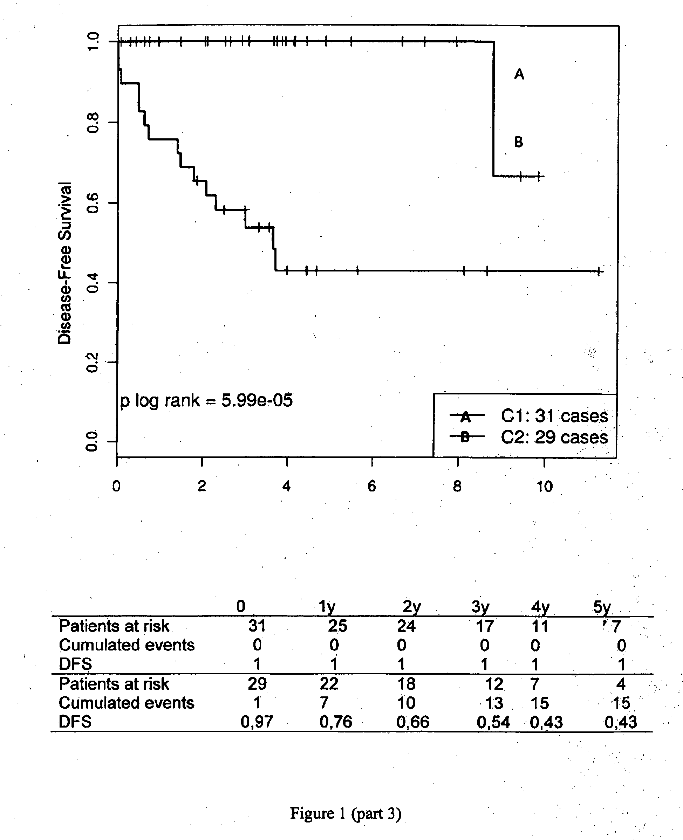

- Figure 1: Kaplan-Meyer analysis of metastasis-free (MFS) and disease-free (DFS) survival according to CINSARC stratification. Centroids have been retrained in a previously published (Yamaguchi *et al*, 2008) series of 32 GISTs (a) and then applied to the present series of 60 GISTs (b).

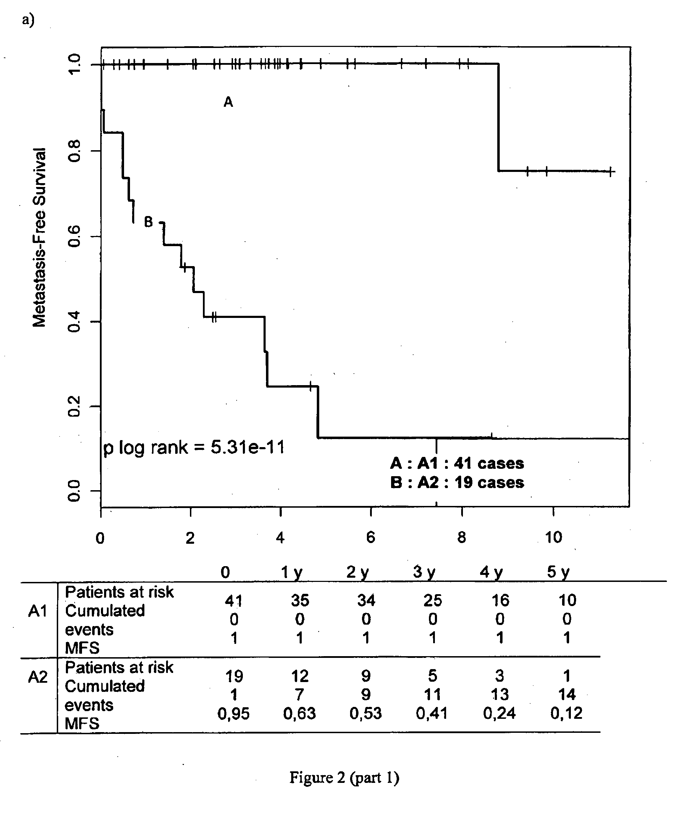

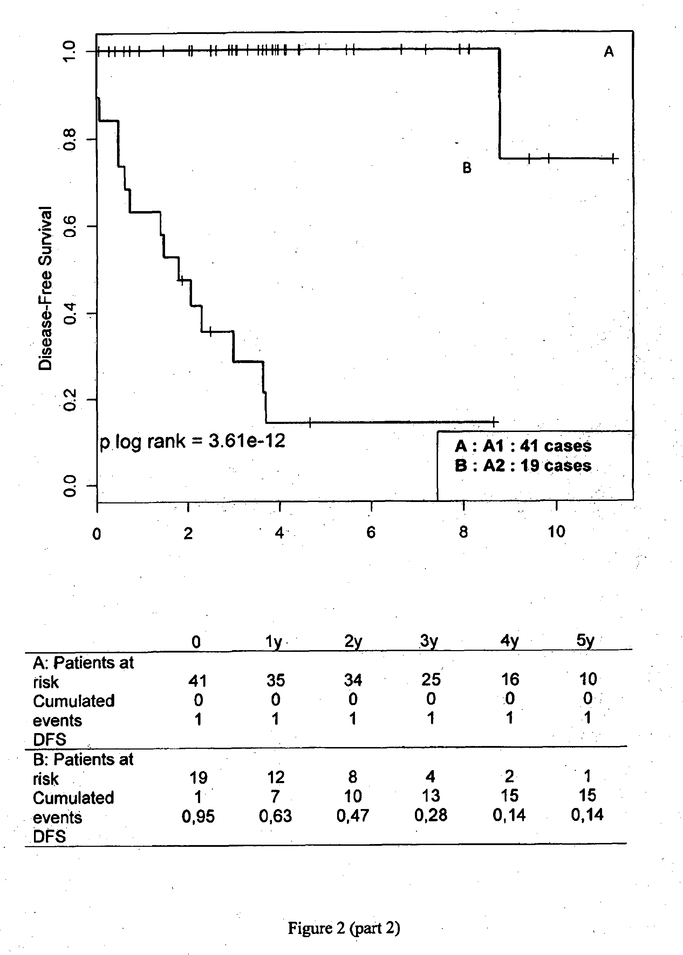

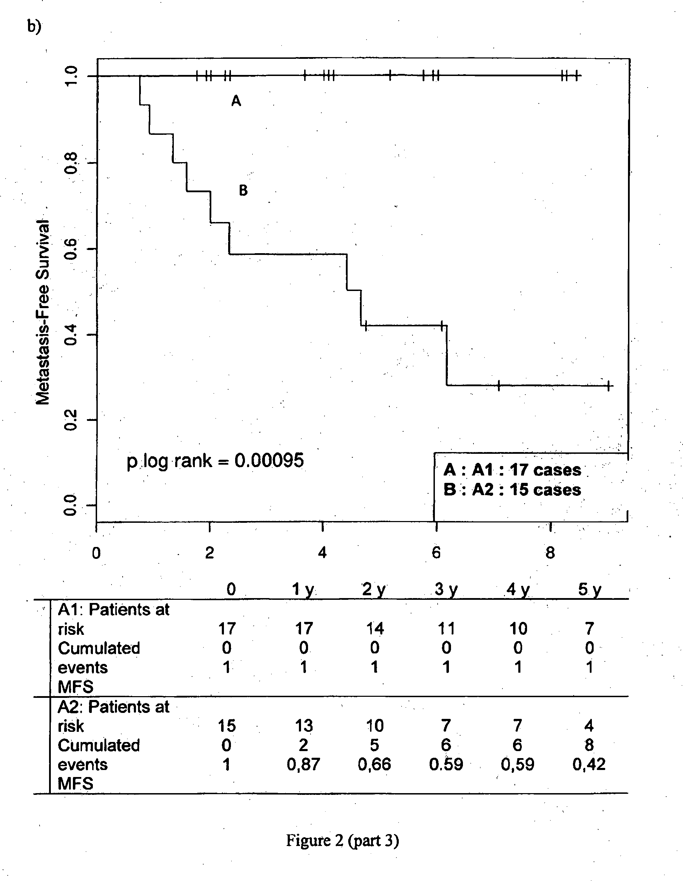

- Figure 2: Kaplan-Meyer analysis of metastasis-free (MFS) and disease-free (DFS) survival according to *AURKA* expression. *AURKA* has been identified in the present 60-GISTs series, this series is considered as (a) the "training set" and the Yamaguchi's series as (b) the "validation set". A1 corresponds to tumors with below-average *AURKA* expression.

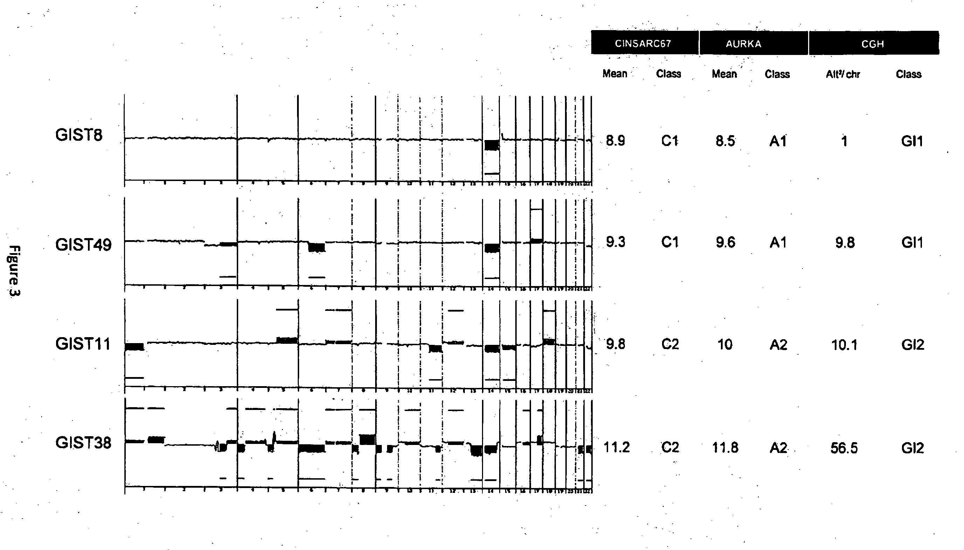

- Figure 3: CGH profiles of four cases representing GISTs with very few rearrangements (GIST #8), GISTs moderately rearranged (GISTs #49 and #11) and GISTs highly rearranged (GIST #38). Genomic alterations are presented and organized from chromosome 1 to 22 on the X axis and log ratio values are reported on the Y axis. Significant gains or losses are indicated by blue lines and blue areas above or below each profile, respectively.

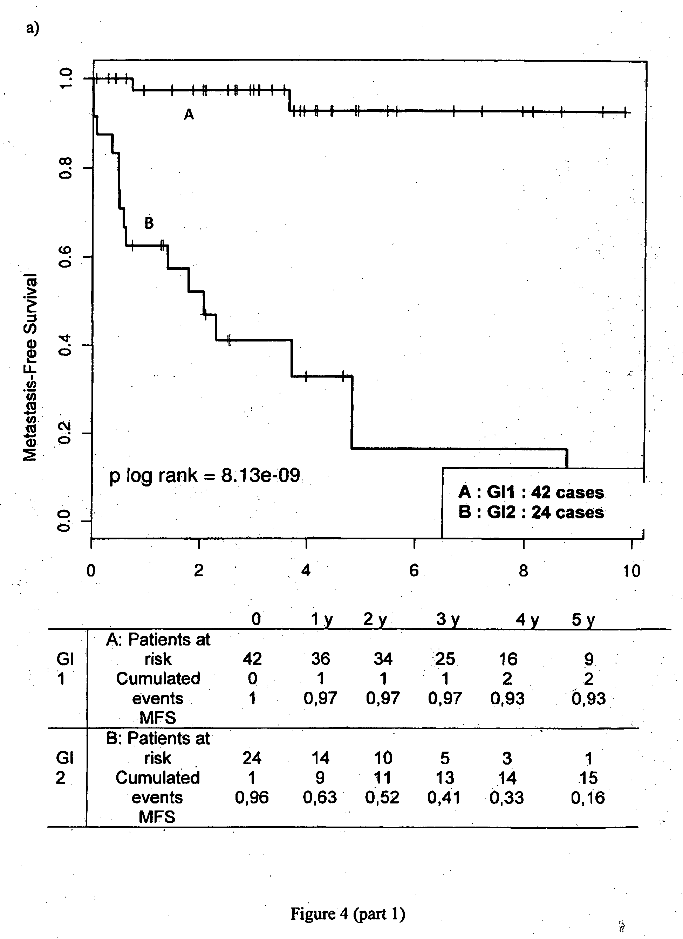

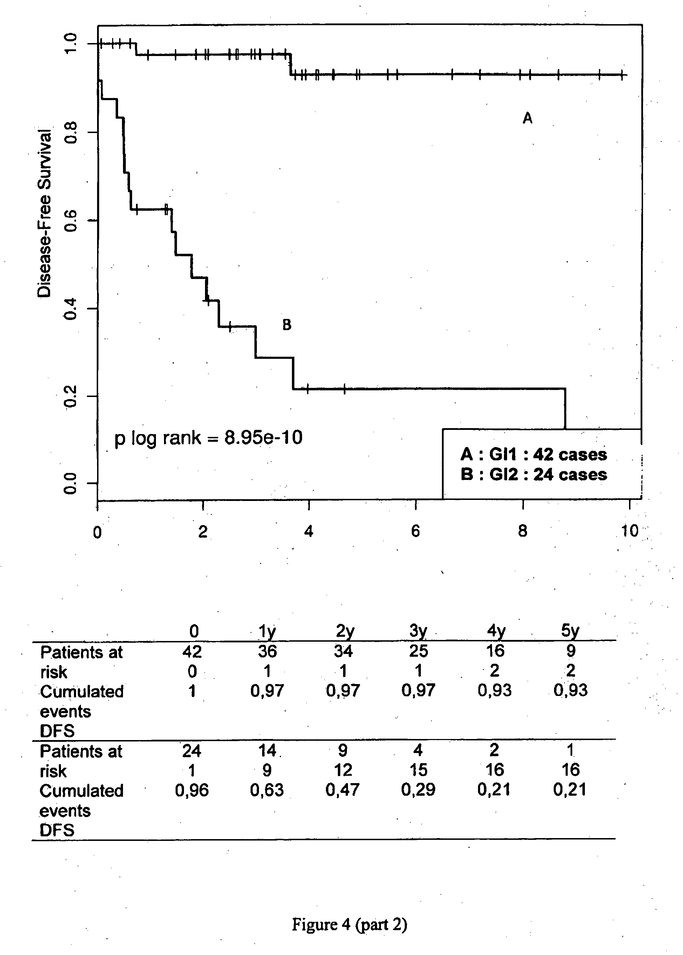

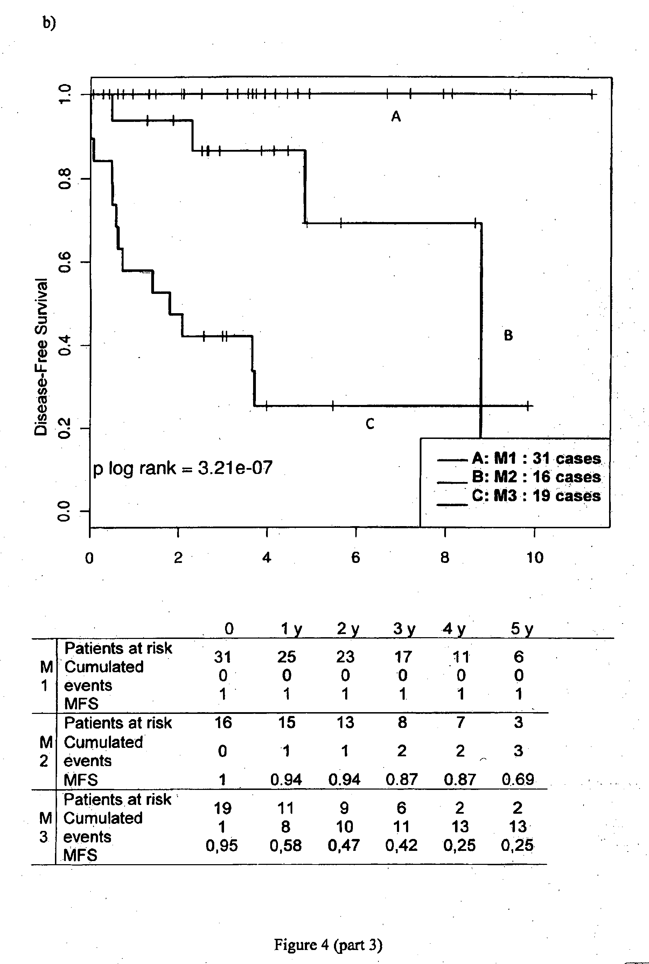

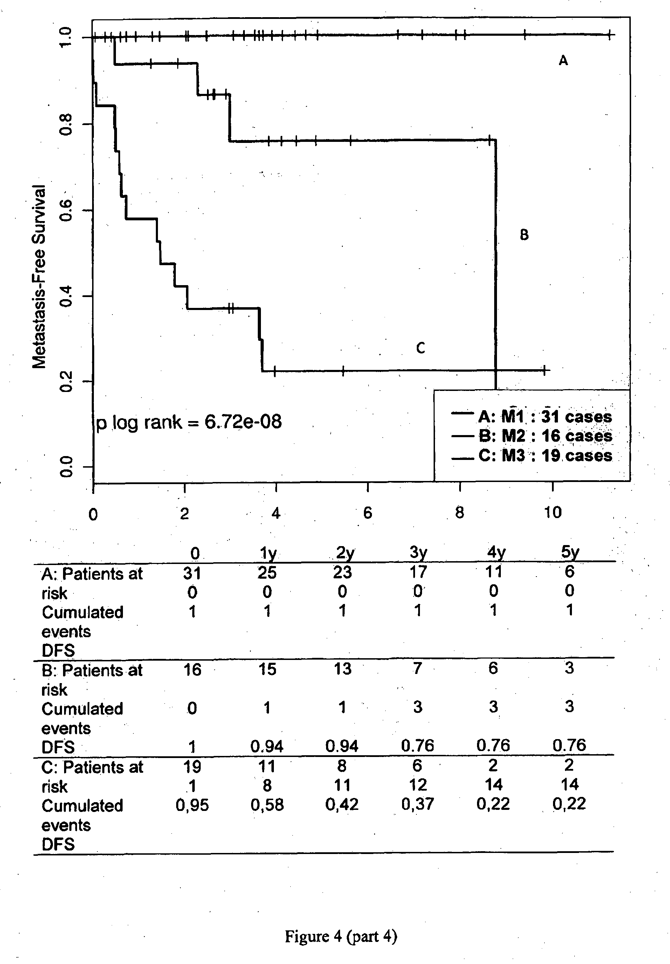

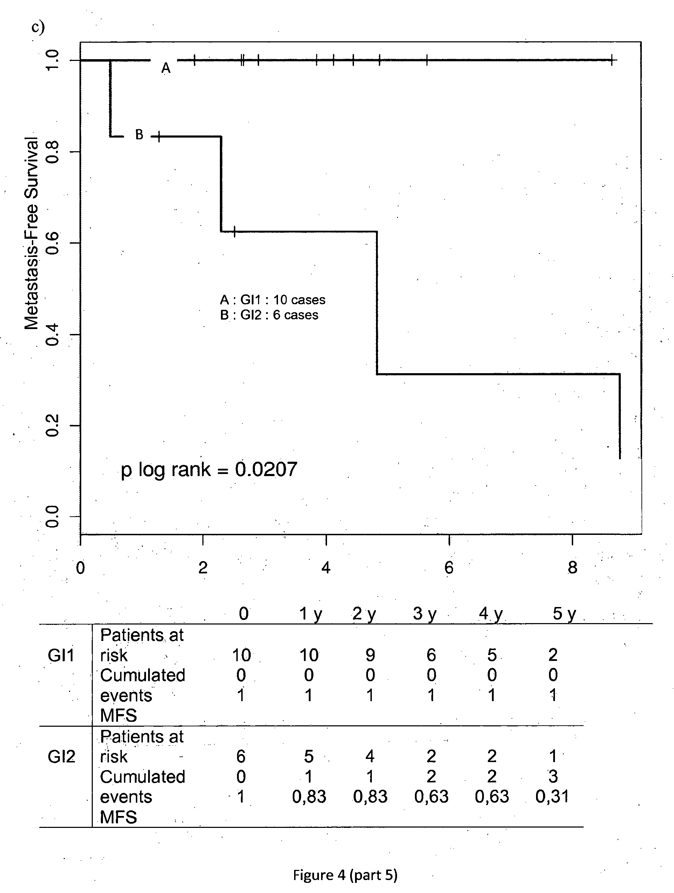

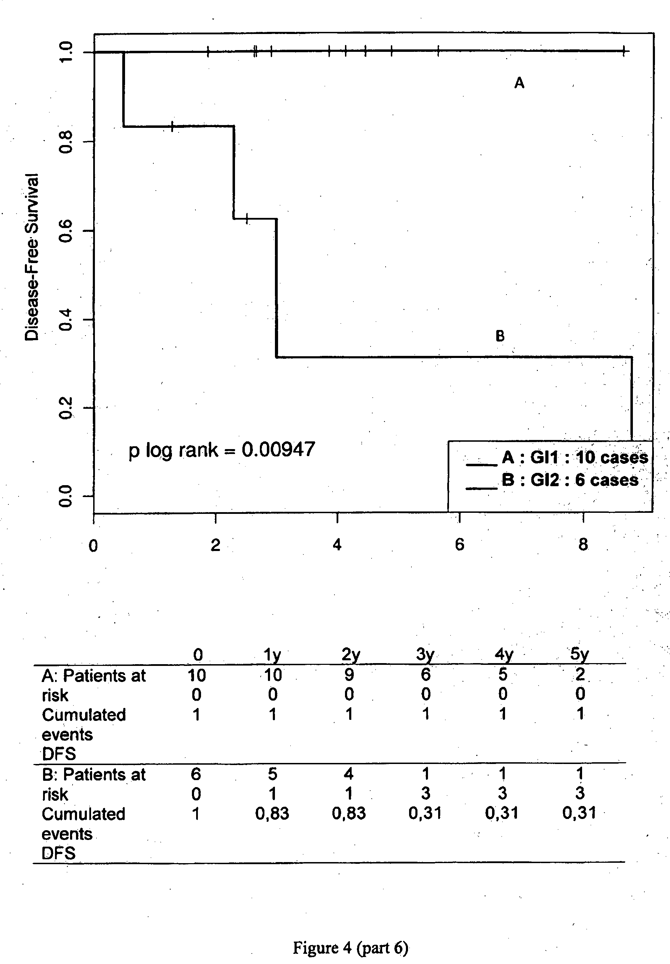

- Figure 4: Kaplan-Meyer analysis of metastasis-free (MFS) and disease-free (DFS) survival according to (a) GI, (b) AFIP classification and (c) GI in the subgroup of AFIP intermediate cases.

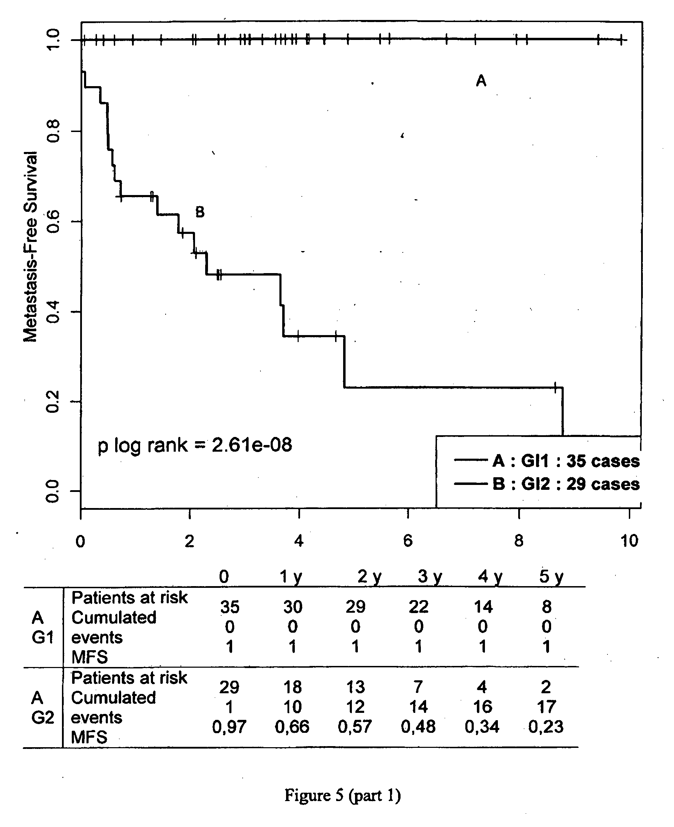

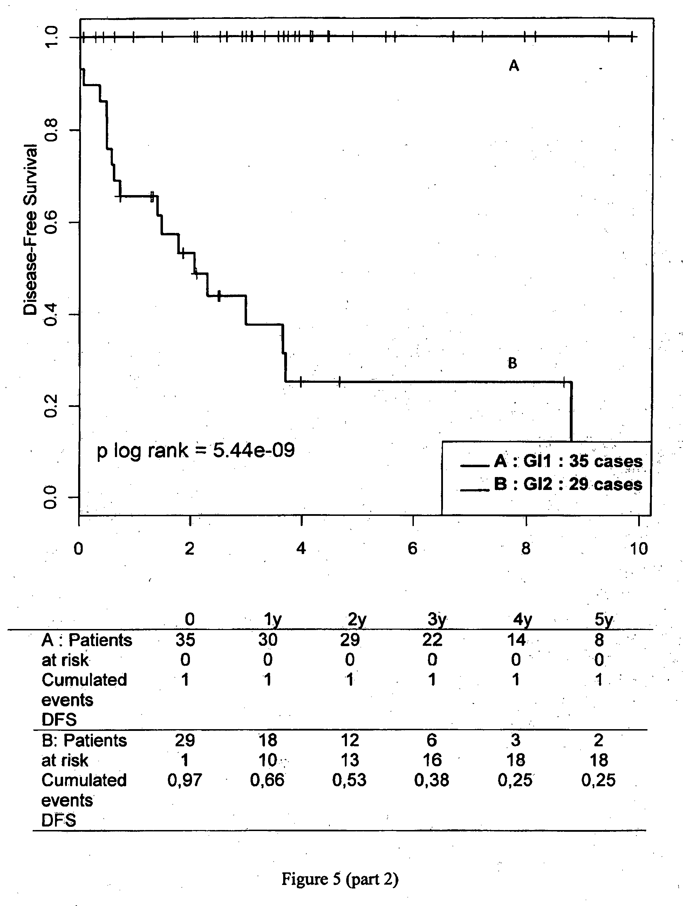

- Figure 5: Kaplan-Meyer analysis of metastasis-free (MFS) and disease-free (DFS) survival according to both GI and *AURKA* expression. **AG1** corresponds to tumors with GI below 10 and *AURKA* expression below average. **AG2** corresponds to tumors with GI over 10 and *AURKA* expression above average.

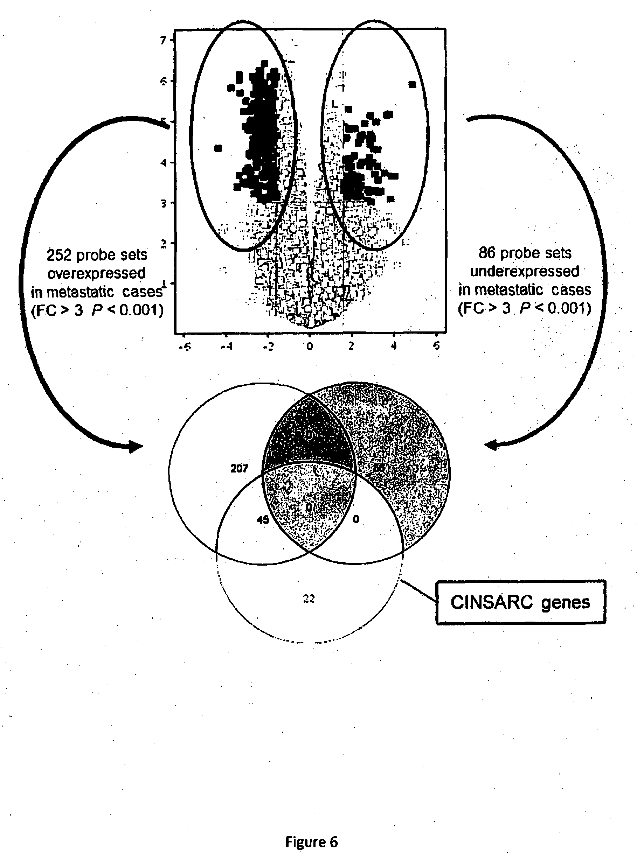

- Figure 6: Volcano-plot representation of t-test comparing expression profiles of GISTs with or without metastasis. Venn diagram at the bottom indicates the number of genes overlapping with CINSARC signature.

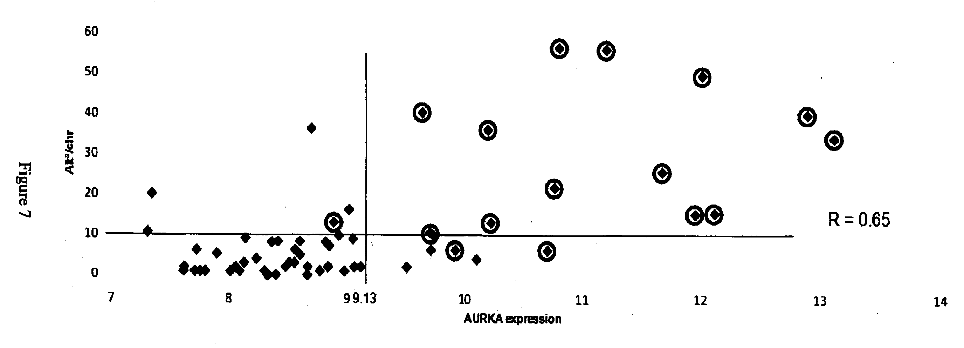

- Figure 7: Scatter-plot presenting the association between Genomic Index (GI, Y axis) and *AURKA* expression (log2, X axis). Horizontal and vertical **black** lines correspond to GI threshold of 10 and to *AURKA* mean expression, respectively. r = Pearson correlation coefficient. **Black** circles indicate metastatic cases.

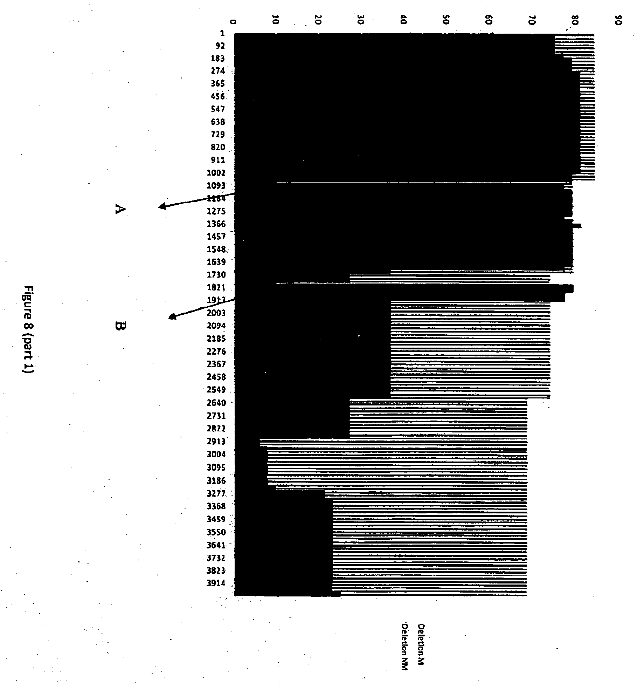

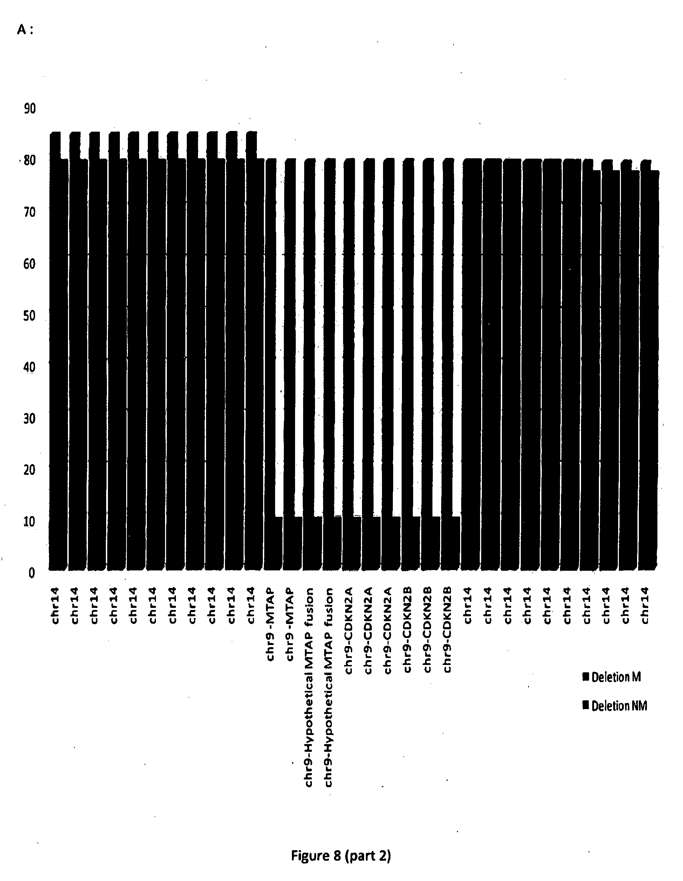

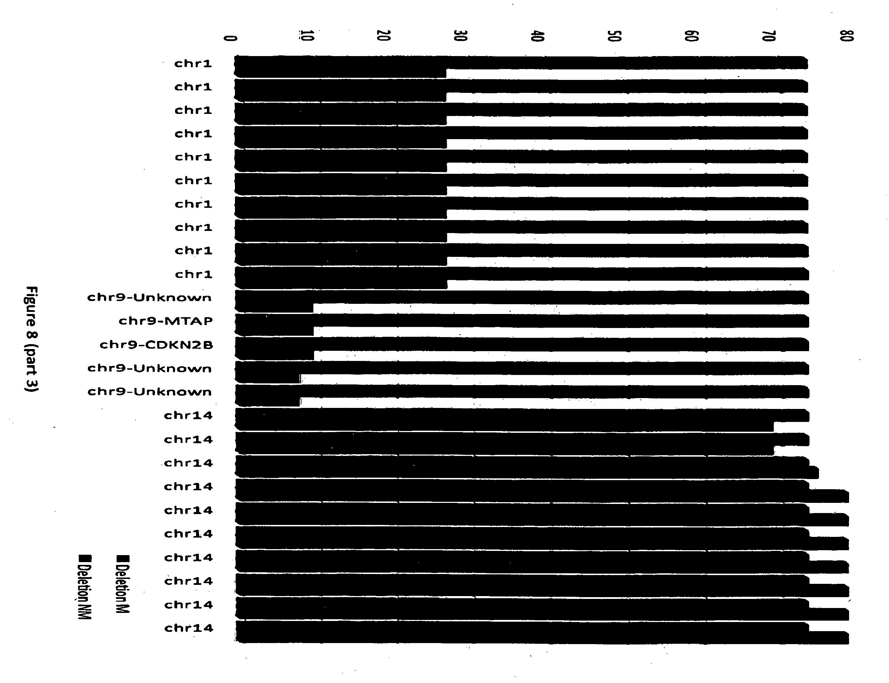

- Figure 8: Histogram presenting the 4000 more frequently deleted probe sets in metastatic (M) cases (blue). Corresponding frequencies for non-metastatic (NM) cases are in red. Y axes represent the deletion frequency. Bottom panels are detailed views of the probe sets with the highest differences between M and NM cases.

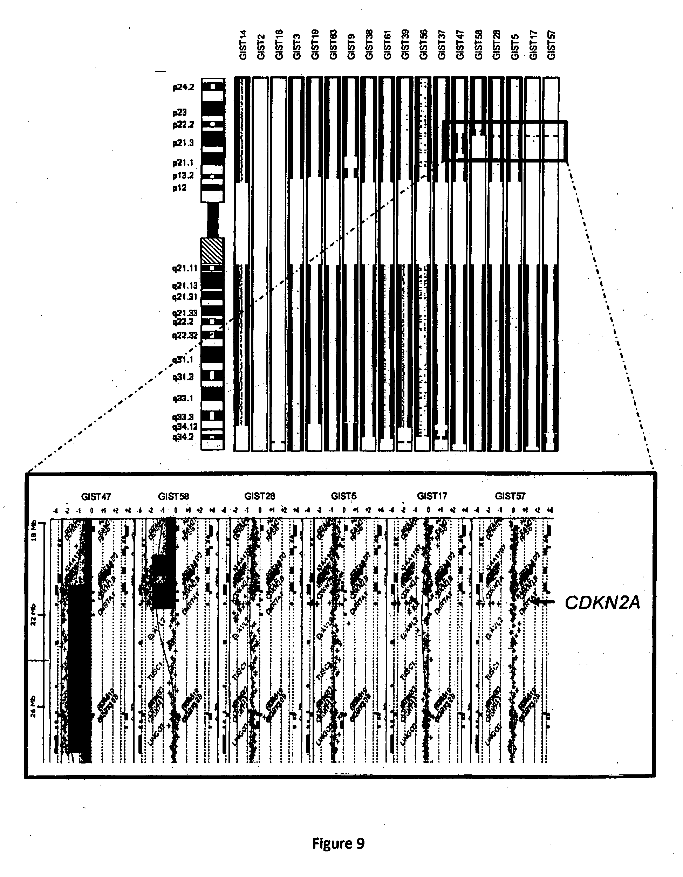

- Figure 9: Chromosome 9 genomic profile of the 18 metastatic GISTs (upper panel). Deletions and gains are indicated in green and red, respectively; and color intensity is proportional to copy number changes. A detailed view is given (bottom panel) for the 6 cases presenting a homozygous 9p21 deletion targeting *CDKN2A* locus (dark green).

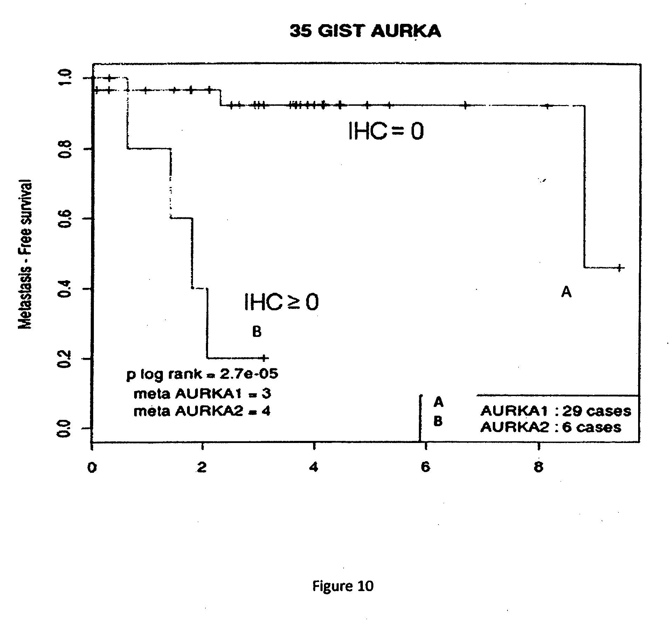

- Figure 10: prognostic values of protein expression of AURKA gene. Kaplan-Meyer analysis of metastasis-free (MFS) survival according to *AURKA* expression. The expression of the AURKA protein has been measured after specific hybridization with an antibody recognizing specifically AURKA protein. The hybridization of the antibody was then revealed by a chromogenic process.

**Examples**

**Example 1: Material and methods**

***Tumor samples***

Sixty seven fresh frozen GIST tumors were selected from the European GIST database CONTICAGIST (www.conticagist.org) which contains data from GIST tissues, including information regarding patients, primary tumor, treatment, follow-up and availability of tumor samples. All GISTs selected were primary untreated tumors. Their characteristics are presented in supplementary table 1. Most GISTs (59/67) were studied by both CGH-array and gene expression profiling (a combination of 66 by CGH-array and 60 by gene expression profiling).

***DNA isolation and array-CGH***

(Agilent arrays (G4450A). Briefly, for each sample, 350 ng of DNA were fragmented by a double enzymatic digestion (AluI + RsaI) and checked with LabOnChip (2100 Bioanalyzer System, Agilent Technologies) before labeling and hybridization. Tumor and control DNAs were labeled by random priming with CY5-dUTPs and CY3-dUTP, respectively, hybridized at 65°C for 24h and rotating at 20 rpm. Arrays were scanned using an Agilent G2585CA DNA Microarray Scanner and image analysis was done using the Feature-Extraction V10.1.1.1 software (Agilent Technologies). Normalization was done using the ranking-mode method available in the Feature-Extraction V10.1.1 software, with default value for any parameter. Raw copy number ratio data were transferred to CGH Analytics v4.0.76 software. The ADM-2 algorithm of CGH Analytics v4.0.76 software (Agilent) was used to identify DNA copy number anomalies at the probe level. A low-level copy number gain was defined as a log 2 ratio >0.25 and a copy number loss was defined as a log 2 ratio <-0.25. A high-level gain or amplification was defined as a log 2 ratio >1.5 and a homozygous deletion is suspected when ration is below -1. To establish decision criteria for prognosis, alterations involving more than 100 probes have been automatically computed using an aberration filter.

***Real-time genomic quantitative PCR and sequencing***

To determine the copy number status of *p16, p15* and *p14,* real-time PCR was performed on genomic DNA using TaqMan® Universal Master Mix (Applied Biosystems). For normalizing the results, we used three reference genes: *GAPDH, ALB* and *RPLP0,* in order to have, for each tumor, at least two of these reference genes in normal copy number (array-CGH). Primers and probe used for *RPLP0* are as follow: (F) 5'-TGGATCTGCTGGTTGTCCAA-3' (SEQ ID NO: 3); (R) 5'-CCAGTCTTGATCAGCTGCACAT-3' (SEQ ID NO: 4); (probe) 5'-AGGTGTTTACTGCCCCACTATTATCTGGTTCAGA-3' (SEQ ID NO: 5). Other primers and probes used were previously described (52). Tumor data were normalized against data obtained for normal DNA. The results were then calculated as previously described (52). A normal status corresponds to $0.8 \leq$ ratio $\leq 1.2$, $0.1 <$ ratio $< 0.8$ is considered as a hemizygous deletion. When ratio is inferior to 0.1, the deletion is considered as homozygous. *CDKN2A* locus has been submitted to sequencing as previously described ([52]) and *RB1* gene was sequenced using genomic DNA according to Houdayer *et al* (53) or cDNA with following primers : (F1) 5'-TCATGTCAGAGAGAGAGCTTGG-3' (SEQ ID NO: 6), (R1) 5'-CGTGCACTCCTGTTCTGACC-3' (SEQ ID NO: 7); (F2) 5'-AATGGTTCACCTCGAACACC -3' (SEQ ID NO: 8), (R2) 5'-CTCGGTAATACAAGCGAACTCC-3' (SEQ ID NO: 9); (F3) 5'-CCTCCACACACTCCAGTTAGG-3' (SEQ ID NO: 10), (R3) 5'-TGATCAGTTGGTCCTTCTCG-3' (SEQ ID NO: 11); (F4) 5'-GCATGGCTCTCAGATTCACC-3' (SEQ ID NO: 12), (R4) 5'-TCGAGGAATGTGAGGTATTGG-3' (SEQ ID NO: 13); (F5) 5'-TCTTCCTCATGCTGTTCAGG-3' (SEQ ID NO: 14), (R5) 5'-TGTACACAGTGTCCACCAAGG-3' (SEQ ID NO: 15).

***RNA Isolation and gene expression profiling by one color assay***

Total RNAs were extracted from frozen tumor samples with TRIzol reagent (Life Technologies, Inc.) and purified using the RNeasy® Min Elute TM Cleanup Kit (Qiagen) according to the manufacturer's procedures. RNA quality was checked on an Agilent 2100 bioanalyzer (Agilent Technologies). RNAs with a RNA Integrity Number (RIN) > 6.5 were used for microarray.

Gene expression analysis was carried out using Agilent Whole human 44K Genome Oligo Array (Agilent Technologies). This specific array represents over 41 000 human genes and transcripts, all with public domain annotations. Total RNA (500 ng) was reverse transcribed into cRNA by incorporating a T7 oligo-dT promoter primer prior to the generation of fluorescent cRNA using an Agilent Quick Amp Labelling Kit (Agilent Technologies). The labeled cRNA was purified using a Qiagen RNeasy Mini Kit (Qiagen) and quantified using a NanoDrop ND-1000 instrument. In these experiments Cy3-labeled (sample) cRNAs were hybridized to the array using a Gene Expression Hybridization Kit (Agilent Technologies). The hybridization was incubated in Agilent SureHyb chambers for 17 hours in a Hyb Oven set to 65°C and rotating at 10 rpm. The microarray slides were washed according to the manufacturer's instructions and then scanned on an Agilent G2565BA DNA Microarray Scanner and image analysis was done using the Feature-Extraction V10.1.1.1 software (Agilent Technologies).

All microarray data were simultaneously normalized using the Quantile algorithm. The t-test was performed using Genespring (Agilent Technologies) and P-values were adjusted using the Benjamini-Hochberg procedure. The P-value and fold change cut-off for gene selection were 0.001 and 3, respectively. Gene ontology analysis was performed to establish statistical enrichment in GO terms using Genespring (Agilent Technologies).

*Real-Time PCR*

Reverse transcription and real-time PCR were performed as previously described (52). We used TaqMan® Gene Expression assays (Applied Biosystems): Hs01582072_m1 for *AURKA*; Hs01078066_m1 for *RB1*; Hs99999905_m1 for *GAPDH*; Hs99999903_m1 for *ACTB* and Hs99999902_m1 for *RPLP0*. *p14* and *p16* expression level was assessed as previously described (52). In order to normalize the results, we used *GAPDH, ACTB* and *RPLP0* genes as reference genes. Triplicates were performed for each sample for each gene. A reference CT (threshold cycle) for each sample was defined as the average measured CT of the three reference genes. Relative mRNA level of AURKA in a sample was defined as: $\Delta CT = CT$ (gene of interest) $-CT$ (mean of the three reference genes).

*Statistical analysis.*

To assign prognosis, we applied the nearest centroid method. Centroids represent a centered mean of expression for the signature genes for each patient outcome (metastatic and non-metastatic). Thus, centroids were calculated from the cohort 1 samples (17) and then each sample of our series (thus considered as a validation set) was allocated to the prognostic class (centroid) with the highest Spearman correlation.

Metastasis- and disease- free survival was calculated by the Kaplan-Meier method from the date of initial diagnosis to the date of first metastasis, relapse, last follow-up or death for patients within diagnosis of metastasis. Survival curves were compared with the log rank test. Hazard ratios were performed with the Cox proportional hazard model. All statistical analyses were performed using R software version 2.11.11 and the package "survival".

**Example 2: Results**

*CINSARC is a significant prognosis factor in GISTs*

To assess the issue whether our previously published signature could have prognostic value in GISTs, we performed expression profiling in a series of 67 GISTs (Table 1).

**Table 1: Description of patients.**

Percentages are indicated in brackets. nd = not determined

| | |
|---|---|
| **Sex** | |
| Male | 27 (40) |
| Female | 40 (60) |
| | |
| **Location** | |
| Stomach | 43 (64) |
| Small intestine | 12 (18) |
| Other | 12 (18) |
| | |
| **Histological subtype** | |
| Spindle | 52 (77.5) |
| Epithelioid | 5 (7.5) |
| Mixed | 10 (15) |
| | |
| **Tumor size** | |
| ≤2 | 5 (7.5) |
| 2-5 | 25 (37) |
| 5-10 | 21 (31,5) |
| >10 | 15 (22,5) |
| nd | 1 (1.5) |
| | |
| **Mitotic index** | |
| ≤5 | 42 (63) |
| >5 | 25 (37) |
| | |
| **AFIP Risk** | |
| Very low | 15 (22) |
| Low | 16 (24) |
| Intermediate | 16 (24) |
| High | 19 (28.5) |
| nd | 1 (1.5) |
| **Surgery margin** | |
| R0 | 46 (69) |
| R1 | 4 (6) |
| nd | 17 (25) |
| **Mutations** | |
| *KIT* | 52 (77.5) |
| Ex 9 | 2 (3) |
| Ex 11 | 48 (71.5) |
| Ex 13 | 1 (1.5) |
| EX 17 | 1 (1.5) |
| *PDGFRA* | 12 (18) |
| Ex 12 | 2 (3) |
| Ex 14 | 1 (1.5) |
| Ex 18 | 9 (13.5) |
| *WT* | 3 (4.5) |
| **Relapse events** | |
| Local | 7 (10) |
| Distance | 18 (27) |

Among them, we obtained sufficient mRNA quality for 60 cases (89.5%). We applied the CINSARC nearest-centroid signature (18) to GISTs, using a published series (17) as a training set to retrain centroids and the present series as the validation set. Kaplan-Meier analysis (figure 1) revealed that in both series the CINSARC signature split tumors into two groups with strongly distinct metastasis-free (MFS) and disease-free (DFS) survival (validation set: MFS:

HR=18.3, 95%CI = [2.4 - 140], *P* = 0.005 and DFS: HR=19.6, 95%CI = [2.6 - 149.5], P = 0.004).

### *Gene expression changes associated with metastatic outcome*

The results presented above indicate that expression of genes involved in mitosis control and chromosome integrity (CINSARC) is associated with survival outcomes in GISTs. We thus asked whether the reciprocal phenomenon was true, that is whether the differential expression between metastatic and non-metastatic cases can identify such genes. To assess this issue, we performed supervised t-test comparing tumor expression profiles stratified according to outcomes (Figure 6). Among the 297 differentially expressed genes (338 probe sets) (Table 2), 70 (86 probe sets) were down-regulated in metastatic cases and 227 (252 probe sets) were up-regulated in metastatic cases (FC > 3 and *P* < 0.001).

**Table 2**: 297 genes differentially expressed between GISTs with or without metastasis (t-test).

UP-regulated genes in metastatic GISts

| Probe Name | Corrected p-value | Fold Change | Genbank Accession | Gene Symbol | Description |
|---|---|---|---|---|---|
| A_23_P71558 | 9.44E-08 | 4.4 | NM_004260 | RECQL4 | Homo sapiens RecQ protein-like 4 (RECQL4), mRNA [NM_004260] |
| A_23_P104651 | 3.83E-07 | 4.6 | NM_080668 | CDCA5 | Homo sapiens cell division cycle associated 5 (CDCA5), mRNA [NM_080668] |
| A_23_P131866 | 5.70E-07 | 5.2 | NM_198433 | AURKA | Homo sapiens aurora kinase A (AURKA), transcript variant 1, mRNA [NM_198433] |
| A_23_P168747 | 5.91E-07 | 3.3 | NM_017760 | NCAPG2 | Homo sapiens non-SMC condensin II complex, subunit G2 (NCAPG2), mRNA [NM_017760] |
| A_23_P333998 | 5.91E-07 | 5.5 | AF090919 | POLQ | Homo sapiens clone HQ0327 PRO0327 mRNA, complete cds. [AF090919] |
| A_23_P32707 | 5.95E-07 | 5.1 | M_012291 | ESPL1 | Homo sapiens extra spindle pole bodies homolog 1 (S. cerevisiae) (ESPL1), mRNA [NM_012291] |
| A_32_P103633 | 5.95E-07 | 3.2 | NM_004526 | MCM2 | Homo sapiens MCM2 minichromosome maintenance deficient 2, mitotin (S. cerevisiae) (MCM2), mRNA [NM_004526] |
| A_23_P29330 | 7.06E-07 | 10.5 | NM_148674 | SMC1 B | Homo sapiens structural maintenance of chromosomes 1B (SMC1 B), mRNA [NM_148674] |
| A_24_P277576 | 7.78E-07 | 6.5 | NM_004237 | TRIP13 | Homo sapiens thyroid hormone receptor interactor 13 (TRIP13), mRNA [NM_004237] |
| A_23_P385861 | 7.78E-07 | 5.7 | NM_152562 | CDCA2 | Homo sapiens cell division cycle associated |
| A_23_P145657 | 7.78E-07 | 6.8 | M_012447 | STAG3 | 2 (CDCA2), mRNA [NM_152562] Homo sapiens stromal antigen 3 (STAG3), mRNA [NM_012447] |
| A_23_P7636 | 7.78E-07 | 5.3 | NM_004219 | PTTG1 | Homo sapiens pituitary tumor-transforming 1 (PTTG1), mRNA [NM_004219] |
| A_24_P195454 | 7.78E-07 | 3.8 | A_24_P195454 | A_24_P195454 | |
| A_23_P212844 | 7.78E-07 | 3.0 | NM_006342 | TACC3 | Homo sapiens transforming, acidic coiled-coil containing protein 3 (TACC3), mRNA [NM_006342] |
| A_23_P18579 | 9.70E-07 | 5.2 | NM_006607 | PTTG2 | Homo sapiens pituitary tumor-transforming 2 (PTTG2), mRNA [NM_006607] |
| A_23_P68610 | 9.70E-07 | 6.3 | NM_012112 | TPX2 | Homo sapiens TPX2, microtubule-associated, homolog (Xenopus laevis) (TPX2), mRNA [NM_012112] |
| A_24_P507383 | 9.70E-07 | 10.6 | THC2705254 | THC2705254 | ALU2_HUMAN (P39189) Alu subfamily SB sequence contamination warning entry, partial (13%) [THC2705254] |
| A_23_P74349 | 1.09E-06 | 4.7 | NM_145697 | NUF2 | Homo sapiens NUF2, NDC80 kinetochore complex component, homolog (S. cerevisiae) (NUF2), transcript variant 1, mRNA [NM_145697] |
| A_23_P218827 | 1.15E-06 | 4.5 | NM_199420 | POLQ | Homo sapiens polymerase (DNA directed), theta (POLQ), mRNA [NM_199420] |
| A_23_P302654 | 1.16E-06 | 3.2 | NM_018140 | CEP72 | Homo sapiens centrosomal protein 72kDa (CEP72), mRNA [NM_018140] |

UP-regulated genes in metastatic GISts

| Probe Name | Corrected p-value | Fold Change | Genbank Accession | Gene Symbol | Description |
|---|---|---|---|---|---|
| A_24_P942335 | 1.40E-06 | 6.0 | BC002881 | C15orf42 | Homo sapiens chromosome 15 open reading frame 42, mRNA (cDNA clone IMAGE:3940845), partial cds. [BC002881] |
| A_23_P253661 | 1.48E-06 | 13.7 | NM_024902 | FLJ13236 | Homo sapiens hypothetical protein FLJ13236 (FLJ13236), mRNA [NM_024902] |
| A_23_P122197 | 1.52E-06 | 3.4 | NM_031966 | CCNB1 | Homo sapiens cyclin B1 (CCNB1), mRNA [NM_031966] |
| A_32_P188921 | 1.58E-06 | 4.2 | BC007606 | BC007606 | Homo sapiens cDNA clone IMAGE:3351130, complete cds. [BC007606] |
| A_23_P429491 | 1.58E-06 | 3.9 | NM_145018 | FLJ25416 | Homo sapiens hypothetical protein FLJ25416 (FLJ25416), mRNA [NM_145018] |
| A_23_P340909 | 1.58E-06 | 5.1 | BC013418 | C13orf3 | Homo sapiens chromosome 13 open reading frame 3, mRNA (cDNA clone MGC:4832 IMAGE:3604003), complete cds. [BC013418] |
| A_23_P375 | 1.58E-06 | 4.7 | NM_018101 | CDCA8 | Homo sapiens cell division cycle associated 8 (CDCA8), mRNA [NM_018101] |
| A_24_P105102 | 1.58E-06 | 3.5 | NM_182687 | PKMYT1 | Homo sapiens protein kinase, membrane associated tyrosine/threonine 1 (PKMYT1), transcript variant 2, mRNA [NM_182687] |
| A_23_P361419 | 1.58E-06 | 5.6 | NM_018369 | DEPDC1B | Homo sapiens DEP domain containing 1 B (DEPDC1 B), mRNA [NM_018369] |
| A_23_P133956 | 1.58E-06 | 4.8 | NM_002263 | KIFC1 | Homo sapiens kinesin family member C1 (KIFC1), mRNA [NM_002263] |
| A_23_P124417 | 1.93E-06 | 5.4 | NM_004336 | BUB1 | Homo sapiens BUB1 budding uninhibited by benzimidazoles 1 homolog (yeast) (BUB1), mRNA [NM_004336] |
| A_24_P306704 | 2.01E-06 | 10.1 | XR_016161 | KRT18P23 | PREDICTED: Homo sapiens similar to Keratin, type I cytoskeletal 18 (Cytokeratin-18) (CK-18) (Keratin-18) (K18) (LOC642448), mRNA [XR_016161] |
| A_24_P113144 | 2.01E-06 | 3.1 | NM_024857 | ATAD5 | Homo sapiens ATPase family, AAA domain containing 5 (ATAD5), mRNA [NM_024857] |
| A_24_P297539 | 2.06E-06 | 6.4 | NM_181803 | UBE2C | Homo sapiens ubiquitin-conjugating enzyme E2C (UBE2C), transcript variant 6, mRNA [NM_181803] |
| A_24_P413884 | 2.08E-06 | 5.8 | NM_001809 | CENPA | Homo sapiens centromere protein A (CENPA), transcript variant 1, mRNA [NM_001809] |
| A_23_P401 | 2.15E-06 | 4.3 | NM_016343 | CENPF | Homo sapiens centromere protein F, 350/400ka (mitosin) (CENPF), mRNA [NM_016343] |
| A_24_P76521 | 2.17E-06 | 4.9 | AK056691 | GSG2 | Homo sapiens cDNA FLJ32129 fis, clone PEBLM2000213, weakly similar to Mus musculus genes for integrin aM290, hapsin. [AK056691] |
| A_32_P151800 | 2.36E-06 | 3.5 | NM_207418 | FAM72A | Homo sapiens family with sequence similarity 72, member A (FAM72A), mRNA [NM 207418] |

UP-regulated genes in metastatic GISts

| Probe Name | Corrected p-value | Fold Change | Genbank Accession | Gene Symbol | Description |
|---|---|---|---|---|---|
| A_23_P150935 | 2.44E-06 | 5.4 | NM_005480 | TROAP | Homo sapiens trophinin associated protein (tastin) (TROAP), mRNA [NM_005480] |
| A_24_P354300 | 2.96E-06 | 3.3 | NM_015.426 | WDR51A | Homo sapiens WD repeat domain 51 A (WDR51A), mRNA [NM_0.15426] |
| A_32_P217911 | 2.96E-06 | 3.8 | BG951379 | BG951379 | MR1-CT0735-120101-003-h03 CT0735 Homo sapiens cDNA, mRNA sequence [BG951379] |
| A_24_P319613 | 3.11E-06 | 8.2 | NM_002497 | NEK2 | Homo sapiens NIMA (never in mitosis gene a)-related kinase 2 (NEK2), mRNA [NM_002497] |
| A_24_P313504 | 3.16E-06 | 3.2 | NM_005030 | PLK1 | Homo sapiens polo-like kinase 1 (Drosophila) (PLK1), mRNA [NM_005036] |
| A_23_P1.43190 | 3.30E-06 | 4.0 | NM_002466 | MYBL2 | Homo sapiens v-myb myeloblastosis viral oncogene homolog (avian)-like 2 (MYBL2), mRNA [NM_002466] |
| A_23_P6001.6 | 3.43E-06 | 5.7 | NR_002734 | PTTG3 | Homo sapiens pituitary tumor-transforming 3 (PTTG3) on chromosome 8 [NR_002734] |
| A_32_P96719 | 3.61E-06 | 4.3 | NM_024745 | SHCBP1 | Homo sapiens SHCSH2-domain binding protein 1 (SHCBP1), mRNA [NM_024745] |
| A_23_P133123 | 3.63E-06 | 3.1 | NM_032117 | MND1 | Homo sapiens meiotic nuclear divisions 1 homolog (S. cerevisiae) (MND1), mRNA [NM_032117] |
| A_23_P118815 | 3.63E-06 | 6.1 | NM_001012271 | BIRC5 | Homo sapiens baculoviral IAP repeat-containing 5 (survivin) (BIRC5), transcript variant 3, mRNA [NM_001012271] |
| A_24_P323598 | 3.63E-06 | 4.8 | NM_001017420 | ESCO2 | Homo sapiens establishment of cohesion 1 homolog 2 (S. cerevisiae) (ESCO2), mRNA [NM_001017420] |
| A_24_P227091 | 3.63E-06 | 3.7 | NM_004523 | KIF11 | Homo sapiens kinesin family member 11 (KIF11), mRNA [NM_004523] |
| A_24_P322354 | 3.63E-06 | 6.3 | NM_145060 | C18orf24 | Homo sapiens chromosome 18 open reading frame 24 (C18orf24), transcript variant 2, mRNA [NM_145060] |
| A_23_P96325 | 3.63E-06 | 6.5 | NM_001009954 | FLJ20105 | Homo sapiens FLJ20105 protein (FLJ20105), transcript variant 2, mRNA [NM_001009954] |
| A_23_P253752 | 3.86E-06 | 3.5 | NM_138419 | FAM54A | Homo sapiens family with sequence similarity 54, member A (FAM54A), mRNA [NM_138419] |
| A_23_P80902 | 4.16E-06 | 4.5 | NM_020242 | KIF15 | Homo sapiens kinesin family member 15 (KIF15), mRNA [NM_020242] |
| A_23_P118174 | 4.45E-06 | 3.5 | NM_005030 | PLK1 | Homo sapiens polo-like kinase 1 (Drosophila) (PLK1), mRNA [NM_005030] |
| A_23_P415443 | 4.45E-06 | 3.5 | NM_015341 | NCAPH | Homo sapiens non-SMC condensin I complex, subunit H (NCAPH), mRNA [NM_015341] |
| A_24_P323434 | 4.47E-06 | 7.1 | NM_152562 | CDCA2 | Homo sapiens cell division cycle associated |

UP-regulated genes in metastatic GISts

| Probe Name | Corrected p-value | Fold Change | Genbank Accession | Gene Symbol | Description |
|---|---|---|---|---|---|
| A_24_P466231 | 4.91E-06 | 3.6 | THC2515749 | THC2515749 | 2 (CDCA2), mRNA [NM_152562] Q6NWY8_HUMAN (Q6NWY8) LOC146909 protein (Fragment), partial (29%) [THC2515749] |
| A_23_P51085 | 5.23E-06 | 6.0 | NM_020675 | SPC25 | Homo sapiens SPC25, NDC80 kinetochore complex component, homolog (S. cerevisiae) (SPC25), mRNA [NM_020675] |
| A_32_P407245 | 5.31E-06 | 4.0 | NM_024902 | FLJ13236 | Homo sapiens hypothetical protein FLJ13236 (FLJ13236), mRNA [NM_024902] |
| A_23_P138507 | 5.32E-06 | 5.5 | NM_001786 | CDC2 | Homo sapiens cell division cycle 2, G1 to S and G2 to M (CDC2), transcript variant 1, mRNA [NM_001786] |
| A_23_P49878 | 5.32E-06 | 7.2 | NM_019013 | FAM64A | Homo sapiens family with sequence similarity 64, member A(FAM64A), mRNA [NM_019013] |
| A_23_P345707 | 5.58E-06 | 5.6 | NM_152259 | C15orf42 | Homo sapiens chromosome 15 open reading frame 42 (C15orf42), mRNA [NM_152259] |
| A_23_P52017 | 5.62E-06 | 7.4 | NM_018136 | ASPM | Homo sapiens asp (abnormal spindle) homolog, microcephaly associated (Drosophila) (ASPM), mRNA [NM_018136] |
| A_23_P88630 | 5.62E-06 | 3.3 | NM_000057 | BLM | Homo sapiens Bloom syndrome (BLM), mRNA [NM_000057] |
| A_24_P680947 | 5.62E-06 | 9.1 | ENST00000335534 | LOC146909 | Homo sapiens hypothetical protein LOC146909, mRNA (cDNA clone IMAGE:4418755), partial cds. [BC048263] |
| A_32_P109296 | 5.67E-06 | 5.8 | NM_152259 | C15orf42 | Homo sapiens chromosome 15 open reading frame 42 (C15orf42), mRNA [NM_152259] |
| A_24_P914479 | 5.84E-06 | 3.4 | BC002724 | SNX5 | Homo sapiens sorting nexin 5, mRNA (cDNA clone IMAGE:3629947), complete cds. [BC002724] |
| A_24_P84970 | 5.88E-06 | 3.0 | XR_016386 | KRT18P42 | PREDICTED: Homo sapiens similar to Keratin, type I cytoskeletal 18 (Cytokeratin-18) (CK-18) (Keratin-18) (K18) (LOC391819), mRNA [XR_016386] |
| A_24_P161827 | 6.30E-06 | 5.1 | XR_018749 | LOC442405 | PREDICTED: Homo sapiens similar to Keratin, type I cytoskeletal 18 (Cytokeratin-18) (CK-18) (Keratin-18) (K18) (LOC442405), mRNA [XR_018749] |
| A_23_P388812 | 6.70E-06 | 5.7 | NM_152515 | CKAP2L | Homo sapiens cytoskeleton associated protein 2-like (CKAP2L), mRNA [NM_152515] |
| A_23_P48669 | 6.84E-06 | 4.4 | NM_005192 | CDKN3 | Homo sapiens cyclin-dependent kinase inhibitor 3 (CDK2-associated dual specificity phosphatase) (CDKN3), mRNA [NM_005192] |
| A_23_P151150 | 7.46E-06 | 4.9 | NM_202002 | FOXM1 | Homo sapiens forkhead box M1 (FOXM1), transcript variant 1, mRNA [NM_202002] |

UP-regulated genes in metastatic GISts

| Probe Name | Corrected p-value | Fold Change | Genbank Accession | Gene Symbol | Description |
|---|---|---|---|---|---|
| A_23_P52278 | 7.65E-06 | 4.0 | NM_004523 | KIF11 | Homo sapiens kinesin family member 11 (KIF11), mRNA [NM_004523] |
| A_23_P34788 | 7.84E-06 | 4.7 | NM_006845 | KIF2C | Homo sapiens kinesin family member 2C (KIF2C), mRNA [NM_006845] |
| A_23_P70007 | 7.84E-06 | 5.3 | NM_012484 | HMMR | Homo sapiens hyaluronan-mediated mobility receptor (RHAMM) (HMMR), transcript variant 1, mRNA [NM_012484] |
| A_23_P161474 | 7.95E-06 | 4.5 | NM_182751 | MCM10 | Homo sapiens MCM10 minichromosome maintenance deficient 10 (S. cerevisiae) (MCM10), transcript variant 1, mRNA [NM_182751] |
| A_24_P48248 | 7.95E-06 | 3.1 | NM_024032 | C17orf53 | Homo sapiens chromosome 17 open reading frame 53 (C17orf53), mRNA [NM_024032] |
| A_23_P252292 | 8.09E-06 | 4.5 | NM_006733 | CENPI | Homo sapiens centromere protein I (CENPI), mRNA [NM_006733] |
| A_24_P14156 | 8.62E-06 | 5.3 | NM_006101 | NDC80 | Homo sapiens NDC80 homolog, kinetochore complex component (S. cerevisiae) (NDC80), mRNA [NM_006101] |
| A_23_P356684 | 8.95E-06 | 5.9 | NM_018685 | ANLN | Homo sapiens anillin, actin binding protein (ANLN), mRNA [NM_018685] |
| A_23_P57588 | 9.10E-06 | 4.7 | NM_016426 | GTSE1 | Homo sapiens G-2 and S-phase expressed 1 (GTSE1), mRNA [NM_016426] |
| A_24_P375360 | 9.10E-06 | 4.3 | XR_019146 | LOC651439 | PREDICTED: Homo sapiens similar to Keratin, type I cytoskeletal 18 (Cytokeratin-18) (CK-18) (Keratin-18) (K18) (LOC651439), mRNA [XR_019146] |
| A_24_P257099 | 9.31E-06 | 7.1 | NM_018410 | DKFZp762E1312 | Homo sapiens hypothetical protein DKFZp762E1312 (DKFZp762E1312), mRNA [NM_018410] |
| A_24_P247233 | 9.39E-06 | 4.9 | XR_018420 | KRT18P16 | PREDICTED: Homo sapiens similar to Keratin, type I cytoskeletal 18 (Cytokeratin-18) (CK-18) (Keratin-18) (K18) (LOC391827), mRNA [XR_018420] |
| A_23_P37704 | 9.39E-06 | 4.4 | NM_030928 | CDT1 | Homo sapiens chromatin licensing and DNA replication factor 1 (CDT1), mRNA [NM_030928] |
| A_23_P164814 | 9.69E-06 | 3.3 | NM_024323 | C19orf57 | Homo sapiens chromosome 19 open reading frame 57 (C19orf57), mRNA [NM_024323] |
| A_23_P88331 | 9.69E-06 | 6.2 | NM_014750 | DLG7 | Homo sapiens discs, large homolog 7 (Drosophila) (DLG7), mRNA [NM_014750] |
| A_23_P57379 | 9.69E-06 | 4.1 | NM_003504 | CDC45L | Homo sapiens CDC45 cell division cycle 45-like (S. cerevisiae) (CDC45L), mRNA [NM_003504] |
| A_23_P259586 | 9.69E-06 | 6.7 | NM_003318 | TTK | Homo sapiens TTK protein kinase (TTK), mRNA [NM_003318] |
| A_23_P210853 | 9.69E-06 | 5.6 | NM_021067 | GINS1 | Homo sapiens GINS complex subunit 1 (Psf1 homolog) (GINS1), mRNA [NM_021067] |
| A_24_P916195 | 9.69E-06 | 4.5 | NM_016426 | GTSE1 | Homo sapiens G-2 and S-phase expressed 1 (GTSE1), mRNA [NM_016426] |

UP-regulated genes in metastatic GISts

| Probe Name | Corrected p-value | Fold Change | Genbank Accession | Gene Symbol | Description |
|---|---|---|---|---|---|
| A_24_P332595 | 9.69E-06 | 4.2 | XR_018618 | KRT18P47 | PREDICTED: Homo sapiens similar to Keratin, type I cytoskeletal 18 (Cytokeratin-18) (CK-18) (Keratin-18) (K18) (LOC390634), mRNA [XR_018618] |
| A_23_P94422 | 9.90E-06 | 5.2 | NM_014791 | MELK | Homo sapiens maternal embryonic leucine zipper kinase (MELK), mRNA [NM_014791] |
| A_23_P206441 | 9.94E-06 | 4.2 | NM_000135 | FANCA | Homo sapiens Fanconi anemia, complementation group A (FANCA), transcript variant 1, mRNA [NM_000135] |
| A_32_P62997 | 1.00E-05 | 7.4 | NM_018492 | PBK | Homo sapiens PDZ binding kinase (PBK), mRNA [NM_018492] |
| A_24_P728920 | 1.05E-05 | 6.6 | BC131554 | BC 131554 | Homo sapiens cDNA clone IMAGE:40108029. [BC131554] |
| A_24_P218979 | 1.18E-05 | 3.3 | NM_031299 | CDCA3 | Homo sapiens cell division cycle associated 3 (CDCA3), mRNA [M_031299] |
| A_24_P378331 | 1.22E-05 | 3.1 | NM_144508 | CASC5 | Homo sapiens cancer susceptibility candidate 5 (CASC5), transcript variant 2, mRNA [NM_144508] |
| A_24_P96780 | 1.22E-05 | 4.6 | NM_016343 | CENPF | Homo sapiens centromere protein F, 350/400ka (mitosin) (CENPF), mRNA [NM_016343] |
| A_23_P256956 | 1.23E-05 | 6.9 | NM_005733 | KIF20A | Homo sapiens kinesin family member 20A (KIF20A), mRNA [NM_005733] |
| A_24_P195164 | 1.23E-05 | 4.3 | THC2524582 | THC2524582 | Q5U0N8_HUMAN (Q5U0N8) Keratin 18 (Cell proliferation-inducing protein 46), partial (46%) [THC2524582] |
| A_23_P65757 | 1.23E-05 | 5.1 | NM_004701 | CCNB2 | Homo sapiens cyclin B2 (CCNB2), mRNA [NM_004701] |
| A_23_P122650 | 1.23E-05 | 4.5 | XR_018843 | LOC649233 | PREDICTED: Homo sapiens similar to Keratin, type I cytoskeletal 18 (Cytokeratin-18) (CK-18) (Keratin-18) (K18) (LOC649233), mRNA [XR_018843] |
| A_24_P306896 | 1.27E-05 | 5.9 | ENST00000323198 | ENST00000323198 | similar to Ubiquitin-conjugating enzyme E2 C (Ubiquitin-protein ligase C) (Ubiquitin carrier protein C) (UbcH10) (LOC648937), mRNA [Source:RefSeq_dna;Acc:XR_018466] [ENST00000323198] |
| A_23_P35219 | 1.27E-05 | 8.7 | NM_002497 | NEK2 | Homo sapiens NIMA (never in mitosis gene a)-related kinase 2 (NEK2), mRNA [NM_002497] |
| A_32_P151544 | 1.27E-05 | 5.1 | NM_000224 | KRT18 | Homo sapiens keratin 18 (KRT18), transcript variant 1, mRNA [NM_000224] |
| A_24_P176374 | 1.30E-05 | 3.9 | NM_030928 | CDT1 | Homo sapiens chromatin licensing and DNA replication factor 1 (CDT1), mRNA [NM_030928] |
| A_24_P153003 | 1.41E-05 | 3.2 | XR_019238 | LOC652192 | PREDICTED: Homo sapiens similar to Keratin, type I cytoskeletal 18 (Cytokeratin-18) (CK-18) (Keratin-18) (K18) (LOC652192), mRNA [XR_019238] |

UP-regulated genes in metastatic GISts

| Probe Name | Corrected p-value | Fold Change | Genbank Accession | Gene Symbol | Description |
|---|---|---|---|---|---|
| A_23_P119254 | 1.44E-05 | 3.4 | NM_018154 | ASF1 B | Homo sapiens ASF1 anti-silencing function 1 homolog B (S. cerevisiae) (ASF1 B), mRNA [NM_018154] |
| A_24_P230486 | 1.44E-05 | 4.1 | A_24_P230486 | A_24_P230486 | |
| A_23_P118834 | 1.46E-05 | 5.2 | NM_001067 | TOP2A | Homo sapiens topoisomerase (DNA) II alpha 170kDa (TOP2A), mRNA [NM_001067] |
| A_23_P155815 | 1.53E-05 | 5.2 | NM_022346 | NCAPG | Homo sapiens non-SMC condensin I complex, subunit G (NCAPG), mRNA [NM_022346] |
| A_24_P911179 | 1.53E-05 | 8.0 | NM_018136 | ASPM | Homo sapiens asp (abnormal spindle) homolog, microcephaly associated (Drosophila) (ASPM), mRNA [NM_018136] |
| A_23_P160537 | 1.57E-05 | 3.8 | NM_024037 | C1orf135 | Homo sapiens chromosome 1 open reading frame 135 (C1orf135), mRNA [NM_024037] |
| A_23_P66732 | 1.58E-05 | 3.5 | NM_031965 | GSG2 | Homo sapiens germ cell associated 2 (haspin) (GSG2), mRNA [NM_031965] |
| A_24_P418687 | 1.62E-05 | 5.6 | XR_015605 | LOC731794 | PREDICTED: Homo sapiens similar to Keratin, type I cytoskeletal 18 (Cytokeratin-18) (CK-18) (Keratin-18) (K18) (LOC731794), mRNA [XR_015605] |
| A_23_P70249 | 1.68E-05 | 7.4 | NM_001790 | CDC25C | Homo sapiens cell division cycle 25 homolog C (S. pombe) (CDC25C), transcript variant 1, mRNA [NM_001790] |
| A_23_P258493 | 1.68E-05 | 3.5 | NM_005573 | LMNB1 | Homosapiens lamin B1 (LMNB1), mRNA [NM_005573] |
| A_23_P115872 | 1.69E-05 | 5.9 | NM_018131 | CEP55 | Homo sapiens centrosomal protein 55kDa (CEP55), mRNA [NM_018131] |
| A_24_P50328 | 1.72E-05 | 4.8 | A_24_P50328 | A_24_P50328 | |
| A_24_P471242 | 1.84E-05 | 4.9 | A_24_P471242 | A_24_P471242 | |
| A_24_P383660 | 1.84E-05 | 6.1 | XR_018670 | KRT18P12 | PREDICTED: Homo sapiens similar to Keratin, type I cytoskeletal 18 (Cytokeratin-18) (CK-18) (Keratin-18) (K18) (LOC643471), mRNA [XR_018670] |
| A_23_P99292 | 1.97E-05 | 3.2 | NM_006479 | RAD51AP1 | Homo sapiens RAD51 associated protein 1 (RAD51AP1), mRNA [NM_006479] |
| A_23_P25069 | 2.13E-05 | 3.7 | BC039117 | OVOS2 | Homo sapiens ovostatin 2, mRNA (cDNA clone IMAGE:4827636). [BC039117] |
| A_24_P161809 | 2.22E-05 | 5.2 | ENST00000333983 | ENST00000333983 | PREDICTED: Homo sapiens similar to Keratin, type I cytoskeletal 18 (Cytokeratin-18) (CK-18) (Keratin-18) (K18) (LOC391179), mRNA [XR_018953] |
| A_23_P259641 | 2.22E-05 | 3.1 | NM_004456 | EZH2 | Homo sapiens enhancer of zeste homolog 2 (Drosophila) (EZH2), transcript variant 1, mRNA [NM_004456] |

UP-regulated genes in metastatic GISts

| Probe Name | Corrected p-value | Fold Change | Genbank Accession | Gene Symbol | Description |
|---|---|---|---|---|---|
| A_24_P255954 | 2.23E-05 | 5.5 | XR_019330 | LOC652370 | PREDICTED: Homo sapiens similar to Keratin, type I cytoskeletal 18 (Cytokeratin-18) (CK-18) (Keratin-18) (K18) (LOC652370), mRNA [XR_019330] |
| A_23_P206059 | 2.25E-05 | 3.5 | NM_003981 | PRC1 | Homo sapiens protein regulator of cytokinesis 1 (PRC1), transcript variant 1, mRNA [NM_003981] |
| A_23_P35871 | 2.26E-05 | 5.3 | NM_024680 | E2F8 | Homo sapiens E2F transcription factor 8 (E2F8), mRNA [NM_024680] |
| A_24_P416079 | 2.33E-05 | 4.4 | NM_016359 | NUSAP1 | Homo sapiens nucleolar and spindle associated protein 1 (NUSAP1), transcript variant 1, mRNA [NM_016359] |
| A_24_P161733 | 2.43E-05 | 5.9 | A_24_P161733 | A_24_P161733 | |
| A_24_P350060 | 2.47E-05 | 5.6 | XR_016386 | KRT18P42 | PREDICTED: Homo sapiens similar to Keratin, type I cytoskeletal 18 (Cytokeratin-18) (CK-18) (Keratin-18) (K18) (LOC391819), mRNA [XR_016386] |
| A_23_P63789 | 2.52E-05 | 3.0 | NM_001005414 | ZWINT | Homo sapiens ZW10 interactor (ZWINT), transcript variant 4, mRNA [NM_001005414] |
| A_24_P412088 | 2.52E-05 | 5.0 | NM_182751 | MCM10 | Homo sapiens MCM10 minichromosome maintenance deficient 10 (S. cerevisiae) (MCM10), transcript variant 1, mRNA [NM_182751] |
| A_32_P108748 | 2.62E-05 | 3.5 | THC2534530 | THC2534530 | AF235023 chromosome condensation protein G {Homo sapiens} (exp=0; wgp=1; cg=0), partial (3%) [THC2534530] |
| A_24_P16230 | 2.66E-05 | 6.6 | XR_019037 | LOC391271 | PREDICTED: Homo sapiens hypothetical LOC391271 (LOC391271), mRNA [XR_019037] |
| A_23_P355075 | 2.80E-05 | 3.0 | AK023669 | CENPN | Homo sapiens cDNA FLJ13607 fis, clone PLACE1010624. [AK023669] |
| A_24_P25872 | 3.08E-05 | 5.4 | NM_017779 | DEPDC1 | Homo sapiens DEP domain containing 1 (DEPDC1), mRNA [NM_017779] |
| A_24_P792988 | 3.09E-05 | 6.0 | A_24_P792988 | A_24_P792988 | |
| A_24_P419132 | 3.09E-05 | 3.5 | NM_006733 | CENPI | Homo sapiens centromere protein I (CENPI), mRNA [NM_006733] |
| A_23_P254733 | 3.21E-05 | 3.3 | NM_024629 | MLF1IP | Homo sapiens MLF1 interacting protein (MLF1IP), mRNA [NM_024629] |
| A_24_P281374 | 321E-05 | 5.2 | XR_018462 | KRT18P45 | PREDICTED: Homo sapiens similar to Keratin, type I cytoskeletal 18 (Cytokeratin-18) (CK-18) (Keratin-18) (K18) (LOC391803), mRNA [XR_018462] |
| A_23_P134910 | 3.27E-05 | 3.9 | NM_003878 | GGH | Homo sapiens gamma-glutamyl hydrolase (conjugase, folylpolygammaglutamyl hydrolase) (GGH), mRNA [NM_003878] |
| A_23_P148475 | 3.48E-05 | 4.5 | NM_012310 | KIF4A | Homo sapiens kinesin family member 4A (KIF4A), mRNA [M_012310] |
| A_24_P358406 | 3.54E-05 | 6.2 | A_24_P358406 | A_24_P358406 | |

EP 2 630 259 B1

UP-regulated genes in metastatic GISts

| Probe Name | Corrected p-value | Fold Change | Genbank Accession | Gene Symbol | Description |
|---|---|---|---|---|---|
| A_24_P169843 | 3.63E-05 | 4.8 | XR_019568 | KRT18P28 | PREDICTED: Homo sapiens similar to Keratin, type I cytoskeletal 18 (Cytokeratin-18) (CK-18) (Keratin-18) (K18) (LOC343326), mRNA [XR_019568] |
| A_23_P150667 | 3.63E-05 | 4.4 | NM_031217 | KIF18A | Homo sapiens kinesin family member 18A (KIF18A), mRNA [NM_031217] |
| A_24_P780319 | 3.69E-05 | 4.1 | A_24_P780319 | A_24_P780319 | |
| A_23_P116123 | 3.69E-05 | 3.5 | NM_001274 | CHEK1 | Homo sapiens CHK1 checkpoint homolog (S. pombe) (CHEK1), mRNA [NM_001274] |
| A_24_P584463 | 3.72E-05 | 5.4 | XR_018311 | LOC139060 | PREDICTED: Homo sapiens similar to Keratin, type I cytoskeletal 18 (Cytokeratin-18) (CK-18) (Keratin-18) (K18) (LOC139060), mRNA [XR_018311] |
| A_23_P323751 | 3.80E-05 | 6.8 | NM 030919 | FAM83D | Homo sapiens family with sequence similarity 83, member D (FAM83D), mRNA [NM_030919] |
| A_24_P186746 | 3.92E-05 | 6.6 | XR_019198 | KRT18P34 | PREDICTED: Homo sapiens similar to Keratin, type I cytoskeletal 18 (Cytokeratin-18) (CK-18) (Keratin-18) (K18) (LOC391589), mRNA [XR_019198] |
| A_24_P84711 | 4.01E-05 | 4.8 | A_24_P84711 | A_24_P84711 | |
| A_24_P230466 | 4.03E-05 | 6.2 | XR_018953 | KRT18P32 | PREDICTED: Homo sapiens similar to Keratin, type I cytoskeletal 18 (Cytokeratin-18) (CK-18) (Keratin-18) (K18) (LOC391179), mRNA [XR_018953] |
| A_32_P9924 | 4.09E-05 | 3.5 | THC2525505 | THC2525505 | |
| A_23_P149668 | 4.16E-05 | 5.8 | NM_014875 | KIF14 | Homo sapiens kinesin family member 14 (KIF14), mRNA [NM_014875] |
| A_23_P25150 | 4.39E-05 | 21.2 | NM_006897 | HOXC9 | Homo sapiens homeobox C9 (HOXC9), mRNA [NM_006897] |
| A_23_P58321 | 4.53E-05 | 3.4 | NM_001237 | CCNA2 | Homo sapiens cyclin A2 (CCNA2), mRNA [NM_001237] |
| A_23_P99320 | 4.57E-05 | 6.6 | NM_000224 | KRT18 | Homo sapiens keratin 18 (KRT18), transcript variant 1, mRNA [NM_000224] |
| A_23_P373708 | 4.60E-05 | 5.8 | NM_173624 | FLJ40504 | Homo sapiens hypothetical protein FLJ40504 (FLJ40504), mRNA [NM_173624] |
| A_24_P358131 | 4.85E-05 | 6.0 | XR_019148 | LOC651696 | PREDICTED: Homo sapiens similar to Keratin, type I cytoskeletal 18 (Cytokeratin-18) (CK-18) (Keratin-18) (K18) (LOC651696), mRNA [XR_019148] |
| A_24_P256063 | 5.02E-05 | 6.7 | XR_019231 | LOC442249 | PREDICTED: Homo sapiens hypothetical LOC442249 (LOC442249), mRNA [XR_019231] |

UP-regulated genes in metastatic GISts

| Probe Name | Corrected p-value | Fold Change | Genbank Accession | Gene Symbol | Description |
|---|---|---|---|---|---|
| A_24_P24645 | 5.30E-05 | 5.7 | XR_018938 | KRT18P21 | PREDICTED: Homo sapiens similar to Keratin, type I cytoskeletal 18 (Cytokeratin-18) (CK-18) (Keratin-18) (K18) (LOC132391), mRNA [XR_018938] |
| A_32_P154726 | 5.37E-05 | 6.8 | THC2603239 | THC2603239 | Q9NJB6_TRYBR (Q9NJB6) Fibrillarin, partial (10%) [THC2603239] |
| A_23_P216517 | 5.67E-05 | 4.0 | NM_032818 | C9orf100 | Homo sapiens chromosome 9 open reading frame 100 (C9orf100), mRNA [NM_032818] |
| A_24_P281443 | 5.82E-05 | 7.2 | XR_018559 | LOC649375 | PREDICTED: Homo sapiens similar to Keratin, type I cytoskeletal 18 (Cytokeratin-18) (CK-18) (Keratin-18) (K18) (LOC649375), mRNA [XR_018559] |
| A_23_P134584 | 5.83E-05 | 3.0 | NM_005431 | XRCC2 | Homo sapiens X-ray repair complementing defective repair in Chinese hamster cells 2 (XRCC2), mRNA [NM_005431] |
| A_24_P6850 | 5.92E-05 | 4.7 | A_24_P6850 | A_24 P6850 | |
| A_24_P264644 | 5.92E-05 | 5.6 | XR_016695 | KRT18P41 | PREDICTED: Homo sapiens similar to Keratin, type I cytoskeletal 18 (Cytokeratin-18) (CK-18) (Keratin-18) (K18) (LOC345430), mRNA [XR_016695] |
| A_23_P368909 | 6.02E-05 | 4.4 | ENST00000328711 | ENST00000328711 | Uncharacterized protein C13orf29. [Source:Uniprot/SWISSPROT;Acc:Q8IVM7] [ENST00000328711] |
| A_24_P42136 | 6.22E-05 | 7.7 | NM_000224 | KRT18 | Homo sapiens keratin 18 (KRT18), transcript variant 1, mRNA [NM_000224] |
| A_24_P230057 | 6.74E-05 | 7.2 | XR_018216 | LOC647913 | PREDICTED: Homo sapiens similar to Keratin, type I cytoskeletal 18 (Cytokeratin-18) (CK-18) (Keratin-18) (K18) (LOC647913), mRNA [XR_018216] |
| A_24_P314571 | 7.33E-05 | 3.5 | NM_182513 | SPC24 | Homo sapiens SPC24, NDC80 kinetochore complex component, homolog (S. cerevisiae) (SPC24), mRNA [NM_182513] |
| A_23_P29723 | 7.60E-05 | 4.4 | NM_001012410 | SGOL1 | Homo sapiens shugoshin-like 1 (S. pombe) (SGOL1), transcript variant A2, mRNA [M_001012410] |
| A_23_P120863 | 8.27E-05 | 5.8 | NM_004861 | GAL3ST1 | Homo sapiens galactose-3-O-sulfotransferase 1 (GAL3ST1), mRNA [NM_004861] |
| A_24_P225970 | 8.78E-05 | 5.9 | NM_001012409 | SGOL1 | Homo sapiens shugoshin-like 1 (S. pombe) (SGOL1), transcript variant A1, mRNA [NM_001012409] |
| A_23_P130182 | 1.01E-04 | 5.2 | NM_004217 | AURKB | Homo sapiens aurora kinase B (AURKB), mRNA [NM_004217] |
| A_23_P10385 | 1.05E-04 | 4.1 | NM_016448 | DTL | Homo sapiens denticleless homolog (Drosophila) (DTL), mRNA [NM_016448] |
| A_23_P92093 | 1.16E-04 | 3.5 | NM_001407 | CELSR3 | Homo sapiens cadherin, EGF LAG seven-pass G-type receptor 3 (flamingo homolog, Drosophila) (CELSR3), mRNA [NM_001407] |

UP-regulated genes in metastatic GISts

| Probe Name | Corrected p-value | Fold Change | Genbank Accession | Gene Symbol | Description |
|---|---|---|---|---|---|
| A_24_P940678 | 1.22E-04 | 4.4 | NM_170589 | CASC5 | Homo sapiens cancer susceptibility candidate 5 (CASC5), transcript variant 1, mRNA [NM_170589] |
| A_24_P401601 | 1.27E-04 | 5.9 | XR_017288 | KRT18P40 | PREDICTED: Homo sapiens similar to Keratin, type I cytoskeletal 18 (Cytokeratin-18) (CK-18) (Keratin-18) (K18) (LOC390904), mRNA [XR_017288] |
| A_23_P500464 | 1.31E-04 | 7.8 | NM_001844 | COL2A1 | Homo sapiens collagen, type II, alpha 1 (primary osteoarthritis, spondyloepiphyseal dysplasia, congenital) (COL2A1), transcript variant 1, mRNA [NM_001844] |
| A_23_P373119 | 1.35E-04 | 3.2 | NR_002165 | HMG4L | Homo sapiens high-mobility group (nonhistone chromosomal) protein 4-like (HMG4L) on chromosome 20 [NR_002165] |
| A_24_P384369 | 1.36E-04 | 3.7 | XR_018339 | LOC648448 | PREDICTED: Homo sapiens similar to Keratin, type I cytoskeletal 18 (Cytokeratin-18) (CK-18) (Keratin-18) (K18) (LOC648448), mRNA [XR_018339] |
| A_23_P200310 | 1.46E-04 | 3.1 | NM_017779 | DEPDC1 | Homo sapiens DEP domain containing 1 (DEPDC1), mRNA [NM_017779] |
| A_24_P247454 | 1.46E-04 | 7.6 | XR_019026 | KRT18P19 | PREDICTED: Homo sapiens similar to Keratin, type I cytoskeletal 18 (Cytokeratin-18) (CK-18) (Keratin-18) (K18) (LOC339781), mRNA [XR_019026] |
| A_23_P85441 | 1.51E-04 | 3.3 | NM_020789 | IGSF9 | Homo sapiens immunoglobulin superfamily, member 9 (IGSF9), mRNA [NM_020789] |
| A_24_P144625 | 1.59E-04 | 7.3 | A_24_P144625 | A_24_P144625 | |
| A_23_P431776 | 1.60E-04 | 8.6 | NM_001986 | ETV4 | Homo sapiens ets variant gene 4 (E1A enhancer binding protein, E1AF) (ETV4), transcript variant 1, mRNA [NM_001986] |
| A_24_P416346 | 1.61E-04 | 8.1 | NM_001986 | ETV4 | Homo sapiens ets variant gene 4 (E1A enhancer binding protein, E1AF) (ETV4), transcript variant 1, mRNA [NM_001986] |
| A_23_P408955 | 1.68E-04 | 4.2 | NM_004091 | E2F2 | Homo sapiens E2F transcription factor 2 (E2F2), mRNA [NM_004091] |
| A_23_P48835 | 1.71E-04 | 3.7 | NM_138555 | KIF23 | Homo sapiens kinesin family member 23 (KIF23), transcript variant 1, mRNA [NM_138555] |
| A_23_P379614 | 1.73E-04 | 3.0 | NM_007280 | OIP5 | Homo sapiens Opa interacting protein 5 (OIP5), mRNA [NM_007280] |
| A_32_P119154 | 1.78E-04 | 4.5 | BE138567 | BE138567 | xr77d10.x2 NCI_CGAP_Ov26 Homo sapiens cDNA clone IMAGE:2766163 3', mRNA sequence [BE138567] |
| A_23_P310 | 1.89E-04 | 4.0 | NM_023009 | MARCKSL1 | Homo sapiens MARCKS-like 1 (MARCKSL1), mRNA [NM_023009] |
| A_32_P76720 | 1.89E-04 | 3.6 | NM_016575 | NT5DC3 | Homo sapiens 5'-nucleotidase domain containing 3 (NT5DC3), transcript variant 2, mRNA [NM_016575] |

UP-regulated genes in metastatic GISts

| Probe Name | Corrected p-value | Fold Change | Genbank Accession | Gene Symbol | Description |
|---|---|---|---|---|---|
| A_23_P50250 | 1.90E-04 | 3.0 | NM_001824 | CKM | Homo sapiens creatine kinase, muscle (CKM), mRNA [NM_001824] |
| A_23_P217236 | 1.99E-04 | 3.1 | NM_005342 | HMGB3 | Homo sapiens high-mobility group box 3 (HMGB3), mRNA [NM_005342] |
| A_24_P346855 | 2.00E-04 | 4.9 | NM_002417 | MKI67 | Homo sapiens antigen identified by monoclonal antibody Ki-67 (MKI67), mRNA [NM_002417] |
| A_24_P68088 | 2.04E-04 | 10.8 | NR_002947 | TCAM1 | Homo sapiens testicular cell adhesion molecule 1 homolog (mouse) (TCAM1) on chromosome 17 [NR_002947] |
| A_24_P399888 | 2.10E-04 | 4.2 | NM_001002876 | CENPM | Homo sapiens centromere protein M (CENPM), transcript variant 2, mRNA [NM_001002876] |
| A_23_P88731 | 2.13E-04 | 3.4 | NM_002875 | RAD51 | Homo sapiens RAD51 homolog (RecA homolog, E. coli) (S. cerevisiae) (RAD51), transcript variant 1, mRNA [NM_002875] |
| A_23_P350754 | 2.14E-04 | 3.5 | AF238487 | OR7E13P | Homo sapiens olfactory-like receptor PJCG2 (PJCG2) mRNA, partial cds. [AF238487] |
| A_23_P117852 | 2.21E-04 | 3.9 | NM_014736 | KIAA0101 | Homo sapiens KIAA0101 (KIAA0101), transcript variant 1, mRNA [NM_014736] |
| A_23_P100127 | 2.25E-04 | 4.8 | NM_170589 | CASC5 | Homo sapiens cancer susceptibility candidate 5 (CASC5), transcript variant 1, mRNA [NM_170589] |
| A_23_P155989 | 2.30E-04 | 3.0 | NM_022145 | CENPK | Homo sapiens centromere protein K (CENPK), mRNA [NM_022145] |
| A_24_P109661 | 2.33E-04 | 7.5 | XR_019191 | KRT18P20 | PREDICTED: Homo sapiens similar to Keratin, type I cytoskeletal 18 (Cytokeratin-18) (CK-18) (Keratin-18) (K18) (LOC121054), mRNA [XR_019191] |
| A_24_P686014 | 2.48E-04 | 6.3 | XR_019186 | LOC651929 | PREDICTED: Homo sapiens similar to Keratin, type I cytoskeletal 18 (Cytokeratin-18) (CK-18) (Keratin-18) (K18) (LOC651929), mRNA [XR_019186] |
| A_23_P50108 | 2.48E-04 | 3.7 | NM_006101 | NDC80 | Homo sapiens NDC80 homolog, kinetochore complex component (S. cerevisiae) (NDC80), mRNA [NM_006101] |
| A_23_P108294 | 2.53E-04 | 3.6 | NM_177543 | PPAP2C | Homo sapiens phosphatidic acid phosphatase type 2C (PPAP2C), transcript variant 3, mRNA [NM_177543] |
| A_23_P110851 | 2.95E-04 | 6.4 | NM_198253 | TERT | Homo sapiens telomerase reverse transcriptase (TERT), transcript variant 1, mRNA [NM_198253] |
| A_24_P264293 | 3.03E-04 | 9.4 | XR_019060 | LOC644030 | PREDICTED: Homo sapiens similar to Keratin, type I cytoskeletal 18 (Cytokeratin-18) (CK-18) (Keratin-18) (K18) (LOC644030), mRNA [XR_019060] |
| A_24_P247303 | 3.33E-04 | 8.7 | A_24_P247303 | A_24_P247303 | |

UP-regulated genes in metastatic GISts

| Probe Name | Corrected p-value | Fold Change | Genbank Accession | Gene Symbol | Description |
|---|---|---|---|---|---|
| A_32_P311737 | 3.42E-04 | 3.2 | AB011171 | PLEKHG3 | Homo sapiens mRNA for KIAA0599 protein, partial cds. [AB011171] |
| A_23_P166306 | 3.44E-04 | 6.9 | NM_000071 | CBS | Homo sapiens cystathionine-beta-synthase (CBS), mRNA [NM_000071] |
| A_23_P23303 | 3.48E-04 | 3.3 | NM_003686 | EXO1 | Homo sapiens exonuclease 1 (EXO1), transcript variant 3, mRNA [NM_003686] |
| A_24_P255836 | 3.52E-04 | 3.5 | A_24_P255836 | A_24_P255836 | |
| A_32_P168561 | 3.61E-04 | 3.1 | THC2634862 | THC2634862 | NM_063888 TAF (TBP-associated transcription factor) family member (taf-13) {Caenorhabditis elegans} (exp=-1; wgp=0; cg=0), partial (15%) [THC2634862] |
| A_24_P254705 | 3.62E-04 | 3.7 | NM_020394 | ZNF695 | Homo sapiens zinc finger protein 695 (ZNF695), mRNA [NM_020394] |
| A_24_P234196 | 3.63E-04 | 3.8 | NM_001034 | RRM2 | Homo sapiens ribonucleotide reductase M2 polypeptide (RRM2), mRNA [NM_001034] |
| A_32_P188127 | 3.68E-04 | 6.8 | A_32_P188127 | A_32_P188127 | |
| A_23_P155711 | 3.77E-04 | 5.3 | NM_018248 | NEIL3 | Homo sapiens nei endonuclease VIII-like 3 (E. coli) (NEIL3), mRNA [NM_018248] |
| A_24_P85498 | 3.84E-04 | 11.6 | AL117481 | DKFZP434B061 | Homo sapiens mRNA; cDNA DKFZp434B061 (from clone DKFZp434B061); partial cds. [AL117481] |
| A_23_P115444 | 3.97E-04 | 4.2 | NM_005092 | TNFSF18 | Homo sapiens tumor necrosis factor (ligand) superfamily, member 18 (TNFSF18), mRNA [NM_005092] |
| A_32_P108938 | 4.05E-04 | 5.7 | THC2536711 | THC2536711 | |
| A_23_P143512 | 4.08E-04 | 3.1 | NM_007031 | HSF2BP | Homo sapiens heat shock transcription factor 2 binding protein (HSF2BP), mRNA [NM_007031] |
| A_23_P252928 | 4.12E-04 | 11.3 | NM_005367 | MAGEA12 | Homo sapiens melanoma antigen family A, 12 (MAGEA12), mRNA [NM_005367] |
| A_32_P147090 | 4.70E-04 | 3.3 | NM_199357 | ARHGAP11A | Homo sapiens Rho GTPase activating protein 11A (ARHGAP11A), transcript variant 2, mRNA [NM_199357] |
| A_23_P102058 | 4.89E-04 | 3.1 | NM_002381 | MATN3 | Homo sapiens matrilin 3 (MATN3), mRNA [NM_002381] |
| A_32_P169500 | 4.92E-04 | 3.2 | THC2537856 | THC2537856 | ALU1_HUMAN (P39188) Alu subfamily J sequence contamination warning entry, partial (14%) [THC2537856] |
| A_23_P163099 | 5.02E-04 | 3.2 | NM_002692 | POLE2 | Homo sapiens polymerase (DNA directed), epsilon 2 (p59 subunit) (POLE2), mRNA [NM_002692] |
| A_23_P405267 | 5.04E-04 | 3.2 | AK057922 | CDH24 | Homo sapiens cDNA FLJ25193 fis, clone JTH00761. [AK057922] |
| A_23_P74115 | 5.23E-04 | 3.1 | NM_003579 | RAD54L | Homo sapiens RAD54-like (S. cerevisiae) (RAD54L), mRNA [NM_003579] |

UP-regulated genes in metastatic GISts

| Probe Name | Corrected p-value | Fold Change | Genbank Accession | Gene Symbol | Description |
|---|---|---|---|---|---|
| A_24_P409420 | 5.78E-04 | 5.6 | A_24_P409420 | A_24_P409420 | |
| A_24_P384018 | 5.78E-04 | 3.7 | NR_002171 | OR7E156P | Homo sapiens olfactory receptor, family 7, subfamily E, member 156 pseudogene (OR7E156P) on chromosome 13 [NR_002171] |
| A_24_P192727 | 5.85E-04 | 6.5 | ENST00000224809 | KAZALD1 | Kazal-type serine protease inhibitor domain-containing protein 1 precursor. [Source: Uniprot/SWISSPROT;Acc:Q96I82] [ENST00000224809] |
| A_32_P210202 | 5.99E-04 | 3.2 | NM_203394 | E2F7 | Homo sapiens E2F transcription factor 7 (E2F7), mRNA [NM_203394] |
| A_23_P58557 | 6.09E-04 | 3:4 | NM_173800 | FLJ90650 | Homo sapiens laeverin (FLJ90650), mRNA [NM_173800] |
| A_32_P43084 | 6.45E-04 | 3.3 | BM980974 | BM980974 | BM980974 UI-CF-EN1-ade-p-19-0-UI.s1 UI-CF-EN1 Homo sapiens cDNA clone UI-CF-EN1-ade-p-19-0-UI 3', mRNA sequence [BM980974] |
| A_23_P135061 | 6.68E-04 | 3.0 | NM_003389 | CORO2A | Homo sapiens coronin, actin binding protein, 2A (CORO2A), transcript variant 1, mRNA [NM_003389] |
| A_32_P32391 | 6.76E-04 | 3.7 | NR_002171 | OR7E156P | Homo sapiens olfactory receptor, family 7, subfamily E, member 156 pseudogene (OR7E156P) on chromosome 13 [NR_002171] |
| A_23_P7412 | 7.04E-04 | 4.2 | NM_024850 | BTNL8 | Homo sapiens butyrophilin-like 8 (BTNL8), transcript variant 1, mRNA [NM_024850] |
| A_32_P46544 | 7.04E-04 | 3.0 | A_32_P46544 | A_32_P46544 | |
| A_23_P113034 | 7.23E-04 | 3.5 | NM_032024 | C10orf11 | Homo sapiens chromosome 10 open reading frame 11 (C10orf11), mRNA [NM_032024] |
| A_23_P50517 | 7.29E-04 | 3.9 | ENST00000314121 | ENST00000314121 | Zinc finger protein 541. [Source:Uniprot/SWISSPROT;Acc:Q9H0D2] [ENST00000314121] |
| A_23_P96291 | 7.47E-04 | 5.3 | NM_004988 | MAGEA1 | Homo sapiens melanoma antigen family A, 1 (directs expression of antigen MZ2-E) (MAGEA1), mRNA [NM_004988] |
| A_23_P16110 | 7.63E-04 | 3.5 | NM_001079935 | OR7E24 | Homo sapiens olfactory receptor, family 7, subfamily E, member 24 (OR7E24), mRNA [NM_001079935] |
| A_32_P150891 | 8.60E-04 | 4.4 | NM_001042517 | DIAPH3 | Homo sapiens diaphanous homolog 3 (Drosophila) (DIAPH3), transcript variant 1, mRNA [NM_001042517] |
| A_23_P207154 | 8.75E-04 | 5.2 | NM_022644 | CSH2 | Homo sapiens chorionic somatomammotropin hormone 2 (CSH2), transcript variant 2, mRNA [NM_022644] |
| A_23_P250164 | 9.04E-04 | 4.3 | NM_000187 | HGD | Homo sapiens homogentisate 1,2-dioxygenase (homogentisate oxidase) (HGD), mRNA [NM_000187] |
| | | | | | Homo sapiens cDNA clone |

UP-regulated genes in metastatic GISts

| Probe Name | Corrected p-value | Fold Change | Genbank Accession | Gene Symbol | Description |
|---|---|---|---|---|---|
| A_24_P820087 | 9.47E-04 | 3.3 | BC053669 | BC053669 | IMAGE:6146402, partial cds. [BC053669] |

Down-regulated genes in metastatic GISTs

| Probe Name | Corrected p-value | Fold Change | Genbank Acession | Gene Symbol | Description |
|---|---|---|---|---|---|
| A_23_P167159 | 1.32E-06 | 28.6 | NM_007281 | SCRG1 | Homo sapiens scrapie responsive protein 1 (SCRG1), mRNA [NM_007281] |
| A_24_P198044 | 5.02E-06 | 3.5 | NM_133464 | ZNF483 | Homo sapiens zinc finger protein 483 (ZNF483), transcript variant 1, mRNA [NM_133464] |
| A_23_P45536 | 6.65E-06 | 13.6 | NM_005369 | MCF2 | Homo sapiens MCF.2 cell line derived transforming sequence (MCF2), mRNA [NM_005369] |
| A_23_P79978 | 7.08E-06 | 12.7 | NM_020689 | SLC24A3 | Homo sapiens solute carrier family 24 (sodium/potassium/calcium exchanger), member 3 (SLC24A3), mRNA [NM_020689] |
| A_23_P62881 | 7.46E-06 | 7.3 | NM_032291 | SGIP1 | Homo sapiens SH3-domain GRB2-like (endophilin) interacting protein 1 (SGIP1), mRNA [NM_032291] |
| A_23_P73117 | 1.07E-05 | 8.9 | NM_013266 | CTNNA3 | Homo sapiens catenin (cadherin-associated protein), alpha 3 (CTNNA3), mRNA [NM_013266] |
| A_32_P65700 | 1.14E-05 | 6.0 | BX106262 | BX106262 | BX106262 Soares_multiple_sclerosis_2NbHMSP Homo sapiens cDNA clone IMAGp998O20625, mRNA sequence [BX106262] |
| A_23_P394567 | 1.41E-05 | 3.1 | NM_020853 | KIAA1467 | Homo sapiens KIAA1467 (KIAA1467), mRNA [NM_020853] |
| A_23_P259442 | 1.53E-05 | 4.4 | NM_001873 | CPE | Homo sapiens carboxypeptidase E (CPE), mRNA[NM_001873] |
| A_24P56363 | 1.58E-05 | 3.6 | NM_030925 | CAB39L | Homo sapiens calcium binding protein 39-like (CAB39L), transcript-variant 1, mRNA [NM_030925] |
| A_24_P333857 | 2.15E-05 | 7.1 | NM_032291 | SGIP1 | sapiens SH3-domain GRB2-like (endophilin) interacting protein 1 (SGIP1), mRNA_[NM_032291] |
| A_24_P153831 | 2.22E-05 | 5.6 | BC022004 | CTNNA3 | Homo sapiens catenin (cadherin-associated protein), alpha 3, mRNA (cDNA clone IMAGE:4823848), complete-cds. [BC022004] |
| A_23_P344194 | 2.40E-05 | 5.2 | NM_001013635 | LOC387856 | Homo sapiens similar to expressed sequence AI836003 (LOC387856), mRNA [NM_001013635] |
| A_23_P92903 | 2.45E-05 | 7.4 | NM_031908 | C1QTNF2 | Homo sapiens C1q and tumor necrosis factor related protein 2 (C1QTNF2), mRNA [NM_031908] |
| A_32_P213861 | 2.52E-05 | 3.3 | AK124663 | C4orf12 | Homo sapiens cDNA FLJ42672 fis, clone BRAMY2026533. [AK124663] |

(continued)

Down-regulated genes in metastatic GISTs

| Probe Name | Corrected p-value | Fold Change | Genbank Acession | Gene Symbol | Description |
|---|---|---|---|---|---|
| A_23_P213810 | 2.66E-05 | 3.2 | NM_015621 | CCDC69 | Homo sapiens coiled-coil domain containing 69 (CCDC69), mRNA [NM_015621] |
| A_23_P92899 | 2.70E-05 | 7.0 | NM_031908 | C1QTNF2 | Homo sapiens C1q and tumor necrosis factor related protein 2 (C1 QTNF2), mRNA [NM_031908] |
| A_23_P95634 | 2.73E-05 | 7.7 | NM_016599 | MYOZ2 | Homo sapiens myozenin 2 (MYOZ2), mRNA [NM_016599] |
| A_24_P45481 | 2.76E-05 | 5.2 | NM_005465 | AKT3 | Homo sapiens v-akt murine thymoma viral oncogene homolog 3 (protein kinase B, gamma) (AKT3), transcript variant 1, mRNA [NM_005465] |
| A_23_P139891 | 3.33E-05 | 4.8 | NM_012306 | FAIM2 | Homo sapiens Fas apoptotic inhibitory molecule 2 (FAIM2), mRNA [NM_012306] |
| A_23_P325690 | 3.73E-05 | 7.3 | NM_144698 | ANKRD35 | Homo sapiens ankyrin repeat domain 35 (ANKRD35), mRNA [NM_144698] |
| A_23_P43810 | 4.79E-05 | 8.1 | NM_206943 | LTBP1 | Homo sapiens latent transforming growth factor beta binding protein 1 (LTBP1), transcript variant 1, mRNA [NM_206943] |
| A_24_P37300 | 5.14E-05 | 9.3 | AF052115 | AF052115 | Homo sapiens clone 23688 mRNA sequence. [AF052115] |
| A_24_P381499 | 7.11E-05 | 4.3 | NM_152436 | GLIPR1L2 | Homo sapiens GLI pathogenesis-related 1 like 2 (GLIPR1L2), mRNA [NM_1152436] |
| A_23_P96285 | 7.67E-05 | 7.0 | NM_022912 | REEP1 | Homo sapiens receptor accessory protein 1 (REEP1), mRNA [NM_022912] |
| A_23_P64510 | 8.17E-05 | 3.8 | NM_024557 | RIC3 | Homo sapiens resistance to inhibitors of cholinesterase 3 homolog (C. elegans) (RIC3), mRNA [NM_024557] |
| A_23_P364024 | 8.78E-05 | 3.2 | NM_006851 | GLIPR1 | Homo sapiens GLI pathogenesis-related 1 (glioma) (GLIPR1), mRNA [NM_006851] |
| A_32_P73991 | 9.58E-05 | 7.1 | THC2667995 | THC2667995 | |
| A_24_P390096 | 9.75E-05 | 3.5 | NM_006851 | GLIPR1 | Homo sapiens GLI pathogenesis-related 1 (glioma) (GLIPR1), mRNA [NM_006851] |
| A_32_P440667 | 1.00E-04 | 5.6 | AK000774 | AK000774 | Homo sapiens cDNA FLJ20767 fis, clone COL06986. [AK000774] |
| A_24_P278747 | 1.05E-04 | 7.8 | NM_001759 | CCND2 | Homo sapiens cyclin D2 (CCND2), mRNA [NM_001759] |
| A_23_P213288 | 1.11E-04 | 3.1 | NM_001037582 | SCD5 | Homo sapiens stearoyl-CoA desaturase 5 (SCD5), transcript variant 1, mRNA [NM_001037582] |
| A_23_P150394 | 1.21E-04 | 3.5 | NM_022003 | FXYD6 | Homo sapiens FXYD domain containing ion transport regulator 6 (FXYD6), mRNA |
| A_23_P47728 | 1.22E-04 | 5.2 | NM_033063 | MAP6 | [NM_022003] Homo sapiens microtubule-associated protein 6 (MAP6), transcript variant 1, mRNA [NM_033063] |

Down-regulated genes in metastatic GISTs

| Probe Name | Corrected p-value | Fold Change | Genbank Acession | Gene Symbol | Description |
|---|---|---|---|---|---|
| A_23_P254165 | 1.28E-04 | 9.3 | NM_021785 | RAI2 | Homo sapiens retinoic acid induced 2 (RAI2), mRNA [NM_021785] |
| A_24_P67350 | 1.30E-04 | 7.0 | NM_020689 | SLC24A3 | Homo sapiens solute carrier family 24 (sodium/potassium/calcium exchanger), member 3 (SLC24A3), mRNA [NM_020689] |
| A_24_P943781 | 1.30E-04 | 3.9 | NM_024913 | FLJ21986 | Homo sapiens hypothetical protein FLJ21986 (FLJ21986), mRNA [NM_024913] |
| A_24_P381505 | 1.31E-04 | 3.5 | NM_152436 | GLIPR1L2 | Homo sapiens GLI pathogenesis-related 1 like 2 (GLIPR1L2), mRNA [NM_152436] |
| A_32_P795513 | 1.86E-04 | 11.5 | NM_198271 | LMOD3 | Homo sapiens leiomodin 3 (fetal) (LMOD3), mRNA [NM_198271] |
| A_24_P76821 | 1.93E-04 | 8.4 | NM_198271 | LMOD3 | Homo sapiens leiomodin 3 (fetal) (LMOD3), mRNA [NM_198271] |
| A_23_P75915 | 1.99E-04 | 3.9 | AY326436 | RIC3 | Homo sapiens RIC3 isoform d (RIC3) mRNA, complete cds. [AY326436] |
| A_32_P91005 | 2.13E-04 | 4.4 | BM697215 | BM697215 | UI-E-DX0-ago-c-07-0-UI.r1 UI-E-DX0 Homo sapiens cDNA clone UI-E-DX0-ago-c-07-0-UI 5', mRNA sequence [BM697215] |
| A_32_P222695 | 2.13E-04 | 3.2 | NM_001001669 | FLJ41603 | Homo sapiens FLJ41603 protein (FLJ41603), mRNA [NM_001001669] |
| A_24_P35537 | 2.13E-04 | 4.7 | NM_024557 | RIC3 | Homo sapiens resistance to inhibitors of cholinesterase 3 homolog (C. elegans) (RIC3), mRNA [NM_024557] |
| A_24_P141520 | 2.18E-04 | 3.4 | AK022297 | AK022297 | Homo sapiens cDNA FLJ12235 fis, clone MAMMA 1001243. [AK022297] |
| A_32_P174040 | 2.20E-04 | 14.5 | THC2675966 | THC2675966 | Q9F8M7_CARHY (Q9F8M7) DTDP-glucose 4,6-dehydratase (Fragment), partial (11 %) [THC2697639] |
| A_23_P5342 | 2.23E-04 | 16.2 | NM_018557 | LRP1B | Homo sapiens low density lipoprotein-related protein 1 B (deleted in tumors) (LRP1 B), mRNA [NM_018557] |
| A_32_P172803 | 2.28E-04 | 3.0 | NM_001039580 | MAP9 | Homo sapiens microtubule-associated protein 9 (MAP9), mRNA [NM_001039580] |
| A_23_P94840 | 2.41E-04 | 5.2 | NM_130897 | DYNLRB2 | Homo sapiens dynein, light chain, roadblock-type 2 (DYNLRB2), mRNA [NM_130897] |
| A_23_P19182 | 2.61E-04 | 4.4 | NM_016606 | REEP2 | Homo sapiens receptor accessory protein 2 (REEP2), mRNA [NM_016606] |
| A_23_P77304 | 2.77E-04 | 4.2 | NM_004644 | AP3B2 | Homo sapiens adaptor-related protein complex 3, beta 2 subunit (AP3B2), mRNA [NM_004644] |
| A_32_P179998 | 2.80E-04 | 9.5 | NM_033053 | DMRTC1 | Homo sapiens DMRT-like family C1 (DMRTC1), mRNA [NM_033053] |
| A_24_P32085 | 2.82E-04 | 3.4 | NM_024761 | MOBKL2B | Homo sapiens MOB1, Mps One Binder kinase activator-like 2B (yeast) (MOBKL2B), mRNA [NM_024761] |

Down-regulated genes in metastatic GISTs

| Probe Name | Corrected p-value | Fold Change | Genbank Acession | Gene Symbol | Description |
|---|---|---|---|---|---|
| A_24_P110983 | 2.95E-04 | 4.8 | ENST00000366539 | AKT3 | RAC-gamma serine/threonine-protein kinase (EC 2.7.11.1) (RAC-PK-gamma) (Protein kinase Akt-3) (Protein kinase B, gamma) (PKB gamma) (STK-2). [Source:Uniprot/SWISSPROT;Acc:Q9Y243] [ENST00000366539] |
| A_23_P500892 | 3.06E-04 | 3.7 | NM_003320 | TUB | Homo sapiens tubby homolog (mouse) (TUB), transcript variant 1, mRNA [NM_003320] |
| A_24_P191781 | 3.10E-04 | 4.5 | NM_015393 | DKFZP564O0823 | Homo sapiens DKFZP564O0823 protein (DKFZP564O0823), mRNA [M_015393] |
| A_24_P97825 | 3.61E-04 | 3.0 | NM_015621 | CCDC69 | Homo sapiens coiled-coil domain containing 69 (CCDC69), mRNA [NM_015621] |
| A_32_P50943 | 3.76E-04 | 5.3 | THC2734830 | THC2734830 | |
| A_24_P769588 | 3.76E-04 | 4.2 | BQ428696 | BQ428696 | AGENCOURT_7904751 NIH_MGC_82 Homo sapiens cDNA clone IMAGE:6105895 5', mRNA sequence [BQ428696] |
| A_24_P810104 | 3.79E-04 | 3.0 | AF052141 | AF052141 | Homo sapiens clone 24626 mRNA sequence. [AF052141] |
| A_24_P942385 | 4.28E-04 | 5.1 | AK023797 | KIAA0672 | Homo sapiens cDNA FLJ13735 fis, clone PLACE3000155, weakly similar to Homo sapiens mRNA for KIAA0672 protein. [AK023797] |
| A_23_P123848 | 4.32E-04 | 3.2 | NM_032552 | DAB2IP | Homo sapiens DAB2 interacting protein (DAB2IP), transcript variant 1, mRNA [NM_032552] |
| A_24_P270235 | 4.37E-04 | 5.1 | NM_001759 | CCND2 | Homo sapiens cyclin D2 (CCND2), mRNA [NM_001759] |
| A_24_P149704 | 4.62E-04 | 3.1 | NM_138709 | DAB2IP | Homo sapiens DAB2 interacting protein (DAB2IP), transcript variant 2, mRNA [NM_138709] |
| A_23_P121665 | 4.70E-04 | 6.6 | NM_020777 | SORCS2 | Homo sapiens sortilin-related VPS10 domain containing receptor 2 (SORCS2), mRNA [NM_020777] |
| A_23_P133068 | 4.72E-04 | 3.6 | NM_001148 | ANK2 | Homo sapiens ankyrin 2, neuronal (ANK2), transcript variant 1, mRNA [NM_001148] |
| A_32_P372337 | 4.98E-04 | 8.3 | ENST00000333010 | ENST00000333010 | Janus kinase and microtubule-interacting protein 2. [Source:Uniprot/SWISSPROT;Acc:Q96AA8] [ENST00000333010] |
| A_23_P351667 | 5.16E-04 | 10.2 | NM_003812 | ADAM23 | Homo sapiens ADAM metallopeptidase domain 23 (ADAM23), mRNA [NM_003812] |
| A_24_P334300 | 5.22E-04 | 8.5 | NM_004113 | FGF12 | Homo sapiens fibroblast growth factor 12 (FGF12), transcript variant 2, mRNA [NM_004113] |
| A_32_P229618 | 5.40E-04 | 4.9 | NM_001364 | DLG2 | Homo sapiens discs, large homolog 2, chapsyn-110 (Drosophila) (DLG2), mRNA [NM_001364] |
| A_32_P228206 | 5.45E-04 | 3.1 | THC2463424 | THC2463424 | AA348270 EST54713 Hippocampus I Homo sapiens cDNA 3' end similar to EST containing Alu repeat, mRNA sequence [AA348270] |

Down-regulated genes in metastatic GISTs

| Probe Name | Corrected p-value | Fold Change | Genbank Acession | Gene Symbol | Description |
|---|---|---|---|---|---|
| A_32_P21354 | 5.47E-04 | 6.9 | THC2688038 | THC2688038 | |
| A_23_P368154 | 5.50E-04 | 9.4 | NM_153703 | PODN | Homo sapiens podocan (PODN), mRNA [NM_153703] |
| A_24_P84668 | 5.85E-04 | 3.8 | NM_015687 | FILIP1 | Homo sapiens filamin A interacting protein 1 (FILIP1), mRNA [NM_015687] |
| A_23_P97990 | 7.14E-04 | 5.3 | NM_002775 | HTRA1 | Homo sapiens HtrA serine peptidase 1 (HTRA1), mRNA [NM_002775] |
| A_24_P192627 | 7.15E-04 | 3.4 | NM_004529 | MLLT3 | Homo sapiens myeloid/lymphoid or mixed-lineage leukemia (trithorax homolog, Drosophila); translocated to, 3 (MLLT3), mRNA [NM_004529] |
| A_23_P110151 | 7.49E-04 | 4.0 | NM_031305 | ARHGAP24 | Homo sapiens Rho GTPase activating protein 24 (ARHGAP24); transcript variant 2, mRNA [NM_031305] |
| A_24_P187799 | 7.72E-04 | 3.4 | NM_024913 | FLJ21986 | Homo sapiens hypothetical protein FLJ21986 (FLJ21986), mRNA [NM_024913] |
| A_32_P60065 | 8.02E-04 | 14.2 | NM_004101 | F2RL2 | Homo sapiens coagulation factor II (thrombin) receptor-like 2 (F2RL2), mRNA [M_004101] |
| A_23_P127915 | 8.60E-04 | 4.3 | NM_030906 | STK33 | Homo sapiens serine/threonine kinase 33 (STK33), mRNA [NM_030906] |
| A_23_P54469 | 8.63E-04 | 4.3 | NM_45805 | ISL2 | Homo sapiens ISL2 transcription factor, LIM/homeodomain, (islet-2) (ISL2), mRNA [NM_145805] |
| A_24_P100996 | 8.75E-04 | 3.6 | ENST00000324559 | TMEM16E | Transmembrane protein 16E (Gnathodiaphyseal dysplasia 1 protein). [Source:Uniprot/SWtSSPROT;Acc:Q75V66] [ENST00000324559] |
| A_23_P57155 | 8.86E-04 | 6.6 | NM_001819 | CHGB | Homo sapiens chromogranin B (secretogranin 1) (CHGB), mRNA [NM_001819] |
| A_24_P380061 | 9.14E-04 | 3.9 | NM_031305 | ARHGAP24 | Homo sapiens Rho GTPase activating protein 24 (ARHGAP24), transcript variant 2, mRNA [NM_031305] |
| A_23_P382584 | 9.27E-04 | 7.3 | NM_001819 | CHGB | Homo sapiens chromogranin B (secretogranin 1) (CHGB), mRNA [M_001819] |
| A_32_P310335 | 9.32E-04 | 4.6 | AK056079 | AK056079 | Homo sapiens cDNA FLJ31517 fis, clone NT2RI2000007. [AK056079] |

EP 2 630 259 B1

Concerning the 70 down-regulated genes, no significantly enriched pathways were identified. In contrast, we observed that 45 of the 227 up-regulated genes belonged to the CINSARC signature (Figure 6). Furthermore, Gene Ontology analysis revealed that pathways enriched in this gene selection (227 metastasis up-regulated genes) were almost all the same as those enriched in the CINSARC signature. Actually, 63 of the 77 (82%) enriched pathways were common with CINSARC genes (Table 3).

Table 3: Comparison of enriched pathways (Gene Ontology analysis) in CINSARC genes and in t-test comparing tumors according to outcome and to p16/Rb1 pathway inactivation.

| Go Accession | GO Term | CINSARC | | | Metastatic GISTs | | | GISTs with p16/R81 pathway altercation | | | Array | |
|---|---|---|---|---|---|---|---|---|---|---|---|---|
| | | corrected p-value | Count in Selection | % in Selection | corrected p-value | Count in Selection | % in Selection | corrected p-value | Count in Selection | % in Selection | Count in Array | % in Array |
| GO:0000279 | M phase | 0 | 39 | 59,09 | 0 | 46 | 42,20 | 4,41 E-38 | 42 | 36,84 | 219 | 1,42 |
| GO:0022402 | cell cycle process | 0 | 42 | 63,64 | 0 | 46 | 42,20 | 4,65E-32 | 42 | 36,84 | 343 | 2,22 |
| GO:0022403 | cell cycle phase | 0 | 41 | 62,12 | 0 | 46 | 42,20 | 5,14E-37 | 42 | 36,84 | 267 | 1,73 |
| GO:0000278 | mitotic cell cycle | 0 | 38 | 57,58 | 9,03E-41 | 39 | 35,78 | 1,08E-32 | 36 | 31,58 | 221 | 1,43 |
| GO:0007049 | cell cycle | 0 | 47 | 71,21 | 1,44E-40 | 57 | 52,29 | 4,90E-31 | 54 | 47,37 | 584 | 3,78 |
| GO:0000087 | M phase of mitotic cell cycle | 0 | 34 | 51,52 | 5,12E-39 | 38 | 34,86 | 3,26E-31 | 35 | 30,70 | 163 | 1,06 |
| GO:0007 67 | mitosis | 0 | 34 | 51,52 | 9,10E-38 | 36 | 33,03 | 3,32E-30 | 33 | 28,95 | 160 | 1,04 |
| GO:0051301 | cell division | 0 | 36 | 54,55 | 2,45E-27 | 32 | 29,36 | 1,16E-24 | 33 | 28,95 | 209 | 1,35 |
| GO:0044427 | chromosomal part | 9,22E-16 | 14 | 21,21 | 7,80E-20 | 16 | 14,68 | 2,36E-11 | 15 | 13,16 | 270 | 1,75 |
| GO:0000775 | chromosome, centromeric region | 1,53E-16 | 14 | 21,21 | 1,57E-19 | 16 | 14,68 | 3,65E-14 | 15 | 13,16 | 66 | 0,43 |
| GO:0005694 | chromosome | 9,22E-16 | 17 | 25,76 | 5,47E-19 | 20 | 18,35 | 1,49E-11 | 20 | 17,54 | 318 | 2,06 |
| GO:0007059 | chromosome segregation | 1,90E-08 | 6 | 9,09 | 2,43E-17 | 13 | 11,93 | 2,91E-12 | 7 | 6,14 | 58 | 0,38 |
| GO:0043228 | non-membrane-bounded organelle | 1,83E-23 | 37 | 56,06 | 1,76E-15 | 35 | 32,11 | 1,47E-13 | 41 | 35,96 | 1509 | 9,77 |
| GO:0043232 | intracellular non-membrane-bounded organelle | 1,83E-23 | 37 | 56,06 | 1,76E-15 | 35 | 32,11 | 1,47E-13 | 41 | 35,96 | 1509 | 9,77 |

| Go Accession | GO Term | CINSARC | | | Metastatic GISTs | | | GISTs with p16/R81 pathway alteration | | | Array | |
|---|---|---|---|---|---|---|---|---|---|---|---|---|
| | | corrected p-value | Count in Selection | % in Selection | corrected p-value | Count in Selection | % in Selection | corrected p-value | Count in Selection | % in Selection | Count in Array | % in Array |
| GO:0007346 | regulation of mitotic cell cycle | 2,43E-17 | 9 | 13,64 | 4,17E-15 | 8 | 7,34 | 1,12E-11 | 4 | 3,51 | 77 | 0,50 |
| GO:0051726 | regulation of cell cycle | 3,31E-14 | 19 | 28,79 | 4,10E-15 | 22 | 20,18 | 8,55E-11 | 21 | 18,42 | 437 | 2,83 |
| GO:0005634 | nucleus | 2,84E-08 | 44 | 66,67 | 1,39E-12 | 80 | 73,39 | 2,44E-05 | 82 | 71,93 | 3992 | 25,84 |
| GO:0015630 | microtubule cytoskeleton | 2,33E-24 | 23 | 34,85 | 1,33E-11 | 18 | 16,51 | 8,53E-11 | 18 | 15,79 | 314 | 2,03 |
| GO:0006996 | organelle organization and biogenesis | 6,03E-14 | 23 | 34,85 | 2,30E-11 | 27 | 24,77 | 4,50E-08 | 25 | 21,93 | 979 | 6,34 |
| GO:0007017 | microtubule-based process | 2,92E-19 | 18 | 27,27 | 3,38E-11 | 16 | 14,68 | 4,07E-10 | 17 | 14,91 | 178 | 1,15 |
| GO:0044446 | intracellular organelle part | 9,22E-16 | 33 | 50,00 | 1,15E-10 | 31 | 28,44 | 1,05E-04 | 30 | 26,32 | 2239 | 14,49 |
| GO:0044422 | organelle part | 9,22E-16 | 33 | 50,00 | 1,22E-10 | 31 | 28,44 | 1,12E-04 | 30 | 26,32 | 2244 | 14,53 |
| GO:0000070 | mitotic sister chromatid segregation | 5,21E-04 | 2 | 3,03 | 3,72E-10 | 9 | 8,26 | 7,51E-06 | 3 | 2,63 | 28 | 0,18 |
| GO:0000819 | sister chromatid segregation | 6,05E-04 | 2 | 3,03 | 5,39E-10 | 9 | 8,26 | 9,60E-06 | 3 | 2,63 | 29 | 0,19 |
| GO:0051276 | chromosome organization and biogenesis | 9,76E-04 | 6 | | 8,55E-10 | 14 | 12,84 | 4,19E-05 | 9 | 7,89 | 347 | 2,25 |
| GO:0007051 | spindle organization and biogenesis | 2,11E-17 | 10 | 15,15 | 9,02E-10 | 6 | 5,50 | 6,38E-07 | 5 | 4,39 | 21 | 0,14 |
| GO:0006259 | DNA metabolic process | 6,01 E-05 | 10 | 15,15 | 1,91E-09 | 22 | 20,18 | 1,74E-05 | 12 | 10,53 | 400 | 2,59 |
| GO:0005819 | spindle | 2,37E-22 | 13 | 19,70 | 7,92E-09 | 10 | 9,17 | 1,85E-07 | 8 | 7,02 | 51 | 0,33 |
| GO:0044430 | cytoskeletal part | 4,65E-18 | 22 | 33,33 | 3,50E-08 | 17 | 15,60 | 1,49E-07 | 17 | 14,91 | 548 | 3,55 |

EP 2 630 259 B1

| Go Accession | GO Term | CINSARC | | | Metastatic GISTs | | | GISTs with p16/R81 pathway alteration | | | Array | |
|---|---|---|---|---|---|---|---|---|---|---|---|---|
| | | corrected p-value | Count in Selection | % in Selection | corrected p-value | Count in Selection | % in Selection | corrected p-value | Count in Selection | % in Selection | Count in Array | % in Array |
| GO:0010564 | regulation of cell cycle process | 0,00474 | 2 | 3,03 | 6,07E-08 | 4 | 3,67 | 9,40E-07 | 4 | 3,51 | 45 | 0,29 |
| GO:0007 88 | regulation of mitosis | 0,00152 | 2 | 3,03 | 1,63E-07 | 4 | 3,67 | 1,88E-06 | 4 | 3,51 | 35 | 0,23 |
| GO:0016043 | cellular component organization and biogenesis | 1,46E-09 | 23 | 34,85 | 1,89E-07 | 28 | 25,69 | 7,19E-05 | 26 | 22,81 | 1450 | 9,39 |
| GO:0000226 | microtubule cytoskeleton organization and biogenesis | 3,57E-16 | 12 | 18,18 | 2,64E-07 | 6 | 5,50 | 6,19E-05 | 5 | 4,39 | 70 | 0,45 |
| GO:0007126 | meiosis | 0,01027 | 2 | 3,03 | 2,99E-07 | 7 | 6,42 | 6,88E-05 | 6 | 5,26 | 53 | 0,34 |
| GO:0051327 | M phase of meiotic cell cycle | 0,01027 | 2 | 3,03 | 2,99E-07 | 7 | 6,42 | 6,88E-05 | 6 | 5,26 | 53 | 0,34 |
| GO:0051321 | meiotic cell cycle | 0,01113 | 2 | 3,03 | 3,54E-07 | 7 | 6,42 | 7,78E-05 | 6 | 5,26 | 54 | 0,35 |
| GO:0000075 | cell cycle checkpoint | 7,52E-10 | 3 | 4,55 | 6,47E-07 | 4 | 3,67 | 3,89E-07 | 1 | 0,88 | 41 | 0,27 |
| GO:0007010 | cytoskeleton organization and biogenesis | 2,67E-13 | 18 | 27,27 | 1,72E-06 | 16 | 14,68 | 1,88E-06 | 18 | 15,79 | 423 | 2,74 |
| GO:0006260 | DNA replication | 0,00227 | 8 | 12,12 | 2,94E-06 | 11 | 10,09 | 2,30E-02 | 8 | 7,02 | 169 | 1,09 |
| GO:0005524 | ATP binding | 4,57E-10 | 28 | 42,42 | 3,20E-016 | 36 | 33,03 | 8,83E-03 | 35 | 30,70 | 1268 | 8,21 |
| GO:0032559 | adenyl ribonucleotide binding | 5,80E-10 | 28 | 42,42 | 4,22E-06 | 36 | 33,03 | 1,09E-02 | 35 | 30,70 | 1282 | 8,30 |
| GO:0003777 | microtubule motor activity | 2,08E-07 | 9 | 13,64 | 1,02E-05 | 10 | 9,17 | 8,79E-07 | 12 | 10,53 | 76 | 0,49 |
| GO:0030554 | adenyl nucleotide binding | 1,80E-09 | 28 | 42,42 | 1,60E-05 | 36 | 33,03 | 3,12E-02 | 35 | 30,70 | 1349 | 8,73 |

EP 2 630 259 B1

(continued)

| Go Accession | GO Term | CINSARC | | | Metastatic GISTs | | | GISTs with p16/R81 pathway alteration | | | Array | |
|---|---|---|---|---|---|---|---|---|---|---|---|---|
| | | corrected p-value | Count in Selection | % in Selection | corrected p-value | Count in Selection | % in Selection | corrected p-value | Count in Selection | % in Selection | Count in Array | % in Array |
| GO:0043226 | organelle | 9,92E-07 | 54 | 81,82 | 1,61E-05 | 88 | 80,73 | 5,91E-02 | 97 | 85,09 | 6717 | 43,48 |
| GO:0043229 | intracellular organelle | 9,92E-07 | 54 | 81,82 | 1,61E-05 | 88 | 80,73 | 5,91 E-02 | 97 | 85,09 | 6715 | 43,47 |
| GO:0006974 | response to DNA damage stimulus | | | | 1,86E-05 | 13 | 11,93 | 1,81 E-02 | 1 | 0,88 | 270 | 1,75 |
| GO:0045840 | positive regulation of mitosis | | | | 3,96E-05 | 1 | 0,92 | 1,53E-04 | 1 | 0,88 | 9 | 0,06 |
| GO:0070018 | microtubule-based movement | 4,49E-08 | 9 | 13,64 | 6,25E-05 | 10 | 9,17 | 8,26E-06 | 12 | 10,53 | 93 | 0,60 |
| GO:0043227 | membrane-bounded organelle | 0,002856 | 44 | 66,67 | 6,25E-05 | 80 | 73,39 | | | | 5904 | 38,22 |
| GO:0043231 | intracellular membrane-bounded organelle | 0,002850 | 44 | 66,67 | 6,25E-05 | 80 | 73,39 | | | | 5901 | 38,20 |
| GO:0030261 | chromosome condensation | | | | 8,82E-05 | 5 | 4,59 | | | | 20 | 0,13 |
| GO:0005874 | microtubule | 7,54E-10 | 14 | 21,21 | 1,53E-04 | 12 | 11,01 | 5,35E-04 | 13 | 11,40 | 198 | 1,28 |
| GO:0007093 | mitotic cell cycle checkpoint | 1,95E-06 | 3 | 4,55 | 1,61E-04 | 4 | 3,67 | | | | 22 | 0,14 |
| GO:0005856 | cytoskeleton | 7,31E-14 | 23 | 34,85 | 1,65E-04 | 18 | 16,51 | 2,41E-07 | 23 | 20,18 | 899 | 5,82 |
| GO:0005875 | microtubule associated complex | 4,99E-06 | 9 | 13,64 | 2,84E-04 | 10 | 9,17 | 5,02E-05 | 8 | 7,02 | 110 | 0,71 |
| GO:0030705 | cytoskeleton-dependent intracellular transport | 2,58E-07 | 9 | 13,64 | 3,32E-04 | 10 | 9,17 | 6,01 E-05 | 12 | 10,53 | 112 | 0,73 |
| GO:0044424 | intracellular part | 6,64E-05 | 55 | 83,33 | 3,79E-04 | 88 | 80,73 | | | | 7677 | 49,70 |

| Go Accession | GO Term | CINSARC | | | Metastatic GISTs | | | GISTs with p16/R81 pathway alteration | | | Array | |
|---|---|---|---|---|---|---|---|---|---|---|---|---|
| | | corrected p-value | Count in Selection | % in Selection | corrected p-value | Count in Selection | % in Selection | corrected p-value | Count in Selection | % in Selection | Count in Array | % in Array |
| GO:0050000 | chromosome localization | | | | 4,16E-04 . | 1 | 0,92 | 1,42E-03 | 1 | 0,88 | 6 | 0,04 |
| GO:0051303 | establishment of chromosome localization | | | | 4,16E-04 | 1 | 0,92 | 1,42E-03 | 1 | 0,88 | 6 | 0,04 |
| GO:0051656 | establishment of organelle localization | | | | 5,46E-04 | 1 | 0,92 | 3,04E-03 | 1 | 0,88 | 27 | 0,17 |
| GO:0006281 | DNA repair | | | | 5,57E-04 | 12 | 11,01 | | | | 224 | 1,45 |
| GO:0051640 | organelle localization | | | | 6,68E-04 | 1 | 0,92 | 3,76E-03 | 1 | 0,88 | 28 | 0,18 |
| GO:0032553 | ribonucleotide binding | 1,01E-07 | 28 | 42,42 | 8,49E-04 | 36 | 33,03 | | | | 1600 | 10,36 |
| GO:0032555 | purine ribonucleotide binding | 1,01E-07 | 28 | 42,42 | 8,49E-04 | 36 | 33,03 | | | | 1600 | 10,36 |
| GO:0007076 | mitotic chromosome condensation | | | | 9,01E-04 | 5 | 4,59 | | | | 16 | 0,10 |
| GO:0045787 | positive regulation of cell cycle | | | | 9,01E-04 | 1 | 0,92 | 4,14E-03 | 1 | 0,88 | 16 | 0,10 |
| GO:0005622 | intracellular | 3,09E-04 | 56 | 84,85 | 0,00174 | 91 | 83,49 | | | | 8242 | 53,35 |
| GO:0003774 | motor activity | 3,37E-05 | 9 | 13,64 | 0,00183 | 10 | 9,17 | 6,12E-05 | 13 | 11,40 | 137 | 0,89 |
| GO:0005876 | spindle microtubule | 7,68E-07 | 6 | 9,09 | 0,00219 | 5 | 4,59 | 1,03E-02 | 5 | 4,39 | 19 | 0,12 |
| GO:0017076 | purine nucleotide binding | 2,58E-07 | 28 | 42,42 | 0,00219 | 36 | 33,03 | | | | 1669 | 10,80 |
| GO:0007052 | mitotic spindle organization and biogenesis | 6,52E-04 | 4 | 6,06 | 0,01084 | 2 | 1,83 | 3,58E-02 | 2 | 1,75 | 12 | 0,08 |

EP 2 630 259 B1

(continued)

| Go Accession | GO Term | CINSARC | | | Metastatic GISTs | | | GISTs with p16/R81 pathway alteration | | | Array | |
|---|---|---|---|---|---|---|---|---|---|---|---|---|
| | | corrected p-value | Count in Selection | % in Selection | corrected p-value | Count in Selection | % in Selection | corrected p-value | Count in Selection | % in Selection | Count in Array | % in Array |
| GO:00009110 | cytokinesis | 0,016369 | 4 | 6,06 | 0,01393 | 4 | 3,67 | 5,72E-02 | 4 | 3,51 | 27 | 0,17 |
| GO:0006310 | DNA recombination | | | | 0,01520 | 4 | 3,67 | | | | 73 | 0,47 |
| GO:0006323 | DNA packaging | | | | 0,02611 | 5 | 4,59 | | | | 111 | 0,72 |
| GO:0000166 | nucleotide binding | 5,35E-06 | 28 | 42,42 | 0,04644 | 36 | 33,03 | | | | 1913 | 12,38 |
| GO:0007094 | mitotic cell cycle spindle assembly checkpoint | | | | 0,04644 | 3 | 2,75 | | | | 6 | 0,04 |
| GO:0031577 | spindle checkpoint | | | | 0,04644 | 3 | 2,75 | | | | 6 | 0,04 |
| GO:0000776 | kinetochore | 0,00036 | 4 | 6,06 | | | | | | | 26 | 0,17 |
| GO:0004672 | protein kinase activity | 0,01408 | 11 | 16,67 | | | | | | | 566 | 3,66 |
| GO:0004674 | protein serine/threonine kinase activity | 0,00054 | 11 | 16,67 | | | | | | | 403 | 2,61 |
| GO:0005515 | protein binding | 0,00105 | 40 | 60,61 | | | | | | | 6165 | 39,91 |
| GO:0005813 | centrosome | 0,00175 | 4 | 6,06 | | | | | | | 68 | 0,44 |
| GO:0005815 | microtubule organizing center | 0,00022 | 4 | 6,06 | | | | | | | 79 | 0,51 |
| GO:0006270 | DNA replication initiation | 0,00741 | 4 | 6,06 | | | | | | | 22 | 0,14 |
| GO:0006468 | protein amino acid | 0,01889 | 12 | 18,18 | | | | | | | 584 | 3,78 |

EP 2 630 259 B1

| Go Accession | GO Term | CINSARC | | | Metastatic GISTs | | | GISTs with p16/R81 pathway alteration | | | Array | |
|---|---|---|---|---|---|---|---|---|---|---|---|---|
| | | corrected p-value | Count in Selection | % in Selection | corrected p-value | Count in Selection | % in Selection | corrected p-value | Count in Selection | % in Selection | Count in Array | % in Array |
| GO:0007089 | phosphorylation traversing start control point of mitotic cell cycle | 0,00529 | 3 | 4,55 | | | | | | | 6 | 0,04 |
| GO:0007096 | regulation of exit from mitosis | 0,03771 | 1 | 1,52 | | | | | | | 11 | 0,07 |
| GO:0009987 | cellular process | 0,00000 | 60 | 90,91 | | | | | | | 9867 | 63,87 |
| GO:0019932 | second-messenger-mediated signaling | 0,03778 | 7 | 10,61 | | | | | | | 182 | 1,18 |
| GO:0032991 | macromolecular complex | 0,04542 | 15 | 22,73 | | | | | | | 1992 | 12,89 |
| GO:0043234 | protein complex | 0,03040 | 15 | 22,73 | | | | | | | 1493 | 9,66 |
| GO:0048015 | phosphoinositide-mediated signaling | 0,00030 | 7 | 10,61 | | | | | | | 83 | 0,54 |
| GO:0051325 | interphase | 0,00202 | 6 | 9,09 | | | | | | | 70 | 0,45 |
| GO:0051329 | interphase of mitotic cell cycle | 0,00163 | 6 | 9,09 | | | | | | | 67 | 0,43 |

EP 2 630 259 B1

36

Moreover, gene enrichment analysis of the 182 genes not included in CINSARC showed that this gene set was also enriched by genes involved in the same pathways as CINSARC genes, i.e. mitosis control and chromosome integrity (Table 4).

**Table 4**: Gene Ontology analysis of the 182 genes differentially expressed between GISTs with or without metastasis and not included in CINSARC signature

| GO ACCESSION | GO Term | p-value | corrected p-value | Count in Selection | % in Selection | Count in Array | % in Array |
|---|---|---|---|---|---|---|---|
| GO:0022403 | cell cycle phase | 5.72E-21 | 1.48E-15 | 22 | 36.07 | 267 | 1.73 |
| GO:0000279 | M phase | 2.61E-20 | 3.37E-15 | 22 | 36.07 | 219 | 1.42 |
| GO:0007049 | cell cycle | 3.45E-19 | 2.97E-14 | 29 | 47.54 | 584 | 3.78 |
| GO:0022402 | cell cycle process | 2.05E-18 | 1.32E-13 | 22 | 36.07 | 343 | 2.22 |
| GO:0044427 | chromosomal part | 9.72E-15 | 5.01E-10 | 9 | 14.75 | 270 | 1.75 |
| GO:0000278 | mitotic cell cycle | 6.57E-14 | 2.82E-09 | 15 | 24.59 | 221 | 1.43 |
| GO:0007059 | chromosome segregation | 9.42E-14 | 3.47E-09 | 7 | 11.48 | 58 | 0.38 |
| GO:0000087 | M phase of mitotic cell cycle | 1.59E-13 | 5.11E-09 | 15 | 24.59 | 163 | 1.06 |
| GO:0005694 | chromosome | 1.88E-13 | 5.39E-09 | 12 | 19.67 | 318 | 2.06 |
| GO:0007067 | mitosis | 2.17E-12 | 5.59E-08 | 13 | 21.31 | 160 | 1.04 |
| GO:0000775 | chromosome, centromeric region | 1.41E-11 | 3.31 E-07 | 9 | 14.75 | 66 | 0.43 |
| GO:0006259 | DNA metabolic process | 9.77E-11 | 2.10E-06 | 16 | 26.23 | 400 | 2.59 |
| GO:0051726 GO:0000074 | regulation of cell cycle | 4.09E-10 | 8.06E-06 | 12 | 19.67 | 437 | 2.83 |
| GO:0000070| GO:0016359 | mitotic sister chromatid segregation | 4.38E-10 | 8.06E-06 | 6 | 9.84 | 28 | 0.18 |
| GO:0000819 | sister chromatid segregation | 5.74E-10 | 9.86E-06 | 6 | 9.84 | 29 | 0.19 |
| GO:0051276| GO:0007001| GO:0051277 | chromosome organization and biogenesis | 8.43E-10 | 1.28E-05 | 9 | 14.75 | 347 | 2.25 |
| GO:0005634 | nucleus | 8.28E-10 | 1.28E-05 | 52 | 85.25 | 3992 | 25.84 |
| GO:0051301 | cell division | 1.00E-09 | 1.44E-05 | 12 | 19.67 | 209 | 1.35 |
| GO:0051327 | M phase of meiotic cell cycle | 1.75E-09 | 2.26E-05 | 7 | 11.48 | 53 | 0.34 |
| GO:0007126 | meiosis | 1.75E-09 | 2.26E-05 | 7 | 11.48 | 53 | 0.34 |
| GO:0051321 | meiotic cell cycle | 2.05E-09 | 2.51E-05 | 7 | 11.48 | 54 | 0.35 |
| GO:0007346 | regulation of mitotic cell cycle | 3.66E-08 | 4.29E-04 | 3 | 4.92 | 77 | 0.50 |
| GO:0006260 | DNA replication | 1.53E-07 | 1.72E-03 | 7 | 11.48 | 169 | 1.09 |
| GO:0006974 | response to DNA damage stimulus | 1.91E-07 | 2.05E-03 | 10 | 16.39 | 270 | 1.75 |
| GO:0043232 | intracellular non-membrane-bounded organelle | 2.40E-07 | 2.38E-03 | 12 | 19.67 | 1509 | 9.77 |
| GO:0043228 | non-membrane-bounded organelle | 2.40E-07 | 2.38E-03 | 12 | 19.67 | 1509 | 9.77 |
| GO:0010564 | regulation of cell cycle process | 4.46E-07 | 4.25E-03 | 3 | 4.92 | 45 | 0.29 |
| GO:0006281 | DNA repair | 2.04E-06 | 1.88E-02 | 9 | 14.75 | 224 | 1.45 |
| GO:0007076 | mitotic chromosome condensation | 2.95E-06 | 2.50E-02 | 4 | 6.56 | 16 | 0.10 |

| GO ACCESSION | GO Term | p-value | corrected p-value | Count in Selection | % in Selection | Count in Array | % in Array |
|---|---|---|---|---|---|---|---|
| GO:0007088 | regulation of mitosis | 3.00E-06 | 2.50E-02 | 3 | 4.92 | 35 | 0.23 |
| GO:0044446 | intracellular organelle part | 2.98E-06 | 2.50 E-02 | 9 | 14.75 | 2239 | 14.49 |
| GO:0044422 | organelle part | 3.13E-06 | 2.52E-02 | 9 | 14.75 | 2244 | 14.53 |
| GO:0006996 | organelle organization and biogenesis | 4.43E-06 | 3.46E-02 | 9 | 14.75 | 979 | 6.34 |
| GO:0030261|GO:0000068 | chromosome condensation | 7.69E-06 | 5.51 E-02 | 4 | 6.56 | 20 | 0.13 |
| GO:0006310 | DNA recombination | 8.03E-06 | 5.60E-02 | 5 | 8.20 | 73 | 0.47 |

***AURKA is a significant marker of metastasis outcome***

We took advantage of the supervised analysis results to test the possibility of reducing the CINSARC signature. Among the top-ranked significant genes sorted in the supervised t-test, *AURKA* (Aurora kinase A, previously designated STK6 or STK15) was the best ranked gene that also belonged to the CINSARC signature (Table 1). We thus tested whether *AURKA* alone could predict outcomes as well as CINSARC and we stratified samples according to their *AURKA* expression (with the mean expression of 9.13 as a cut-off, table 5).

Table 5: Expression of p16 and RB1 measured by expression array and by RT-qPCR. Expression array data are log2 transformed and RT-qPCR data are difference between tested gene and reference genes CTs, that means that the highest the value, the lowest the expression. High expressions are indicated in red and low expressions in green. Main clinical data and results are reported from table 1. *p16* and *RB1* copy number: 2 = without detectable deletion; 1 = hemizygous deletion, *indicate truncating mutation; 0 = no copy. nd = not done.

| GIST | Expression (Agilent) | | | | CGH | | | | CRLM/PCR copy number | | | | Histology | Site of primary tumor | Local recurrence | Metastasis | Mutated gene | Mutation |
|---|---|---|---|---|---|---|---|---|---|---|---|---|---|---|---|---|---|---|
| GIST1 | 8.68 | C: | 8.12 | A: | 3 | 3 | 3 | GH | AGI | 2 | 2 | 2 | 1 | high risk | Stomach | No | No | P18 | p.D842V |
| GIST10 | 7.95 | C: | 8.56 | A: | 5 | 4 | 6.25 | GH | AGI | 2 | 2 | 2 | 2 | low risk | small intestine | No | No | K11 | p.V560D |
| GIST13 | 8.84 | C: | 8.05 | A: | 2 | 2 | 2 | GH | AGI | 2 | 2 | 2 | 2 | intermediate | stomach | No | No | K11 | p.W557R |
| GIST15 | 9.37 | C: | 7.89 | A: | 4 | 3 | 5.33 | GH | AGI | 2 | 2 | 2 | 2 | low risk | stomach | No | No | K11 | p.V559D |
| GIST18 | 8.93 | C: | 9.05 | A: | 6 | 4 | 9 | GH | AGI | 2 | 2 | 2 | 2 | intermediate | duodenum | No | No | K11 | p.L576P |
| GIST21 | 9.41 | C: | 8.66 | A: | 2 | 2 | 2 | GH | AGI | 2 | 2 | 2 | 2 | intermediate | stomach | No | No | K11 | p.L576P |
| GIST23 | 8.42 | C: | 8.39 | A: | 0 | 0 | 0 | GH | AGI | nd | nd | nd | 2 | low risk | small intestine | No | No | P12 | p.Y555C |
| GIST24 | 9.04 | C: | 8.23 | A: | 4 | 4 | 4 | GH | AGI | 2 | 2 | 2 | 2 | low risk | peritoneum | No | No | K11 | p.T574_R586insK |
| GIST27 | 8.67 | C: | 7.75 | A: | 1 | 1 | 1 | GH | AGI | 2 | 2 | 2 | 2 | high risk | stomach | No | No | K11 | p.K581_S590dup |
| GIST30 | 8.31 | C: | 7.62 | A: | 2 | 2 | 2 | GH | AGI | 2 | 2 | 2 | 2 | intermediate | stomach | No | No | K11 | p.L576_R588dup |
| GIST32 | 9.15 | C: | 8.09 | A: | 1 | 1 | 1 | GH | AGI | 2 | 2 | 2 | 2 | intermediate | stomach | No | No | K11 | p.W557R |
| GIST33 | 9.02 | C: | 8.55 | A: | 3 | 3 | 3 | GH | AGI | 2 | 2 | 2 | 2 | very low | stomach | No | No | P18 | p.D842V |
| GIST36 | 8.34 | C: | 7.61 | A: | 1 | 1 | 1 | GH | AGI | 2 | 2 | 2 | 2 | very low | stomach | No | No | K11 | p.V559D |
| GIST40 | 8.52 | C: | 7.80 | A: | 1 | 1 | 1 | GH | AGI | 2 | 2 | 2 | 2 | low risk | stomach | No | No | K11 | p.P573_T574dup; T574dup; Q575_R586dup |
| GIST43 | 9.12 | C: | 8.01 | A: | 1 | 1 | 1 | GH | AGI | 2 | 2 | 2 | 2 | very low | stomach | No | No | K11 | p.T574_L589dup |
| GIST44 | 9.37 | C: | 8.41 | A: | 5 | 3 | 8.33 | GH | AGI | 2 | 2 | 2 | 2 | low risk | stomach | No | No | K11 | p.Q556_V559del |
| GIST46 | 9.20 | C: | 8.60 | A: | 5 | 3 | 8.33 | GH | AGI | 2 | 2 | 2 | 2 | very low | small intestine | No | No | K11 | p.Q556_V559del |
| GIST48 | 8.17 | C: | 8.14 | A: | 8 | 7 | 9.14 | GH | AGI | 2 | 2 | 2 | 2 | low risk | small intestine | No | No | K11 | p.M552_E561del |
| GIST49 | 9.35 | C: | 8.93 | A: | 7 | 5 | 9.8 | GH | AGI | 2 | 2 | 2 | 2 | very low | stomach | No | No | K11 | p.E554_K558del |
| GIST50 | 7.67 | C: | 8.36 | A: | 7 | 6 | 8.17 | GH | AGI | 1 | 1 | 1 | 2 | high risk | small intestine | No | No | K11 | p.M552_E554delinsK |
| GIST51 | 9.31 | C: | 8.33 | A: | 0 | 0 | 0 | GH | AGI | 2 | 2 | 2 | 2 | very low | stomach | No | No | K11 | p.W557R |
| GIST55 | 8.70 | C: | 7.72 | A: | 5 | 4 | 6.25 | GH | AGI | 2 | 2 | 2 | 2 | very low | stomach | No | No | K11 | p.D572_D579dupinsL |

| | | | | | | | | | | | | | | | | | | |
|---|---|---|---|---|---|---|---|---|---|---|---|---|---|---|---|---|---|---|
| GIST60 | 9.21 | C1 | 8.77 | A1 | 1 | 1 | 1 | GI1 | AG1 | 2 | 2 | 2 | 2 | very low | stomach | No | No | P18 | p.D842V |
| GIST62 | 9.47 | C1 | 8.30 | A1 | 1 | 1 | 1 | GI1 | AG1 | 2 | 2 | 2 | 2 | very low | stomach | No | No | K11 | p.N566_P573del |
| GIST64 | 9.31 | C1 | 8.60 | A1 | 5 | 5 | 5 | GI1 | AG1 | 2 | 2 | 2 | 2 | low risk | small intestine | No | No | K11 | p.V560D |
| GIST66 | 8.47 | C1 | 8.82 | A1 | 7 | 6 | 8.17 | GI1 | AG1 | 1 | 1 | 1 | 2 | low risk | duodenum | No | No | K11 | p.V559G |
| GIST8 | 8.90 | C1 | 7.71 | A1 | 1 | 1 | 1 | GI1 | AG1 | 2 | 2 | 2 | 2 | low risk | stomach | No | No | K11 | p.W557_K558del |
| GIST59 | 7.93 | C1 | 7.31 | A1 | 8 | 6 | 10.7 | GI2 | AG2 | 2 | 2 | 2 | 2 | very low | stomach | No | No | K11 | p.N567_L576delinsKE homo |
| GIST65 | 8.30 | C1 | 8.69 | A1 | 20 | 11 | 36.36 | GI2 | AG2 | 1 | 1 | 1 | 2 | intermediate | small intestine | No | No | K13 | p.K642E |
| GIST67 | 7.99 | C1 | 7.35 | A1 | 11 | 6 | 20.17 | GI2 | AG2 | 2 | 2 | 2 | 2 | low risk | Stomach | No | No | K11 | p.V560D |
| GIST39 | 8.80 | C1 | 8.88 | A1 | 12 | 11 | 13.09 | GI2 | AG2 | 1 | 1 | 1 | 2 | intermediate | stomach | No | Yes | K11 | p.W557_V559delins F |
| GIST25 | nd | nd | nd | nd | 0 | 0 | 0 | GI1 | nd | 2 | 2 | 2 | 2 | very low | stomach | No | No | P18 | p.D842V |
| GIST7 | nd | nd | nd | nd | 1 | 1 | 1 | GI1 | nd | 2 | 2 | 2 | 2 | intermediate | stomach | No | No | K11 | p.W557_E561del |
| GIST52 | 9.50 | C2 | 8.32 | A1 | nd | nd | nd | nd | nd | 2 | 2 | 2 | nd | very low | stomach | No | No | K11 | p.P573_H580ins |
| GIST12 | 9.00 | C2 | 8.66 | A1 | 0 | 0 | 0 | GI1 | AG1 | 2 | 2 | 2 | 2 | high risk | retroperitoneum | No | No | WT | WT |
| GIST29 | 9.65 | C2 | 8.48 | A1 | 2 | 2 | 2 | GI1 | AG1 | 2 | 2 | 2 | 2 | intermediate | stomach | No | No | K11 | p.D572_T574dup |
| GIST31 | 9.07 | C2 | 8.51 | A1 | 3 | 3 | 3 | GI1 | AG1 | 2 | 2 | 2 | 2 | low risk | stomach | No | No | P18 | p.I843_D846del |
| GIST4 | 9.63 | C2 | 9.06 | A1 | 2 | 2 | 2 | GI1 | AG1 | 2 | 2 | 2 | 2 | low risk | Stomach | No | No | K11 | p.V559D |
| GIST41 | 9.38 | C2 | 8.97 | A1 | 1 | 1 | 1 | GI1 | AG1 | 2 | 2 | 2 | 2 | low risk | stomach | No | No | P12 | p.D561V |
| GIST45 | 9.43 | C2 | 8.84 | A1 | 2 | 2 | 2 | GI1 | AG1 | 2 | 2 | 2 | 2 | very low | stomach | No | No | P18 | p.D842V |
| GIST35 | 9.32 | C2 | 8.85 | A1 | 6 | 5 | 7.2 | GI1 | AG1 | 2 | 2 | 2 | 1 | intermediate | stomach | No | No | P14 | p.N659K |
| GIST54 | 9.50 | C2 | 9.11 | A1 | 2 | 2 | 2 | GI1 | AG1 | 2 | 2 | 2 | 1 | very low | stomach | No | No | P18 | p.D842V |
| GIST20 | 9.60 | C2 | 9.02 | A1 | 9 | 5 | 16.2 | GI2 | AG2 | 2 | 2 | 2 | 2 | high risk | abdominal wall | No | No | K11 | p.W557R |
| GIST22 | 9.45 | C2 | 9.71 | A2 | 5 | 4 | 6.25 | GI1 | AG2 | 2 | 2 | 2 | 2 | intermediate | stomach | No | No | P18 | p.D842V |
| GIST42 | 9.89 | C2 | 9.50 | A2 | 2 | 2 | 2 | GI1 | AG2 | 1 | 1 | 1 | 2 | low risk | stomach | No | No | WT | WT |
| GIST6 | 11.51 | C2 | 12.11 | A2 | 13 | 11 | 15.36 | GI2 | AG2 | 2 | 2 | 2 | 0 | high risk | small intestine | Yes | No | K11 | p.E554_K558del |
| GIST53 | 11.01 | C2 | 10.10 | A2 | 4 | 4 | 4 | GI1 | AG2 | 0 | 0 | 0 | 2 | intermediate | stomach | No | No | K11 | p.Q556_I563del |
| GIST11 | 9.79 | C2 | 9.73 | A2 | 9 | 8 | 10.13 | GI2 | AG2 | nd | nd | nd | 2 | low risk | duodenum | No | No | K11 | p.V560A |
| GIST14 | 11.59 | C2 | 11.95 | A2 | 11 | 8 | 15.13 | GI2 | AG2 | 2 | 2 | 2 | 1 | intermediate | mesenterium | Yes | Yes | K17 | p.N822K |
| GIST16 | 9.60 | C2 | 9.70 | A2 | 8 | 6 | 10.67 | GI2 | AG2 | 2 | 2 | 2 | 1 | high risk | jejunum | No | Yes | K9 | p.A502_Y503dup |
| GIST19 | 11.45 | C2 | 12.01 | A2 | 29 | 17 | 49.47 | GI2 | AG2 | 2 | 2 | 2 | 1 | intermediate | colon | Yes | Yes | K9 | p.A502_Y503dup |
| GIST2 | 9.86 | C2 | 10.22 | A2 | 12 | 11 | 13.09 | GI2 | AG2 | 2 | 2 | 2 | 1 | high risk | small intestine | No | Yes | K11 | p.Y553_Q556del |
| GIST37 | 11.09 | C2 | 11.20 | A2 | 29 | 15 | 56.07 | GI2 | AG2 | 1 | 1 | 1 | 2 | intermediate | stomach | Yes | Yes | K11 | p.W557_K558del |
| GIST38 | 11.23 | C2 | 10.80 | A2 | 31 | 17 | 56.53 | GI2 | AG2 | 1 | 1 | 1 | 1 | high risk | stomach | No | Yes | K11 | p.W557_V560delinsF |
| GIST56 | 11.97 | C2 | 13.11 | A2 | 21 | 13 | 33.92 | GI2 | AG2 | 1 | 1 | 1 | 2 | high risk | small intestine | Yes | Yes | WT | WT |
| GIST61 | 12.74 | C2 | 12.89 | A2 | 26 | 17 | 39.76 | GI2 | AG2 | 1 | 1 | 1 | 1 | high risk | stomach | No | Yes | P18 | p.D842V |
| GIST63 | 10.87 | C2 | 10.70 | A2 | 5 | 4 | 6.25 | GI1 | AG2 | 1 | 1 | 1 | 2 | high risk | rectum | No | Yes | K11 | p.V560D |
| GIST9 | 11.36 | C2 | 11.67 | A2 | 16 | 10 | 25.6 | GI2 | AG2 | 1 | 1 | 1 | 1 | high risk | stomach | Yes | Yes | K11 | p.V560D |
| GIST28 | 10.73 | C2 | 10.76 | A2 | 14 | 9 | 21.78 | GI2 | AG2 | 0 | 0 | 0 | 2 | high risk | stomach | No | Yes | K11 | p.W557_V559delinsF |
| GIST47 | 10.32 | C2 | 9.64 | A2 | 22 | 12 | 40.33 | GI2 | AG2 | 0 | 0 | 0 | 2 | high risk | stomach | No | Yes | K11 | p.E554_D572delinsF |
| GIST5 | 10.45 | C2 | 9.92 | A2 | 5 | 4 | 6.25 | GI1 | AG2 | 0 | 0 | 1 | 2 | high risk | stomach | No | Yes | K11 | p.W557_K558 del |
| GIST58 | 10.86 | C2 | 10.19 | A2 | 17 | 8 | 36.13 | GI2 | AG2 | 0 | 0 | 0 | 2 | high risk | stomach | No | Yes | K11 | p.W557_K558delinsFP |
| GIST57 | nd | nd | nd | nd | 13 | 10 | 16.9 | GI2 | AG1 | 0 | 0 | 0 | 2 | high risk | small intestine | No | Yes | K11 | p.V559D |
| GIST17 | nd | nd | nd | nd | 26 | 13 | 52 | GI2 | AG2 | 0 | 0 | 0 | 2 | nd | duodenum | Yes | Yes | K11 | p.V569_L576del |
| GIST3 | nd | nd | nd | nd | 16 | 10 | 25.6 | GI2 | AG2 | 2 | 2 | 2 | 1 | high risk | Stomach | No | Yes | K11 | p.V560D |
| GIST26 | nd | nd | nd | nd | 11 | 7 | 17.29 | GI2 | AG2 | 2 | 2 | 2 | 1 | intermediate | mediastinum | No | No | K11 | p.K558_V559delinsN homo |
| GIST34 | nd | nd | nd | nd | 11 | 8 | 15.13 | GI2 | AG2 | 2 | 2 | 2 | 1 | very low | small intestine | No | No | K11 | p.V560D |

For this purpose, we considered the present 67-GISTs series as the training set and the Yamaguchi's one as the validation set. Expression data were then validated by qRT-PCR and we found a high correlation between both techniques (Pearson correlation coefficient = 0.94; $P < 1 \times 10^{-15}$). Survival analyses revealed that the two groups obtained had very different outcomes, both in the training set (Present series, MFS: $P = 5.31 \times 10^{-11}$ and DFS: $P = 3.61 \times 10^{-12}$, fig. 2a) and in the validation set (Yamaguchi's series, MFS: $P = 9.5 \times 10^{-4}$, fig. 2b).

### Chromosomal complexity is a significant prognosis factor of GISTs

We have previously shown that the CINSARC signature is associated with the genome complexity (18), therefore the question arises whether the alteration level of the GISTs genome is correlated with the CINSARC signature and with the metastatic outcome. Genome profiling with arrays containing 60 000 oligonucleotides (see material and methods) has been performed on 66 GISTs with sufficient DNA quality. Different profiles were obtained, ranging from simple, i.e. without any detectable changes, to complex, with numerical and segmental gains and losses (Figure 3). No high level amplification was detected across the 66 profiles with the exception of one case with 5p amplification (GIST17). Most of the alterations involved whole chromosome arms or chromosomes without rearrangements. In fact, when only a few changes were detected (less than eight), these always affected whole arms or chromosomes, whereas when the profile was composed of more than 10 changes, intra-chromosomal gains or losses could be observed. To define a numerical method taking into account these criteria, i.e. the number and type of alterations, a Genomic Index (GI) was calculated for each profile as follows: GI= $A^2$ x C (A= number of alterations and C= number of involved chromosomes). Then, tumors were assigned to two groups, GI1 or GI2, depending on whether their GI was not-greater or greater than 10, respectively (Table 5). Kaplan-Meier metastasis- and disease-free survival analyses demonstrated that this stratification split tumors into two groups with strongly distinct outcome (fig 4a). Moreover, this genomic stratification can identify GISTs with different metastatic outcomes in the intermediate-risk group of the AFIP classification (9) (fig 4b & c).

### Integrative analysis allows identification of a no-risk group of patients

Considering these results as a whole, we construct a decisional algorithm based on GI and *AURKA* expression. More specifically, a positive correlation exists between GI and *AURKA* expression (Pearson correlation r=0.65, Figure 7). We observed that tumors with below-average *AURKA* expression and with GI less than 10 never develop metastasis or recurrence (Figure 7, Table 5). In line with this, Kaplan-Meier MFS and DFS analyses demonstrated that tumors with good prognostic factors (AG1: *AURKA* expression <mean and GI<10), have a 5-years MFS of 100%, whereas tumors with poor prognostic factors (AG2: *AURKA* expression > mean or GI>10) have a 5-years MFS of 23%, $P = 2.61 \times 10^{-8}$ , Figure 5). Hence, this algorithm leads to the individualization of a no-risk patient group (AG 1: *A URKA* expression < 9.13 and GI<10).

### P16/RB1 pathway is associated to metastatic outcome.

As a result of these findings, we reconsidered CGH array data to examine whether any specific alterations were associated with patients' outcome. We compared alteration frequency of each probe set between GISTs with or without metastatic outcome (Figure 8). No significant difference in gain frequencies was observed between those two groups (data not shown). However, among the top-ranked deletion frequencies in metastatic cases, the highest difference was observed for eight probe sets deleted in 78.9% and 9.6% of the metastatic and non-metastatic cases, respectively (Figure 8). All probe sets localize in 9p21 and target either *CDKN2A* (3 probe sets), *CDKN2B* (3 probe sets) or *MTAP* (2 probe sets) loci. 9p21 deletions were observed in 18 cases (18/66 = 27%) and among these tumors, 13 developed metastasis (13/18 = 72%). These deletions either involved the whole 9p arms or were restricted only to the *CDKN2A/B* loci and were assumed to be homozygous for 7 cases (6/7 with metastatic outcome) as indicated by the very low CGH ratios (Figure 9). The most frequently deleted region appeared to involve 3 loci (*CDKN2A*, *CDKN2B* and *MTAP*), but homozygous deletions allowed us to identify more precisely genes of interest since two tumors with homozygous deletion excluded *MTAP* (GISTs #5 and #17). Accordingly, we checked *CDKN2A* and *CDKN2B* copy number status by genomic qPCR and fully confirmed all CGH results. Notably, suspected homozygous deletions were confirmed and refined, we observed that homozygous deletion in GIST #5 involved only *CDKN2A* but not *CDKN2B* (of which one copy was retained) (Table 5). Subsequently, all GISTs without homozygous deletion and from which DNA was available (58 cases) were submitted to *CDKN2A* sequencing. We did not detect any mutations (data not shown) and only three SNPs (RS3731249, RS11515 and one unreferenced silent SNP: c.*56G>A) were indentified in 4, 14 and 20 cases respectively. To precisely quantify the *p14* and *p16* expressions (both mRNA hybridized to the same (Agilent probe sets), we quantified expression using specific probes for *p14* and *p16* RT-qPCR (Table 5). In all the tumors without any copy of *p16* (7 cases) and in three tumors with only one copy, *p16* mRNA was nearly absent; and no protein was detected by IHC in the two cases with homozygous and the three cases with hemizygous deletion for which histological blocs were available (data not shown). The lack of *p16* mRNA and/or protein is therefore evidenced in 10 cases with nine belonging to the metastatic group (18 cases).

We thus hypothesized that another genomic alteration could lead to p16 pathway inactivation in cases without *p16* homozygous deletion. We observed one homozygous deletion and 13 hemizygous deletion of the *RB1* locus (Table 6).

**Table 6**: Results of the CINSARC analysis, *AURKA* expression (A = 9.13 as cut-off), CGH analysis (GI = 10 as cut-off) and *CDKN2A/2B* and *RB1* copy number determined by genomic qPCR and array-CGH, respectively (2 = without detectable deletion; 1 = hemizygous deletion; 0 = no copy). P = *PDGFRA*, K = *KIT*, WT= Wild type, nd = not done.

| | Exp array | | CGH | | | | Local recurrence | Metastasis | p16 Expression | | RB1 Expression | |
|---|---|---|---|---|---|---|---|---|---|---|---|---|
| | CINSARC Grading | AURKA | Genomic index | AGH | p16 tumor number | RB1 tumor number | | | qPCR | cDNA array | qPCR | cDNA array |
| GIST10 | C1 | A1 | GI1 | AG1 | 2 | 2 | No | No | 10.67 | 10.13 | 6.82 | 6.13 |
| GIST13 | C1 | A1 | GI1 | AG1 | 2 | 2 | No | No | 8.66 | 10.21 | 7.94 | 4.98 |
| GIST15 | C1 | A1 | GI1 | AG1 | 2 | 2 | No | No | 8.36 | 11.80 | 8.46 | 4.68 |
| GIST18 | C1 | A1 | GI1 | AG1 | 2 | 2 | No | No | 11.86 | 6.89 | 6.96 | 5.58 |
| GIST21 | C1 | A1 | GI1 | AG1 | 2 | 2 | No | No | 12.43 | 6.81 | 8.19 | 4.39 |
| GIST23 | C1 | A1 | GI1 | AG1 | 2 | 2 | No | No | 8.75 | nd | 7.00 | nd |
| GIST24 | C1 | A1 | GI1 | AG1 | 2 | 2 | No | No | 9.26 | 11.06 | 8.17 | 4.91 |
| GIST27 | C1 | A1 | GI1 | AG1 | 2 | 2 | No | No | 9.57 | 12.03 | 7.62 | 5.32 |
| GIST30 | C1 | A1 | GI1 | AG1 | 2 | 2 | No | No | 8.68 | 14.01 | 7.81 | 5.20 |
| GIST32 | C1 | A1 | GI1 | AG1 | 2 | 2 | No | No | 10.90 | 8.96 | 8.02 | 4.46 |
| GIST33 | C1 | A1 | GI1 | AG1 | 2 | 2 | No | No | 9.31 | 12.47 | 7.11 | 5.63 |
| GIST36 | C1 | A1 | GI1 | AG1 | 2 | 2 | No | No | 10.39 | 10.01 | 7.53 | 5.08 |
| GIST40 | C1 | A1 | GI1 | AG1 | 2 | 2 | No | No | 10.02 | 9.15 | 8.67 | 4.35 |
| GIST43 | C1 | A1 | GI1 | AG1 | 2 | 2 | No | No | 10.75 | 8.98 | 7.73 | 4.84 |
| GIST44 | C1 | A1 | GI1 | AG1 | 2 | 2 | No | No | 11.23 | 8.88 | 7.50 | 5.52 |
| GIST46 | C1 | A1 | GI1 | AG1 | 2 | 2 | No | No | 11.06 | 9.08 | 7.29 | 5.68 |
| GIST48 | C1 | A1 | GI1 | AG1 | 2 | 2 | No | No | 10.08 | 10.23 | 8.12 | 5.16 |
| GIST49 | C1 | A1 | GI1 | AG1 | 2 | 2 | No | No | 12.40 | 6.38 | 8.91 | 5.08 |
| GIST51 | C1 | A1 | GI1 | AG1 | 2 | 2 | No | No | 12.80 | 5.89 | 7.65 | 4.94 |
| GIST55 | C1 | A1 | GI1 | AG1 | 2 | 2 | No | No | 9.44 | 11.19 | 7.92 | 5.10 |
| GIST60 | C1 | A1 | GI1 | AG1 | 2 | 2 | No | No | 9.53 | 11.57 | 7.89 | 4.98 |
| GIST62 | C1 | A1 | GI1 | AG1 | 2 | 2 | No | No | 8.32 | 13.57 | 8.29 | 4.35 |
| GIST64 | C1 | A1 | GI1 | AG1 | 2 | 2 | No | No | 9.10 | 11.98 | 8.71 | 4.41 |
| GIST8 | C1 | A1 | GI1 | AG1 | 2 | 2 | No | No | 8.78 | 15.46 | 7.95 | 5.15 |

| | | | | | | | | | | | |
|---|---|---|---|---|---|---|---|---|---|---|---|
| GIST12 | C2 | A1 | GI1 | AG1 | 2 | 2 | No | No | 13.31 | 8.47 | 8.73 | 4.21 |
| GIST29 | C2 | A1 | GI1 | AG1 | 2 | 2 | No | No | 10.65 | 9.89 | 8.20 | 5.16 |
| GIST31 | C2 | A1 | GI1 | AG1 | 2 | 2 | No | No | 9.03 | 11.57 | 7.99 | 4.79 |
| GIST4 | C2 | A1 | GI1 | AG1 | 2 | 2 | No | No | 10.27 | 12.02 | 7.89 | 5.10 |
| GIST41 | C2 | A1 | GI1 | AG1 | 2 | 2 | No | No | 10.04 | 10.03 | 7.29 | 5.46 |
| GIST52 | C2 | A1 | nd | nd | 2 | nd | No | No | 9.72 | 13.49 | 7.20 | 5.30 |
| GIST45 | C2 | A1 | GI1 | AG1 | 2 | 2 | No | No | 7.44 | 15.35 | 6.93 | 5.77 |
| GIST50 | C1 | A1 | GI1 | AG1 | 1 | 2 | No | No | 10.70 | 9.27 | 8.32 | 5.95 |
| GIST66 | C1 | A1 | GI1 | AG1 | 1 | 2 | No | No | 9.85 | 9.52 | 7.70 | 5.39 |
| GIST59 | C1 | A1 | GI2 | AG2 | 2 | 2 | No | No | 13.72 | 3.71 | 7.78 | 4.30 |
| GIST67 | C1 | A1 | GI2 | AG2 | 2 | 2 | No | No | 11.94 | 7.98 | 7.30 | 5.36 |
| GIST20 | C2 | A1 | GI2 | AG2 | 2 | 2 | No | No | 12.89 | 6.43 | 8.25 | 4.32 |
| GIST22 | C2 | A2 | GI1 | AG2 | 2 | 2 | No | No | 11.59 | 7.42 | 8.05 | 4.91 |
| GIST11 | C2 | A2 | GI2 | AG2 | nd | 2 | No | No | 10.76 | 9.47 | 6.87 | 5.78 |
| GIST65 | C1 | A1 | GI2 | AG2 | 1 | 2 | No | No | 9.22 | 9.23 | 8.06 | 5.20 |
| GIST25 | nd | nd | GI1 | nd | 2 | 2 | No | No | nd | nd | nd | nd |
| GIST7 | nd | nd | GI1 | nd | 2 | 2 | No | No | nd | nd | nd | nd |
| GIST1 | C1 | A1 | GI1 | AG1 | 2 | 1 | No | No | 9.96 | 10.33 | 6.06 | 6.93 |
| GIST35 | C2 | A1 | GI1 | AG1 | 2 | 1 | No | No | 10.42 | 10.39 | 6.28 | 6.75 |
| GIST54 | C2 | A1 | GI1 | AG1 | 2 | 1 | No | No | 9.39 | 11.79 | 6.61 | 6.02 |
| GIST26 | nd | nd | GI2 | AG2 | 2 | 1 | No | No | nd | nd | nd | nd |
| GIST34 | nd | nd | GI2 | AG2 | 2 | 1 | No | No | nd | nd | nd | nd |
| GIST42 | C2 | A2 | GI1 | AG2 | 1 | 2 | No | No | 12.27 | 12.51 | 9.02 | 5.32 |
| GIST53 | C2 | A2 | GI1 | AG2 | 0 | 2 | No | No | 5.64 | 14.20 | 8.40 | 4.96 |
| GIST6 | C2 | A2 | GI2 | AG2 | 2 | 0 | Yes | No | 15.17 | 3.38 | 4.32 | 9.01 |
| GIST39 | C1 | A1 | GI2 | AG2 | 1 | 2 | No | Yes | 11.60 | 7.25 | 7.93 | 5.56 |
| GIST3 | nd | nd | GI2 | AG2 | 2 | 1 | No | Yes | nd | nd | nd | nd |
| GIST37 | C2 | A2 | GI2 | AG2 | 1 | 2 | Yes | Yes | 12.33 | 7.14 | 8.68 | 4.99 |
| GIST63 | C2 | A2 | GI1 | AG2 | 1 | 2 | No | Yes | 9.38 | nd | 7.22 | nd |
| GIST38 | C2 | A2 | GI2 | AG2 | 1 | 1 | No | Yes | 11.89 | 6.59 | 7.02 | 6.42 |
| GIST14 | C2 | A2 | GI2 | AG2 | 2 | 1 | Yes | Yes | 14.13 | 3.91 | 8.91 | 7.19 |
| GIST16 | C2 | A2 | GI2 | AG2 | 2 | 1 | No | Yes | 11.73 | 9.67 | 7.22 | 5.47 |
| GIST19 | C2 | A2 | GI2 | AG2 | 2 | 1 | Yes | Yes | 12.64 | 8.02 | 7.22 | 5.96 |
| GIST2 | C2 | A2 | GI2 | AG2 | 2 | 1 | No | Yes | 13.77 | 4.95 | 7.17 | 6.76 |
| GIST61 | C2 | A2 | GI2 | AG2 | 1 | 1* | No | Yes | 14.80 | 5.18 | 7.63 | 7.58 |
| GIST56 | C2 | A2 | GI2 | AG2 | 1 | 2 | Yes | Yes | 11.96 | 14.13 | 8.89 | 5.05 |
| GIST9 | C2 | A2 | GI2 | AG2 | 1 | 1 | Yes | Yes | 6.51 | 12.92 | 6.97 | 4.05 |
| GIST57 | nd | nd | GI2 | AG1 | 0 | 2 | No | Yes | nd | nd | nd | nd |
| GIST28 | C2 | A2 | GI2 | AG2 | 0 | 2 | No | Yes | 5.69 | 15.29 | 8.01 | 4.82 |
| GIST47 | C2 | A2 | GI2 | AG2 | 0 | 2 | No | Yes | 6.08 | 13.37 | 7.48 | 4.80 |
| GIST5 | C2 | A2 | GI1 | AG2 | 0 | 2 | No | Yes | 3.80 | 16.20 | 8.96 | 5.09 |
| GIST58 | C2 | A2 | GI2 | AG2 | 0 | 2 | No | Yes | 6.17 | 14.57 | 7.89 | 5.22 |
| GIST17 | nd | nd | GI2 | AG2 | 0 | 2 | Yes | Yes | nd | nd | nd | nd |

Eleven tumors harboring *RB1* deletions are classified AG2 and eight developed metastases. Interestingly tumors with a *p16* homozygous deletion are without any *RB1* deletion although they are highly rearranged. Sequencing *PB1* in all patients with available RNA and DNA (66 cases) we indentified one mutation (c.1959_1960del/ p.Lys653AsnfsX14) in the retained copy in GIST #61. qPCR analysis confirmed that deleted tumors had a significantly down regulated expression of *RB1* (Table 6).

Conclusion:

We demonstrated that CINSARC is a very powerful signature to predict metastatic outcome. CINSARC is composed of 67 genes which are all involved in chromosome integrity and mitosis control pathways, indicating that such mechanisms appear to be driving the development of metastasis in this tumor type, as we recently demonstrated in sarcomas (18). This is in line with results from the reciprocal approach, which is the identification of genes differentially expressed between GISTs with or without metastatic outcome. Actually, among the 227 up-regulated genes in the 18 metastasizing tumors, 45 were common with the CINSARC signature and the activated pathways were almost all the same. These results indicate that genome integrity and mitosis control are the effective restrain mechanisms underlying development of metastasis, and moreover, that these mechanisms appear to be sufficient, or at least the strongest. In line with this, we show that expression of the top ranked gene in both approaches, *AURKA,* is as efficient as CINSARC to predict metastatic outcome in both series of GISTs.

Following our results demonstrating the central role of the genome integrity control, we hypothesized that a defect of such mechanisms should lead to chromosome imbalances and that the resulting genome complexity should also predict outcome in GISTs. This is exactly what we show here since tumor stratification according to a CGH Genome Index (GI) which integrates the number of alterations and of altered chromosomes forms two groups of clearly distinct outcomes. This is clearly in agreement with the *AURKA* expression results, since whole chromosome losses are the most frequent alterations observed in GISTs and these are assumed to originate from the chromosome segregation deficiency induced by mitosis check-point defects, such as the AURKA overexpression (34).

In contrast to results of Yamagushi and colleagues, our study demonstrates that CINSARC, *AURKA* expression or CGH prognostic values are irrespective to tumor location. Furthermore, as mentioned above, the biological meaning of CINSARC and its association to genomic changes strongly indicate that CINSARC genes are involved in malignant progression and are not just a consequence of the process. This hypothesis is supported by the association we observe here between *CDKN2A* deletions (homozygous deletions in 6 cases and hemizygous deletions in 9 cases among 18 cases with metastatic outcome *versus* 5 hemizygous deletions in 48 non-metastatic patients), CINSARC prognosis groups and metastatic occurrence. Previous studies have already pointed out the potential association of *CDKN2A* alterations or expression of p16$^{INK4a}$ to tumor progression (11-16, 43). Nevertheless, data remain controversial mainly due to lack of clear delineation of the targeted gene at the 9p21 locus. It is still unclear which gene of *CDKN2A, CDKN2B* or *MTAP* is driving the association to poor prognosis. At the genomic level, even if *CDKN2A* and *2B* appear to be systematically codeleted (37, 44, 45), two studies indicate that 9p21 deletions are likely to target the *MTAP* gene and not exclusively *CDKN2A* and *CDKN2B* (35, 46). Here, CGH and genomic qPCR analyses demonstrated that homozygous deletions specifically target *CDKN2A* and that the common region of deletion excludes *CDKN2B* and *MTAP.* Surprisingly, we did not find any harmful *CDKN2A* mutations in any of GISTs case tested. Schnieder-Stock and colleagues (14) reported 9 so-called mutations in a series of 43 GISTs. But two of them are identical and have been detected in a tumor and its recurrence, one is now referenced as a SNP, two are silent mutations and in one case no interpretable sequence was obtained. Considering all this, the authors evidenced only four *CDKN2A* mutations (4/43= 9%). According to these data we expected around five mutations in our study and we have identified three changes, two SNP and one silent change not referred so far. One explication for this discrepancy could be sampling bias, but it is of interest to note that, we detected twice more homozygous *CDKN2A* deletions than reported in the study of Schneider-Stock *et al* (7/63 vs 2/43). Following the idea that another exclusive alteration could explain aggressive tumors (CINSARC C2, AG2) without *p16* inactivation we identified two tumors without *RB1* functional copy and 12 significantly down-regulated due to loss of one *RB1 copy* (p value). We did not detect any truncating mutation in these tumors but we hypothesize that micro-deletions, that we did not identify because of the lowest resolution of the arrays, could account for this second inactivation, as in sarcomas (47). An exclusive occurrence of *p16* and *RB1* alterations is highly supported by the observation that none of the tumors with *CDKN2A* homozygous deletion harbors any *RB1* deletion and among the 29 GISTs with one of these deletions, only three cases harbor both deletions (table 1). Altogether, p16/RB1 pathway is inactivated or down regulated in 14/18 (78%) and in 3/48 (6%) patients with and without metastatic outcome, respectively, which clearly means that inactivation of p16/RB1 pathway is associated to metastatic development.

*CDKN2A* codes for two key tumor suppressor proteins, the p16$^{INK4a}$ and the p14$^{ARF}$, which are involved in the regulation of the cell cycle G1 and G2/M transition. Together, these proteins regulate two important cell cycle checkpoints, the p53 and the RB1 pathways for p14 and p16$^{INK4a}$, respectively. Loss of these genes can lead to replicate senescence, cell immortalization and tumor growth (48-51). Most of the CINSARC genes are under the transcriptional control of E2F, which is tightly regulated by RB1 interaction. Actually, RB1 sequestrates E2F which is delivered upon RB1 phosphorylation by CDK4 (Cyclin Dependent Kinase 4) and p16$^{INK4a}$ inhibits CDK4. Therefore, our results allow us to hypothesize that inactivation of the p16/RB1 pathway in GISTs, mainly by deletion, is likely to be the causative alteration that leads to the over-expression of genes involved in mitosis control. This deregulation triggers cell genome rearrangements until a combination is naturally selected and fixed. Thus, the resulting genome complexity and its related expression confer the tumor cell aggressiveness and metastatic potential. Although this hypothesis has to be experimentally validated in cellular and mouse models, it is supported by the expression analysis of the GISTs with or without functional p16/RB1 pathway which shows that 42/225 (19%) genes up regulated in GISTs without functional p16/RB1 pathway are common with CINSARC signature. Moreover these 225 genes are involved in the same pathways than those enriched in CINSARC and metastases signatures (Supplementary table 3).

Imatinib mesylate has been proven to target KIT-aberrant signaling inhibiting the proliferation and survival in GIST cells. Until 2009, imatinib therapy was restricted to disseminated or advanced disease at the time of diagnosis. Since then, adjuvant treatment has been approved and the necessity to apply selection criteria to identify patients susceptible to benefit from such management has emerged. Patient selection foreseen by FDA (Food and Drug Administration) and to a lesser extent by EMA (European Medicines Agency) is essentially based on the histological risk evaluation. Both AFIP (9) and NIH (8) histological-based staging systems are widely accepted as "gold standards" in determining tumor metastatic risk and to determine whether a GIST patient is eligible or not for adjuvant therapy with imatinib. Here we show that the CINSARC signature and *AURKA* expression outperform the AFIP classification (survival analysis according to AFIP classification is presented in fig 4), particularly when associated to the CGH genomic index. Of particular interest, the Genomic Index is able to distinguish good and poor prognosis patients in GISTs classified as intermediate-risk by these histopathological systems (which represent around 25% of diagnoses). More specifically, among the 16 AFIP intermediate-risk cases, four developed metastasis. These cases were classified as poor prognosis by GI (fig. 4 and Table 5). GI established in this study is therefore a very powerful tool to manage GIST patient more likely to benefit from therapy since CGH is a technique already used in the daily practice by a growing number of pathology departments and is applicable to formalin-fixed paraffin-embedded (FFPE) samples. To validate the clinical application of GI, we collect a larger cohort of FFPE GIST samples to perform CGH with DNA from FFPE blocks.

We thus propose two possible decisional methods either to enhance the AFIP or NIH grading systems or to replace these histopathological methods. Firstly, when using the AFIP or NIH classifications, intermediate-risk cases are problematic for therapeutic management and our results demonstrate that the use of CGH profiling can easily and rapidly solve such a problem. Secondly, our results suggest that the combined use of GI and AURKA expression offer a better selection of patients for imatinib therapy than the AFIP classification does. Both methods offer equally efficient treatments for patients with metastatic risk, but CINSARC/AURKA-based selection, which is totally investigator-independent, would diminish consistently the number of patients, without metastatic risk, who are falsely declared eligible for imatinib therapy.

**Reference List**

1. Sircar,K. et al. Interstitial cells of Cajal as precursors of gastrointestinal stromal tumors. Am J Surg Pathol 23, 377-389 (1999).

2. Kindblom,L.G., Remotti,H.E., Aldenborg,F., & Meis-Kindblom,J.M. Gastrointestinal pacemaker cell tumor (GIPACT): gastrointestinal stromal tumors show phenotypic characteristics of the interstitial cells of Cajal. Am J Pathol 152, 1259-1269 (1998).

3. Hirota,S. et al. Gain-of-function mutations of c-kit in human gastrointestinal stromal tumors. Science 279, 577-580 (1998).

4. Heinrich,M.C. et al. PDGFRA activating mutations in gastrointestinal stromal tumors. Science 299, 708-710 (2003).

5. Corless,C.L., Fletcher,J.A., & Heinrich,M.C. Biology of gastrointestinal stromal tumors. J Clin Oncol 22, 3813-3825 (2004).

6. Wozniak,A. et al. Array CGH analysis in primary gastrointestinal stromal tumors: cytogenetic profile correlates with anatomic site and tumor aggressiveness, irrespective of mutational status. Genes Chromosomes Cancer 46, 261-276 (2007).

7. Rubin,B.P., Heinrich,M.C., & Corless,C.L. Gastrointestinal stromal tumour. Lancet 369, 1731-1741 (2007).

8. Fletcher,C.D. et al. Diagnosis of gastrointestinal stromal tumors: A consensus approach. Hum Pathol 33, 459-465 (2002).

9. Miettinen,M. & Lasota,J. Gastrointestinal stromal tumors: review on morphology, molecular pathology, prognosis, and differential diagnosis. Arch Pathol Lab Med 130, 1466-1478 (2006).

10. Gunawan,B. et al. Biological and clinical significance of cytogenetic abnormalities in low-risk and high-risk gastrointestinal stromal tumors. Hum Pathol 33, 316-321 (2002).

11. el-Rifai,W., Sarlomo-Rikala,M., Miettinen,M., Knuutila,S., & Andersson,L.C. DNA copy number losses in chromosome 14: an early change in gastrointestinal stromal tumors. Cancer Res 56, 3230-3233 (1996).

12. el-Rifai,W., Sarlomo-Rikala,M., Andersson,L.C., Knuutila,S., & Miettinen,M. DNA sequence copy number changes in gastrointestinal stromal tumors: tumor progression and prognostic significance. Cancer Res 60, 3899-3903 (2000).

13. Kim,N.G. et al. Putative chromosomal deletions on 9P, 9Q and 22Q occur preferentially in malignant gastrointestinal stromal tumors. Int J Cancer 85, 633-638 (2000).

14. Schneider-Stock,R. et al. High prognostic value of p16INK4 alterations in gastrointestinal stromal tumors. J Clin Oncol 21, 1688-1697 (2003).

15. Schneider-Stock,R. et al. Loss of p16 protein defines high-risk patients with gastrointestinal stromal tumors: a tissue microarray study. Clin Cancer Res 11, 638-645 (2005).

16. Romeo,S. et al. Cell cycle/apoptosis molecule expression correlates with imatinib response in patients with advanced gastrointestinal stromal tumors. Clin Cancer Res 15, 4191-4198 (2009).

17. Yamaguchi,U. et al. Distinct gene expression-defined classes of gastrointestinal stromal tumor. J Clin Oncol 26, 4100-4108 (2008).

18. Chibon,F. et al. Validated prediction of clinical outcome in sarcomas and multiple types of cancer on the basis of a gene expression signature related to genome complexity. Nat. Med 16, 781-787 (2010).

19. DeMatteo,R.P., Heinrich,M.C., El-Rifai,W.M., & Demetri,G. Clinical management of gastrointestinal stromal tumors: before and after STI-571. Hum Pathol 33, 466-477 (2002).

20. Kimura,M. et al. Cell cycle-dependent expression and spindle pole localization of a novel human protein kinase, Aik, related to Aurora of Drosophila and yeast Ipl1. J Biol Chem. 272, 13766-13771 (1997).

21. Bischoff,J.R. & Plowman,G.D. The Aurora/Ipl1p kinase family: regulators of chromosome segregation and cytokinesis. Trends Cell Biol 9, 454-459 (1999).

22. Marumoto,T., Zhang,D., & Saya,H. Aurora-A - a guardian of poles. Nat. Rev Cancer 5, 42-50 (2005).

23. Zhou,H. et al. Tumour amplified kinase STK15/BTAK induces centrosome amplification, aneuploidy and transformation. Nat. Genet. 20, 189-193 (1998).

24. Sen,S., Zhou,H., & White,R.A. A putative serine/threonine kinase encoding gene BTAK on chromosome 20q13 is amplified and overexpressed in human breast cancer cell lines. Oncogene 14, 2195-2200 (1997).

25. Bischoff,J.R. et al. A homologue of Drosophila aurora kinase is oncogenic and amplified in human colorectal cancers. EMBO J 17, 3052-3065 (1998).

26. Lam,A.K., Ong,K., & Ho,Y.H. Aurora kinase expression in colorectal adenocarcinoma: correlations with clinicopathological features, p16 expression, and telomerase activity. Hum Pathol 39, 599-604 (2008).

27. Shang, X. et al. Aurora A is a negative prognostic factor and a new therapeutic target in human neuroblastoma. Mol Cancer Ther 8, 2461-2469 (2009).

28. Reiter,R. et al. Aurora kinase A messenger RNA overexpression is correlated with tumor progression and shortened survival in head and neck squamous cell carcinoma. Clin Cancer Res 12, 5136-5141 (2006).

29. Benten, D. et al. Aurora kinase inhibitor PHA-739358 suppresses growth of hepatocellular carcinoma in vitro and in a xenograft mouse model. Neoplasia. 11, 934-944 (2009).

30. Gorgun, G. et al. A novel Aurora-A kinase inhibitor MLN8237 induces cytotoxicity and cell-cycle arrest in multiple myeloma. Blood 115, 5202-5213 (2010).

31. Kovar,H. AURKA inhibitors: right in time. Pediatr. Blood Cancer 55, 3-4 (2010).

32. Maris,J.M. et al. Initial testing of the aurora kinase A inhibitor MLN8237 by the Pediatric Preclinical Testing Program (PPTP). Pediatr. Blood Cancer 55, 26-34 (2010).

33. Shimomura,T. et al. MK-5108, a highly selective Aurora-A kinase inhibitor, shows antitumor activity alone and in combination with docetaxel. Mol Cancer Ther 9, 157-166 (2010).

34. Schvartzman,J.M., Sotillo,R., & Benezra,R. Mitotic chromosomal instability and cancer: mouse modelling of the human disease. Nat. Rev Cancer 10, 102-115(2010).

35. Astolfi,A. et al. A molecular portrait of gastrointestinal stromal tumors: an integrative analysis of gene expression profiling and high-resolution genomic copy number. Lab Invest (2010).

36. Debiec-Rychter,M., Lasota,J., Sarlomo-Rikala,M., Kordek,R., & Miettinen,M. Chromosomal aberrations in malignant gastrointestinal stromal tumors: correlation with c-KIT gene mutation. Cancer Genet. Cytogenet. 128, 24-30 (2001).

37. Belinsky,M.G. et al. High density DNA array analysis reveals distinct genomic profiles in a subset of gastrointestinal stromal tumors. Genes Chromosomes Cancer 48, 886-896 (2009).

38. Allander,S.V. et al. Gastrointestinal stromal tumors with KIT mutations exhibit a remarkably homogeneous gene expression profile. Cancer Res 61, 8624-8628 (2001).

39. Price,N.D. et al. Highly accurate two-gene classifier for differentiating gastrointestinal stromal tumors and leiomyosarcomas. Proc Natl. Acad. Sci. U. S. A 104, 3414-3419 (2007).

40. Subramanian,S. et al. Gastrointestinal stromal tumors (GISTs) with KIT and PDGFRA mutations have distinct gene expression profiles. Oncogene 23, 7780-7790 (2004).

41. Antonescu, C.R. et al. Gene expression in gastrointestinal stromal tumors is distinguished by KIT genotype and anatomic site. Clin Cancer Res 10, 3282-3290 (2004).

42. Kang, H.J. et al. Correlation of KIT and platelet-derived growth factor receptor alpha mutations with gene activation and expression profiles in gastrointestinal stromal tumors. Oncogene 24, 1066-1074 (2005).

43. Haller,F. et al., Loss of 9p leads to p16INK4A down-regulation and enables RB/E2F1-dependent cell cycle promotion in gastrointestinal stromal tumours (GISTs). J Pathol 215, 253-262 (2008).

44. Perrone,F. et al. 9p21 locus analysis in high-risk gastrointestinal stromal tumors characterized for c-kit and platelet-derived growth factor receptor alpha gene alterations. Cancer 104, 159-169 (2005).

45. Assamaki, R. et al. Array comparative genomic hybridization analysis of chromosomal imbalances and their target genes in gastrointestinal stromal tumors. Genes Chromosomes Cancer 46, 564-576 (2007).

46. Huang,H.Y. et al. Homozygous deletion of MTAP gene as a poor prognosticator in gastrointestinal stromal tumors. Clin Cancer Res 15; 6963-6972 (2009).

47. Chibon. F. et al. The RB1 gene is the target of chromosome 13 deletions in malignant fibrous histiocytoma. Cancer Res. Nov 15, 6339-45 (2000).

48. Serrano,M., Hannon,G.J., & Beach,D. A new regulatory motif in cell-cycle control causing specific inhibition of cyclin D/CDK4. Nature 366, 704-707 (1993).

49. Hannon,G.J. & Beach,D. p15INK4B is a potential effector of TGF-beta-induced cell cycle arrest. Nature 371, 257-261 (1994).

50. Kamb,A. et al. Analysis of the p16 gene (CDKN2) as a candidate for the chromosome 9p melanoma susceptibility locus. Nat. Genet. 8, 23-26 (1994).

**EP 2 630 259 B1**

51. Enders,G.H. The INK4a/ARF locus and human cancer. Methods Mol Biol 222, 197-209 (2003).

52. Pérot,G. et al. Constant p53 Pathway Inactivation in a Large Series of Soft Tissue Sarcomas with Complex Genetics. Am J Pathol. 177, 2080-2090 (2010).

53. Houdayer,C., et al. Comprehensive screening for constitutional RB1 mutations by DHPLC and QMPSF. Hum Mutat. 23, 193-202 (2004).

54. Carpinelli P et al. PHA-739358, a potent inhibitor of Aurora kinases with a selective target inhibition profile relevant to cancer. Mol Cancer Ther. 2007 Dec;6(12 Pt 1):3158-68.

55. PCT/FR2010/000323

**Claims**

1. Method for *in vitro* predicting survival and/or metastatic outcome of gastrointestinal stromal tumors (GISTs), **characterized in that** it comprises the measure of the level, in a patient-derived biological sample of GIST, of a pool of polypeptides or polynucleotides consisting in Aurora kinase A (AURKA), wherein GIST is classified in a group with high risk to develop metastases within 5 years, i.e. with a risk to develop metastases within 5 years of more than 80%, when AURKA is up-regulated compared to a group with no risk to develop metastases within 5 years, wherein AURKA is down-regulated and less than 9.13, wherein 9.13 is calculated from the AURKA mean expression of stratified tumor samples.

2. Method according to claim 1, wherein said measure of the level of the pool of polypeptides is a measure of the expression level of a pool of polynucleotides consisting in AURKA.

3. Method according to any one of claim 1 to 2, comprising the calculation of the Genomic Index (GI), i.e. the number and type of alterations of the GIST genome, according to the formula as follows:

$$GI = A^2 \times C,$$

wherein A is the number of alterations in GIST genome and C is the number of involved chromosomes in GIST.

4. Method according to claim 3, wherein GIST is classified in a group of metastasis- and disease-free survival group when AURKA is down-regulated and the GI is equal or less than 10.

5. Method according to claim 3, wherein GIST is classified in a group with low risk to develop metastases within 5 years, i.e. with a risk to develop metastases within 5 years equal to 0%, when AURKA expression is equal or less than the mean of AURKA expression and GI is equal or less than 10, said mean being the mean of AURKA expression in several GISTs.

6. Method according to claim 3, wherein GIST is classified in a group with high risk to develop metastases within 5 years, i.e. with a risk to develop metastases within 5 years more than 75%, when AURKA expression is more than the mean of AURKA expression and GI is more than 10, said mean being the mean of AURKA expression in several GISTs.

**Patentansprüche**

1. Verfahren zur In-vitro-Vorhersage von Überleben und/oder metastatischem Ergebnis bei gastrointestinalen stromalen Tumoren (GISTs), **dadurch gekennzeichnet, dass** es die Messung des Niveaus eines Pools von Polypeptiden oder Polynukleotiden, die aus Aurorakinase A (AURKA) bestehen, in einer Patienten-abgeleiteten Probe von GIST umfasst, wobei GIST in eine Gruppe mit einem hohen Risiko der Entwicklung von Metastasen innerhalb von 5 Jahren eingestuft wird, d. h. mit einem Risiko von mehr als 80 % der Entwicklung von Metastasen innerhalb von 5 Jahren, wenn AURKA hochreguliert ist im Vergleich zu einer Gruppe ohne Risiko der Entwicklung von Metastasen innerhalb von 5 Jahren, in der AURKA herabreguliert und kleiner als 9,13 ist, wobei 9,13 aus der mittleren AURKA-Expression von stratifizierten Tumorproben berechnet ist.

2. Verfahren gemäß Anspruch 1, wobei die Messung des Niveaus des Pools von Polypeptiden eine Messung des Expressionsniveaus eines Pools von Polynukleotiden, die aus AURKA bestehen, ist.

3. Verfahren gemäß einem der Ansprüche 1 bis 2, umfassend das Berechnen des genomischen Index (GI), d. h. von Anzahl und Typ von Veränderung des GIST-Genoms, gemäß folgender Formel:

$$GI = A^2 \times C,$$

wobei A die Anzahl von Veränderungen in dem GIST-Genom ist und C die Anzahl von bei GIST beteiligten Chromosomen ist.

4. Verfahren gemäß Anspruch 3, wobei GIST in eine Gruppe einer metastasen- und erkrankungsfreien Überlebensgruppe eingestuft wird, wenn AURKA herabreguliert ist und der GI gleich oder kleiner als 10 ist.

5. Verfahren gemäß Anspruch 3, wobei GIST in eine Gruppe mit niedrigem Risiko der Entwicklung von Metastasen innerhalb von 5 Jahren eingestuft wird, d. h. mit einem Risiko gleich 0 % der Entwicklung von Metastasen innerhalb von 5 Jahren, wenn die AURKA-Expression gleich oder kleiner als der Mittelwert der AURKA-Expression ist und der GI gleich oder kleiner als 10 ist, wobei der Mittelwert der Mittelwert der AURKA-Expression in mehreren GISTs ist.

**6.** Verfahren gemäß Anspruch 3, wobei GIST in eine Gruppe mit hohem Risiko der Entwicklung von Metastasen innerhalb von 5 Jahren eingestuft wird, d. h. mit einem Risiko von mehr als 75 % der Entwicklung von Metastasen innerhalb von 5 Jahren, wenn die AURKA-Expression größer als als der Mittelwert der AURKA-Expression ist und der GI größer als 10 ist, wobei der Mittelwert der Mittelwert der AURKA-Expression in mehreren GISTs ist.

## Revendications

**1.** Méthode pour la prédiction *in vitro* de la survie et/ou de l'évolution métastasique de tumeurs stromales gastrointestinales (GISTs), **caractérisée en ce qu'**elle comprend la mesure, dans un échantillon biologique de GIST dérivé d'un patient, du niveau d'un pool de polypeptides ou de polynucleotides consistant en la kinase Aurora A (AURKA), dans laquelle un GIST est classé dans un groupe avec un risqué élevé de plus de 80 % de développer des métastases sous 5 ans, quand AURKA est sur-régulé, en comparaison avec un groupe au risque nul de développer des métastases sous 5 ans, dans lequel AURKA est sous-régulé et inférieur à 9,13, la valeur de 9,13 étant calculée à partir de l'expression moyenne de AURKA d'échantillons stratifiés de tumeurs.

**2.** Méthode selon la revendication 1, dans laquelle ladite mesure du niveau d'un pool de polypeptides est une mesure du niveau d'expression d'un pool de polynucleotides consistant en AURKA.

**3.** Méthode selon l'une quelconque des revendications 1 ou 2, comprenant le calcul de l'Index Génomique (IG), i.e. le nombre et le type d'altérations du génome du GIST, selon la formule suivante:

$$IG = A^2 \text{ x } C,$$

dans laquelle A est le nombre d'altérations dans le genome du GIST et C est le nombre de chromosomes impliqués dans le GIST.

**4.** Méthode selon la revendication 3, dans laquelle le GIST est classé dans un groupe de survie sans métastase ni maladie lorsque AURKA est sous-régulé et que l'IG est inférieur ou égal à 10.

**5.** Méthode selon la revendication 3, dans laquelle le GIST est classé dans un groupe à faible risque de developper des metastases sous 5 ans, i.e. avec un risqué de developper des metastases sous 5 ans égal à 0%, lorsque l'expression d'AURKA est inférieure ou égale à la moyenne de l'expression d'AURKA et que l'IG est inférieur ou égal à 10, ladite moyenne étant la moyenne de l'expression d'AURKA dans plusieurs GISTs.

**6.** Méthode selon la revendication 3, dans laquelle le GIST est classé dans un groupe à haut risque de développer des metastases sous 5 ans, i.e. avec un risque de développer des métastases sous 5 ans de plus de 75%, lorsque l'expression d'AURKA est supérieure à la moyenne de l'expression d'AURKA et que l'IG est supérieur à 10, ladite moyenne étant la moyenne de l'expression d'AURKA dans plusieurs GISTs.

a)

| | | 0 | 1 y | 2 y | 3 y | 4 y | 5 y |
|---|---|---|---|---|---|---|---|
| C 1 | Patients at risk | 17 | 17 | 14 | 11 | 10 | 7 |
| | Cumulated events | 0 | 0 | 0 | 0 | 0 | 0 |
| | MFS | 1 | 1 | 1 | 1 | 1 | 1 |
| C 2 | Patients at risk | 15 | 13 | 10 | 7 | 7 | 4 |
| | Cumulated events | 0 | 2 | 5 | 6 | 6 | 8 |
| | MFS | 1 | 0,87 | 0,66 | 0.59 | 0,59 | 0,42 |

Figure 1 (part 1)

b)

Figure 1 (part 2)

| | | 0 | 1 y | 2 y | 3 y | 4 y | 5 y |
|---|---|---|---|---|---|---|---|
| C1 | Patients at risk | 31 | 25 | 24 | 17 | 11 | 7 |
| | Cumulated events MFS | 0 1 | 0 1 | 0 1 | 0 1 | 0 1 | 0 1 |
| C2 | Patients at risk | 29 | 22 | 19 | 13 | 8 | 4 |
| | Cumulated events MFS | 1 0.97 | 7 0,76 | 9 0,69 | 11 0,62 | 13 0,50 | 14 0,40 |

|  | 0 | 1y | 2y | 3y | 4y | 5y |
|---|---|---|---|---|---|---|
| Patients at risk | 31 | 25 | 24 | 17 | 11 | 7 |
| Cumulated events | 0 | 0 | 0 | 0 | 0 | 0 |
| DFS | 1 | 1 | 1 | 1 | 1 | 1 |
| Patients at risk | 29 | 22 | 18 | 12 | 7 | 4 |
| Cumulated events | 1 | 7 | 10 | 13 | 15 | 15 |
| DFS | 0,97 | 0,76 | 0,66 | 0,54 | 0,43 | 0,43 |

Figure 1 (part 3)

a)

|  |  | 0 | 1 y | 2 y | 3 y | 4 y | 5 y |
|---|---|---|---|---|---|---|---|
| A1 | Patients at risk | 41 | 35 | 34 | 25 | 16 | 10 |
|  | Cumulated events | 0 | 0 | 0 | 0 | 0 | 0 |
|  | MFS | 1 | 1 | 1 | 1 | 1 | 1 |
| A2 | Patients at risk | 19 | 12 | 9 | 5 | 3 | 1 |
|  | Cumulated events | 1 | 7 | 9 | 11 | 13 | 14 |
|  | MFS | 0,95 | 0,63 | 0,53 | 0,41 | 0,24 | 0,12 |

Figure 2 (part 1)

p log rank = 3.61e-12

A : A1 : 41 cases
B : A2 : 19 cases

| | 0 | 1y | 2y | 3y | 4y | 5y |
|---|---|---|---|---|---|---|
| A: Patients at risk | 41 | 35 | 34 | 25 | 16 | 10 |
| Cumulated events | 0 | 0 | 0 | 0 | 0 | 0 |
| DFS | 1 | 1 | 1 | 1 | 1 | 1 |
| B: Patients at risk | 19 | 12 | 8 | 4 | 2 | 1 |
| Cumulated events | 1 | 7 | 10 | 13 | 15 | 15 |
| DFS | 0,95 | 0,63 | 0,47 | 0,28 | 0,14 | 0,14 |

Figure 2 (part 2)

b)

Metastasis-Free Survival

A

B

p log rank = 0.00095

A : A1 : 17 cases
B : A2 : 15 cases

| | 0 | 1 y | 2 y | 3 y | 4 y | 5 y |
|---|---|---|---|---|---|---|
| A1: Patients at risk | 17 | 17 | 14 | 11 | 10 | 7 |
| Cumulated | 0 | 0 | 0 | 0 | 0 | 0 |
| events | 1 | 1 | 1 | 1 | 1 | 1 |
| MFS | | | | | | |
| A2: Patients at risk | 15 | 13 | 10 | 7 | 7 | 4 |
| Cumulated | 0 | 2 | 5 | 6 | 6 | 8 |
| events | 1 | 0,87 | 0,66 | 0.59 | 0,59 | 0,42 |
| MFS | | | | | | |

Figure 2 (part 3)

Figure 3

|  | CINSARC67 | | AURKA | | CGH | |
|---|---|---|---|---|---|---|
|  | Mean | Class | Mean | Class | Alt²/chr | Class |
| GIST8 | 8.9 | C1 | 8.5 | A1 | 1 | GI1 |
| GIST49 | 9.3 | C1 | 9.6 | A1 | 9.8 | GI1 |
| GIST11 | 9.8 | C2 | 10 | A2 | 10.1 | GI2 |
| GIST38 | 11.2 | C2 | 11.8 | A2 | 56.5 | GI2 |

a)

| | | 0 | 1 y | 2 y | 3 y | 4 y | 5 y |
|---|---|---|---|---|---|---|---|
| GI 1 | A: Patients at risk | 42 | 36 | 34 | 25 | 16 | 9 |
| | Cumulated events | 0 | 1 | 1 | 1 | 2 | 2 |
| | MFS | 1 | 0,97 | 0,97 | 0,97 | 0,93 | 0,93 |
| GI 2 | B: Patients at risk | 24 | 14 | 10 | 5 | 3 | 1 |
| | Cumulated events | 1 | 9 | 11 | 13 | 14 | 15 |
| | MFS | 0,96 | 0,63 | 0,52 | 0,41 | 0,33 | 0,16 |

Figure 4 (part 1)

| | 0 | 1y | 2y | 3y | 4y | 5y |
|---|---|---|---|---|---|---|
| Patients at risk | 42 | 36 | 34 | 25 | 16 | 9 |
| | 0 | 1 | 1 | 1 | 2 | 2 |
| Cumulated events DFS | 1 | 0,97 | 0,97 | 0,97 | 0,93 | 0,93 |
| Patients at risk | 24 | 14 | 9 | 4 | 2 | 1 |
| | 1 | 9 | 12 | 15 | 16 | 16 |
| Cumulated events DFS | 0,96 | 0,63 | 0,47 | 0,29 | 0,21 | 0,21 |

Figure 4 (part 2)

b)

| | | 0 | 1 y | 2 y | 3 y | 4 y | 5 y |
|---|---|---|---|---|---|---|---|
| M 1 | Patients at risk | 31 | 25 | 23 | 17 | 11 | 6 |
| | Cumulated events | 0 | 0 | 0 | 0 | 0 | 0 |
| | MFS | 1 | 1 | 1 | 1 | 1 | 1 |
| M 2 | Patients at risk | 16 | 15 | 13 | 8 | 7 | 3 |
| | Cumulated events | 0 | 1 | 1 | 2 | 2 | 3 |
| | MFS | 1 | 0.94 | 0.94 | 0.87 | 0.87 | 0.69 |
| M 3 | Patients at risk | 19 | 11 | 9 | 6 | 2 | 2 |
| | Cumulated events | 1 | 8 | 10 | 11 | 13 | 13 |
| | MFS | 0,95 | 0,58 | 0,47 | 0,42 | 0,25 | 0,25 |

Figure 4 (part 3)

| | 0 | 1y | 2y | 3y | 4y | 5y |
|---|---|---|---|---|---|---|
| A: Patients at risk | 31 | 25 | 23 | 17 | 11 | 6 |
| | 0 | 0 | 0 | 0 | 0 | 0 |
| Cumulated events DFS | 1 | 1 | 1 | 1 | 1 | 1 |
| B: Patients at risk | 16 | 15 | 13 | 7 | 6 | 3 |
| | 0 | 1 | 1 | 3 | 3 | 3 |
| Cumulated events DFS | 1 | 0.94 | 0.94 | 0.76 | 0.76 | 0.76 |
| C: Patients at risk | 19 | 11 | 8 | 6 | 2 | 2 |
| | 1 | 8 | 11 | 12 | 14 | 14 |
| Cumulated events DFS | 0,95 | 0,58 | 0,42 | 0,37 | 0,22 | 0,22 |

Figure 4 (part 4)

c)

A : GI1 : 10 cases
B : GI2 : 6 cases

p log rank = 0.0207

| | | 0 | 1 y | 2 y | 3 y | 4 y | 5 y |
|---|---|---|---|---|---|---|---|
| GI1 | Patients at risk | 10 | 10 | 9 | 6 | 5 | 2 |
| | Cumulated events | 0 | 0 | 0 | 0 | 0 | 0 |
| | MFS | 1 | 1 | 1 | 1 | 1 | 1 |
| GI2 | Patients at risk | 6 | 5 | 4 | 2 | 2 | 1 |
| | Cumulated events | 0 | 1 | 1 | 2 | 2 | 3 |
| | MFS | 1 | 0,83 | 0,83 | 0,63 | 0,63 | 0,31 |

Figure 4 (part 5)

| | 0 | 1y | 2y | 3y | 4y | 5y |
|---|---|---|---|---|---|---|
| A: Patients at risk | 10 | 10 | 9 | 6 | 5 | 2 |
| | 0 | 0 | 0 | 0 | 0 | 0 |
| Cumulated events DFS | 1 | 1 | 1 | 1 | 1 | 1 |
| B: Patients at risk | 6 | 5 | 4 | 1 | 1 | 1 |
| | 0 | 1 | 1 | 3 | 3 | 3 |
| Cumulated events DFS | 1 | 0,83 | 0,83 | 0,31 | 0,31 | 0,31 |

Figure 4 (part 6)

| | | 0 | 1 y | 2 y | 3 y | 4 y | 5 y |
|---|---|---|---|---|---|---|---|
| A G1 | Patients at risk | 35 | 30 | 29 | 22 | 14 | 8 |
| | Cumulated events | 0 | 0 | 0 | 0 | 0 | 0 |
| | MFS | 1 | 1 | 1 | 1 | 1 | 1 |
| A G2 | Patients at risk | 29 | 18 | 13 | 7 | 4 | 2 |
| | Cumulated events | 1 | 10 | 12 | 14 | 16 | 17 |
| | MFS | 0,97 | 0,66 | 0,57 | 0,48 | 0,34 | 0,23 |

Figure 5 (part 1)

| | 0 | 1y | 2y | 3y | 4y | 5y |
|---|---|---|---|---|---|---|
| A : Patients at risk | 35 | 30 | 29 | 22 | 14 | 8 |
| | 0 | 0 | 0 | 0 | 0 | 0 |
| Cumulated events DFS | 1 | 1 | 1 | 1 | 1 | 1 |
| B: Patients at risk | 29 | 18 | 12 | 6 | 3 | 2 |
| | 1 | 10 | 13 | 16 | 18 | 18 |
| Cumulated events DFS | 0,97 | 0,66 | 0,53 | 0,38 | 0,25 | 0,25 |

Figure 5 (part 2)

Figure 6

Figure 7

Figure 8 (part 1)

A :

Figure 8 (part 2)

Figure 8 (part 3)

**Figure 9**

## 35 GIST AURKA

IHC = 0

IHC ≥ 0

B

A

p log rank = 2.7e-05
meta AURKA1 = 3
meta AURKA2 = 4

A
B

AURKA1 : 29 cases
AURKA2 : 6 cases

Metastasis - Free survival

Figure 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010068951 A
- WO 2006129064 A
- FR 2010000323 W
- EP 10013806 A

### Non-patent literature cited in the description

- **YAMASHITA et al.** Chromosomal numerical abnormality profiles of gastrointestinal stromal tumors. *Japanese Journal of clinical oncology,* 2006, vol. 36 (2), 85-92
- **SIRCAR,K. et al.** Interstitial cells of Cajal as precursors of gastrointestinal stromal tumors. *Am J Surg Pathol,* 1999, vol. 23, 377-389
- **KINDBLOM,L.G. ; REMOTTI,H.E. ; ALDENBORG,F. ; MEIS-KINDBLOM,J.M.** Gastrointestinal pacemaker cell tumor (GIPACT): gastrointestinal stromal tumors show phenotypic characteristics of the interstitial cells of Cajal. *Am J Pathol,* 1998, vol. 152, 1259-1269
- **HIROTA,S. et al.** Gain-of-function mutations of c-kit in human gastrointestinal stromal tumors. *Science,* 1998, vol. 279, 577-580
- **HEINRICH,M.C. et al.** PDGFRA activating mutations in gastrointestinal stromal tumors. *Science,* 2003, vol. 299, 708-710
- **CORLESS,C.L. ; FLETCHER,J.A. ; HEINRICH,M.C.** Biology of gastrointestinal stromal tumors. *J Clin Oncol,* 2004, vol. 22, 3813-3825
- **WOZNIAK,A. et al.** Array CGH analysis in primary gastrointestinal stromal tumors: cytogenetic profile correlates with anatomic site and tumor aggressiveness, irrespective of mutational status. *Genes Chromosomes Cancer,* 2007, vol. 46, 261-276
- **RUBIN,B.P. ; HEINRICH,M.C. ; CORLESS,C.L.** Gastrointestinal stromal tumour. *Lancet,* 2007, vol. 369, 1731-1741
- **FLETCHER,C.D. et al.** Diagnosis of gastrointestinal stromal tumors: A consensus approach. *Hum Pathol,* 2002, vol. 33, 459-465
- **MIETTINEN,M. ; LASOTA,J.** Gastrointestinal stromal tumors: review on morphology, molecular pathology, prognosis, and differential diagnosis. *Arch Pathol Lab Med,* 2006, vol. 130, 1466-1478
- **GUNAWAN,B. et al.** Biological and clinical significance of cytogenetic abnormalities in low-risk and high-risk gastrointestinal stromal tumors. *Hum Pathol,* 2002, vol. 33, 316-321
- **EL-RIFAI,W. ; SARLOMO-RIKALA,M. ; MIETTINEN,M. ; KNUUTILA,S. ; ANDERSSON,L.C.** DNA copy number losses in chromosome 14: an early change in gastrointestinal stromal tumors. *Cancer Res,* 1996, vol. 56, 3230-3233
- **EL-RIFAI,W. ; SARLOMO-RIKALA,M. ; ANDERSSON,L.C. ; KNUUTILA,S. ; MIETTINEN,M.** DNA sequence copy number changes in gastrointestinal stromal tumors: tumor progression and prognostic significance. *Cancer Res,* 2000, vol. 60, 3899-3903
- **KIM,N.G. et al.** Putative chromosomal deletions on 9P, 9Q and 22Q occur preferentially in malignant gastrointestinal stromal tumors. *Int J Cancer,* 2000, vol. 85, 633-638
- **SCHNEIDER-STOCK,R. et al.** High prognostic value of p16INK4 alterations in gastrointestinal stromal tumors. *J Clin Oncol,* 2003, vol. 21, 1688-1697
- **SCHNEIDER-STOCK,R. et al.** Loss of p16 protein defines high-risk patients with gastrointestinal stromal tumors: a tissue microarray study. *Clin Cancer Res,* 2005, vol. 11, 638-645
- **ROMEO,S. et al.** Cell cycle/apoptosis molecule expression correlates with imatinib response in patients with advanced gastrointestinal stromal tumors. *Clin Cancer Res,* 2009, vol. 15, 4191-4198
- **YAMAGUCHI,U. et al.** Distinct gene expression-defined classes of gastrointestinal stromal tumor. *J Clin Oncol,* 2008, vol. 26, 4100-4108
- **CHIBON,F. et al.** Validated prediction of clinical outcome in sarcomas and multiple types of cancer on the basis of a gene expression signature related to genome complexity. *Nat. Med,* 2010, vol. 16, 781-787
- **DEMATTEO,R.P. ; HEINRICH,M.C. ; EL-RIFAI,W.M. ; DEMETRI,G.** Clinical management of gastrointestinal stromal tumors: before and after STI-571. *Hum Pathol,* 2002, vol. 33, 466-477
- **KIMURA,M. et al.** Cell cycle-dependent expression and spindle pole localization of a novel human protein kinase, Aik, related to Aurora of Drosophila and yeast Ipl1. *J Biol Chem.,* 1997, vol. 272, 13766-13771

- **BISCHOFF,J.R. ; PLOWMAN,G.D.** The Aurora/Ipl1p kinase family: regulators of chromosome segregation and cytokinesis. *Trends Cell Biol,* 1999, vol. 9, 454-459
- **MARUMOTO,T. ; ZHANG,D. ; SAYA,H.** Aurora-A - a guardian of poles. *Nat. Rev Cancer,* 2005, vol. 5, 42-50
- **ZHOU,H. et al.** Tumour amplified kinase STK15/BTAK induces centrosome amplification, aneuploidy and transformation. *Nat. Genet.,* 1998, vol. 20, 189-193
- **SEN,S. ; ZHOU,H. ; WHITE,R.A.** A putative serine/threonine kinase encoding gene BTAK on chromosome 20q13 is amplified and overexpressed in human breast cancer cell lines. *Oncogene,* 1997, vol. 14, 2195-2200
- **BISCHOFF,J.R. et al.** A homologue of Drosophila aurora kinase is oncogenic and amplified in human colorectal cancers. *EMBO J,* 1998, vol. 17, 3052-3065
- **LAM,A.K. ; ONG,K. ; HO,Y.H.** Aurora kinase expression in colorectal adenocarcinoma: correlations with clinicopathological features, p16 expression, and telomerase activity. *Hum Pathol,* 2008, vol. 39, 599-604
- **SHANG, X. et al.** Aurora A is a negative prognostic factor and a new therapeutic target in human neuroblastoma. *Mol Cancer Ther,* 2009, vol. 8, 2461-2469
- **REITER,R. et al.** Aurora kinase A messenger RNA overexpression is correlated with tumor progression and shortened survival in head and neck squamous cell carcinoma. *Clin Cancer Res,* 2006, vol. 12, 5136-5141
- **BENTEN, D. et al.** Aurora kinase inhibitor PHA-739358 suppresses growth of hepatocellular carcinoma in vitro and in a xenograft mouse model. *Neoplasia,* 2009, vol. 11, 934-944
- **GORGUN, G. et al.** A novel Aurora-A kinase inhibitor MLN8237 induces cytotoxicity and cell-cycle arrest in multiple myeloma. *Blood,* 2010, vol. 115, 5202-5213
- **KOVAR,H.** AURKA inhibitors: right in time. *Pediatr. Blood Cancer,* 2010, vol. 55, 3-4
- **MARIS,J.M et al.** Initial testing of the aurora kinase A inhibitor MLN8237 by the Pediatric Preclinical Testing Program (PPTP). Pediatr. *Blood Cancer,* 2010, vol. 55, 26-34
- **SHIMOMURA,T. et al.** MK-5108, a highly selective Aurora-A kinase inhibitor, shows antitumor activity alone and in combination with docetaxel. *Mol Cancer Ther,* 2010, vol. 9, 157-166
- **SCHVARTZMAN,J.M. ; SOTILLO,R. ; BENEZRA,R.** Mitotic chromosomal instability and cancer: mouse modelling of the human disease. *Nat. Rev Cancer,* 2010, vol. 10, 102-115
- **ASTOLFI,A. et al.** A molecular portrait of gastrointestinal stromal tumors: an integrative analysis of gene expression profiling and high-resolution genomic copy number. *Lab Invest,* 2010
- **DEBIEC-RYCHTER,M. ; LASOTA,J. ; SARLOMO-RIKALA,M. ; KORDEK,R ; MIETTINEN,M.** Chromosomal aberrations in malignant gastrointestinal stromal tumors: correlation with c-KIT gene mutation. *Cancer Genet. Cytogenet.,* 2001, vol. 128, 24-30
- **BELINSKY,M.G. et al.** High density DNA array analysis reveals distinct genomic profiles in a subset of gastrointestinal stromal tumors. *Genes Chromosomes Cancer,* 2009, vol. 48, 886-896
- **ALLANDER,S.V. et al.** Gastrointestinal stromal tumors with KIT mutations exhibit a remarkably homogeneous gene expression profile. *Cancer Res,* 2001, vol. 61, 8624-8628
- **PRICE,N.D. et al.** Highly accurate two-gene classifier for differentiating gastrointestinal stromal tumors and leiomyosarcomas. *Proc Natl. Acad. Sci. U. S. A,* 2007, vol. 104, 3414-3419
- **SUBRAMANIAN,S. et al.** Gastrointestinal stromal tumors (GISTs) with KIT and PDGFRA mutations have distinct gene expression profiles. *Oncogene,* 2004, vol. 23, 7780-7790
- **ANTONESCU, C.R. et al.** Gene expression in gastrointestinal stromal tumors is distinguished by KIT genotype and anatomic site. *Clin Cancer Res,* 2004, vol. 10, 3282-3290
- **KANG, H.J. et al.** Correlation of KIT and platelet-derived growth factor receptor alpha mutations with gene activation and expression profiles in gastrointestinal stromal tumors. *Oncogene,* 2005, vol. 24, 1066-1074
- **HALLER,F. et al.** Loss of 9p leads to p16INK4A down-regulation and enables RB/E2F1-dependent cell cycle promotion in gastrointestinal stromal tumours (GISTs). *J Pathol,* 2008, vol. 215, 253-262
- **PERRONE,F. et al.** 9p21 locus analysis in high-risk gastrointestinal stromal tumors characterized for c-kit and platelet-derived growth factor receptor alpha gene alterations. *Cancer,* 2005, vol. 104, 159-169
- **ASSAMAKI, R. et al.** Array comparative genomic hybridization analysis of chromosomal imbalances and their target genes in gastrointestinal stromal tumors. *Genes Chromosomes Cancer,* 2007, vol. 46, 564-576
- **HUANG,H.Y. et al.** Homozygous deletion of MTAP gene as a poor prognosticator in gastrointestinal stromal tumors. *Clin Cancer Res,* 2009, vol. 15, 6963-6972
- **CHIBON. F. et al.** The RB1 gene is the target of chromosome 13 deletions in malignant fibrous histiocytoma. *Cancer Res.,* 15 November 2000, 6339-45

- **SERRANO,M. ; HANNON,G.J. ; BEACH,D.** A new regulatory motif in cell-cycle control causing specific inhibition of cyclin D/CDK4. *Nature,* 1993, vol. 366, 704-707
- **HANNON,G.J. ; BEACH,D.** p15INK4B is a potential effector of TGF-beta-induced cell cycle arrest. *Nature,* 1994, vol. 371, 257-261
- **KAMB,A. et al.** Analysis of the p16 gene (CDKN2) as a candidate for the chromosome 9p melanoma susceptibility locus. *Nat. Genet.,* 1994, vol. 8, 23-26
- **ENDERS,G.H.** The INK4a/ARF locus and human cancer. *Methods Mol Biol,* 2003, vol. 222, 197-209
- **PÉROT,G. et al.** Constant p53 Pathway Inactivation in a Large Series of Soft Tissue Sarcomas with Complex Genetics. *Am J Pathol.,* 2010, vol. 177, 2080-2090
- **HOUDAYER,C. et al.** Comprehensive screening for constitutional RB1 mutations by DHPLC and QMPSF. *Hum Mutat.,* 2004, vol. 23, 193-202
- **CARPINELLI P et al.** PHA-739358, a potent inhibitor of Aurora kinases with a selective target inhibition profile relevant to cancer. *Mol Cancer Ther.,* December 2007, vol. 6, 3158-68